# EUROPEAN PATENT SPECIFICATION

(11) **EP 1 102 528 B1**
(45) Date of publication and mention of the grant of the patent: **19.11.2008**
(21) Application number: 99942025.0
(22) Date of filing: 03.08.1999
(51) Int. Cl.: A01H 4/00, A01H 5/00, C12N 15/29, C12N 15/31, C12N 15/65, C12N 15/67, C12N 15/82, C12N 15/84

(54) **TRANSLATION CONTROL ELEMENTS FOR HIGH-LEVEL PROTEIN EXPRESSION IN THE PLASTIDS OF HIGHER PLANTS AND METHODS OF USE THEREOF**
TRANSLATIONS-KONTROLLELEMENTE FÜR PROTEINEXPRESSION MIT HOHER AUSBEUTE IN DEN PLASTIDEN VON HÖHEREN PFLANZEN UND VERFAHREN ZU DEREN VERWENDUNG
DES ELEMENTS DE REGULATION DE LA TRADUCTION POUR L'EXPRESSION A UN HAUT NIVEAU DE PROTEINES DANS LES PLASTES DE PLANTES SUPERIEURES ET LEURS PROCEDES D'UTILISATION

(30) Priority: 03.08.1998 US 95163 P; 03.08.1998 US 95167 P; 15.12.1998 US 112257 P; 29.04.1999 US 131611 P; 11.06.1999 US 138764 P
(43) Date of publication of application: 30.05.2001
(73) Proprietor: RUTGERS UNIVERSITY, New Brunswick, NJ 08901 (US)
(72) Inventor: MALIGA, Pal, East Brunswick, NJ 08816 (US); KURODA, Hiroshi, Highland Park, NJ 08904 (US); KHAN, Muhammad, Sarwar, Piscataway, NJ 08854 (US)
(74) Representative: O'Connell, Maura
(86) International application number: PCT/US1999/017806
(87) International publication number: WO 2000/007431

(56) References cited:
- WO-A1-97/06250
- VASIL V ET AL: "HERBICIDE RESISTANT FERTILE TRANSGENIC WHEAT PLANTS OBTAINED BY MICROPROJECTILE BOMBARDMENT OF REGENERABLE EMBRYOGENIC CALLUS" BIO/TECHNOLOGY, NATURE PUBLISHING CO. NEW YORK, US, vol. 10, no. 6, June 1992 (1992-06), pages 667-674, XP002922145 ISSN: 0733-222X
- WATANABE T ET AL: "THE DISTRIBUTION OF HOMOLOGOUS ENTEROCOCCAL PLASMID DNA SEQUENCES IN HUMAN FAECAL ISOLATES" JOURNAL OF APPLIED BACTERIOLOGY, vol. 73, no. 2, 1992, pages 131-135, XP008034667 ISSN: 0021-8847
- KURODA HIROSHI ET AL: "Complementarity of the 16S rRNA penultimate stem with sequences downstream of the AUG destabilizes the plastid mRNAs" NUCLEIC ACIDS RESEARCH, vol. 29, no. 4, 15 February 2001 (2001-02-15), pages 970-975, XP002294359 ISSN: 0305-1048
- KURODA HIROSHI ET AL: "Sequences downstream of the translation initiation codon are important determinants of translation efficiency in chloroplasts" PLANT PHYSIOLOGY (ROCKVILLE), vol. 125, no. 1, January 2001 (2001-01), pages 430-436, XP002294360 ISSN: 0032-0889
- KHAN M S ET AL: "FLUORESCENT ANTIBIOTIC RESISTANCE MARKER FOR TRACKING PLASTID TRANSFORMATION IN HIGHER PLANTS" NATURE BIOTECHNOLOGY, NATURE PUB. CO, NEW YORK, NY, US, vol. 17, September 1999 (1999-09), pages 910-915, XP002943529 ISSN: 1087-0156
- NIELSEN ET AL.: 'Characterization of the Pea Chloroplast DNA OriA Region' PLASMID vol. 30, 1993, pages 197 - 211, XP002922148
- SEKIYA ET AL.: 'Sequence of the gene for isoleucine tRNA1 and the surrounding region in a ribosomal RNA operon of Escherichia coli' NUCLEIC ACIDS RESEARCH vol. 6, 1979, pages 575 - 592, XP002922147
- SPRENGART ET AL.: 'The downstream box: an efficient and independent translation initiation signal in Escherichia coli' THE EMBO JOURNAL vol. 15, no. 3, 1996, pages 665 - 674, XP002922146
- VASIL ET AL.: 'Herbicide resistant fertile transgenic wheat plants obtained by microprojectile bombardment of regenerable embryogenic callus' BIO/TECHNOLOGY vol. 10, no. 6, June 1992, pages 667 - 674, XP002922145
- PANG ET AL.: 'An Improved Green Fluorescent Protein Gene as a Vital Marker in Plants' PLANT PHYSIOLOGY vol. 112, 1996, pages 893 - 900, XP002922144
- HIRATSUKA ET AL.: 'The complete sequence of the rice (Oryza sativa) chloroplast genome: Intermolecular recombination between distinct tRNA genes accounts for a major plastid DNA inversion during the evolution of cereals' MOLECULAR AND GENERAL GENETICS vol. 217, 1989, pages 185 - 194, XP002922143
- ZHANG ET AL.: 'Efficient regeneration of transgenic plants from rice protoplasts and correctly regulated expression of the foreign gene in plants' THEORETICAL AND APPLIED GENETICS vol. 76, 1988, pages 835 - 840, XP002922142
- THOMPSON ET AL.: 'Protoplast culture of rice (Oryza sativa L.) using media solidified with agarose' PLANT SCIENCE vol. 47, no. 2, 1986, pages 123 - 133, XP002922141
- MAKRIDES S.C.: 'Strategies for Achieving High-Level Expression of Genes in Escherichia coli' MICROBIOLOGICAL REVIEWS vol. 60, no. 3, September 1996, pages 512 - 538, XP002922140
- MURRAY ET AL.: 'Codon usage in plant genes' NUCLEIC ACIDS RESEARCH vol. 17, no. 2, 1989, pages 477 - 498, XP002922139
- ZOUBENKO O. ET AL: 'Efficient targeting of foreign genes into the tobacco plastide genome' NUCLEIC ACID RESEARCH vol. 22, no. 19, 1994, pages 3819 - 3824

## Description

This application claims priority from United States Provisional Applications 60/095,163, filed August 3, 1998, 60/112,257, filed December 15, 1998, 60/095,167 filed August 3, 1998, 60/131,611, filed April 29, 1999 and 60/138,764, filed June 11, 1999 under 35 U.S.C. §119(e).

Pursuant to 35 U.S.C. §202(c) it is acknowledged that the U.S. Government has certain rights in the invention described herein, which was made in part with funds from the National Science Foundation, Grant Number MCB-96-30763.

### FIELD OF THE INVENTION

This invention relates to the fields of transgenic plants and molecular biology. More specifically, the invention provides vectors targeting the plastid genome which contain translation control elements facilitating high levels of protein expression in the plastids of higher plants. Both monocots and dicots are successfully transformed with the DNA constructs provided herein.

### BACKGROUND OF THE INVENTION

Several publications are referenced in this application in order to more fully describe the state of the art to which this invention pertains.

The chloroplasts of higher plants accumulate individual components of the photosynthetic machinery as a relatively large fraction of total cellular Protein. The best example is the enzyme ribulose-1,5-bisphosphate carboxylase-oxygenase (Rubisco) involved in CO₂ fixation which can make up 65% of the total leaf protein (Ellis, R.J. 1979). Because of the potentially attainable high protein levels, there is significant interest in exploring chloroplasts as an alternative system for protein expression. To date, protein levels expressed from transgenes in chloroplasts are below the levels of highly-expressed chloroplast genes. Highest levels reported thus far in leaves are as follows: 1% of neomycin phophotransferase (Carrer et al., 1993); 2.5% β-glucuronidase. (Staub and Maliga, 1993) and 3-5% of Bacillus thuringiensis (Bt) crystal toxins (McBride et al., 1995). An alternative system, based on a nuclear-encoded, plastid-targeted T7 RNA polymerase may offer higher levels of protein expression (McBride t al., 1994), although this yield may come at a price.

Additional vectors suitable for efficient targeting of foreign genes into the tobacco plastid genome are described in Zoubenko et al. NAR (1994)22:3819-3824. The vectors are based on the pUC119 plasmid which replicates in *E. coli* but not in plastids. The spectinomycin resistance (aadA) gene and a multiple cloning site (MCS) are flanked by 1.8-kb and 1.2-kb ptDNA sequences. Biolistic delivery of vector DNA, followed by spectinomycin selection, yields plastid transformants at a reproducible frequency, approximately 1 transplastomic line per bombarded sample.

WO/97 06250 describes another series of vectors for plastid transformation. The constructs described contain unique promoters that are transcribed by both nuclear encoded plastid polymerases and plastid encoded plastid polymerases. The constructs described facilitate transformation of a wider range of plant species and enables tissue specific expression of a transforming DNA in plastids of multicellular plants.

In bacteria, the rate limiting step of protein synthesis is usually the initiation of translation, involving the binding of the initiator tRNA (formyl-methionyl-tRNA_{f}) and mRNA to the 70S ribosome, recognition of the initiator codon, and the precise phasing of the reading frame of the mRNA. Translation initiation depends on three initiation factors (IF1, IF2, IF3) and requires GTP. The 30S subunit is guided to the initiation codon by RNA-RNA base pairing between the 3' of the 16S rRNA and the mRNA ribosome binding site, or Shine-Dalgarno (SD) sequence, located about 10 nucleotides upstream of the translation initiation codon (Voorma, 1996) - RNA-RNA interaction between the "downstream box" (DB), a 15 nt sequence downstream of the AUG translational initiation codon and complementary sequences in the 16S rRNA 3' sequence or anti-downstream box (ADB; nucleotide positions 1469-1483) may also facilitate loading of the mRNA onto the 30S ribosome subunit (Sprengart et al., 1996). In addition, specific protein-RNA interactions may also facilitate translation initiation (Voorma, 1996).

Key components of the prokaryotic translation machinery have been identified in plastids, including homologues of the bacterial IF1, IF2 and IF3 initiation factors and an SI-like ribosomal protein (Stern et al., 1997). Most plastid mRNAs (92%) contain a ribosome binding site or SD sequence: GGAGG, or its truncated tri- or tetranucleotide variant. This sequence is similar to the bacterial SD consensus 5'-UAAGGAGGUGA-(Voorma, 1996). High level expression of foreign genes of interest in the plastids of higher plants is extremely desirable. The present invention provides novel genetic translational control elements for use in plastid transformation vectors. Incorporation of these elements into such vectors results in protein expression levels comparable to those observed for highly expressed chloroplast genes in both monocots and dicots.

### SUMMARY OF THE INVENTION

5' genetic regulatory regions contain promoters with distinct DNA sequence information which facilitates recognition by the RNA polymerase and translational control elements which facilitate translation. Both of these components act together to drive gene expression.

In accordance with the present invention, chimeric 5' regulatory regions have been constructed which incorporate translation control elements. Incorporation of these chimeric 5' regulatory regions into plastid transforming vectors followed by transformation of target plant cells gives rise to dramatically enhanced levels of protein expression. These chimeric 5' regulatory regions may be used to advantage to express foreign genes of interest in a wide range of plant tissues. It is an object of the present invention to provide DNA constructs and methods for stably transforming plastids of multicellular plants containing such promoters.

In one embodiment of the invention recombinant DNA constructs for expressing at least one heterologous protein in the plastids of higher plants are provided. The constructs comprise a 5' regulatory region which includes a promoter element, a leader sequence and a downstream box element operably linked to a coding region of said at least one heterologous protein. The chimeric regulatory region acts to enhance translational efficiency of an mRNA molecule encoded by said DNA construct. Vectors comprising the DNA constructs are also contemplated in the present invention. Exemplary DNA constructs of the invention include the following chimeric regulatory regions: PrnnLatpB+DBwt, PrrnLatpB+DBm, PrrnLclpP+DBwt, PrrnLrbcL+DBwt, PrrnLrbcL+DBm, PrrnLpsbB+DBwt, PrrnLpsbA+DBwt, PrrnLT7g10+DB/EC and PrrnLT7g10+bB/pt. Downstream box sequences preferred for use in the constructs of the invention have the following sequences:

5'TCCAGTCACTAGCCCTGCCTTCGGCA'3 and 5'CCCAGTCATGAATCACA
AAGTGGTAA'3.

The 5' regulatory segments of the invention have been successfully employed to drive the expression of the bar gene from *S. hydroscopicus* in the plastids of higher plants. Synthetic bar genes have also been generated and expressed using the DNA constructs of the present invention. These constructs have been engineered to maximize transgene containment in plastids by incorporating rare codons into the coding region that are not preferred for protein translation in microorganisms and fungi.

In yet another embodiment of the invention, at least one fusion protein is produced utilizing the DNA constructs of the invention. An exemplary fusion protein has a first and second coding region operably linked to the 5' regulatory regions described herein such that production of said fusion protein is regulated by said 5' regulatory region. In one embodiment the first coding region encodes a selectable marker gene and the second coding region encodes a fluorescent molecule to facilitate visualization of transformed plant cells. Vectors comprising a DNA construct encoding such a fusion protein are also within the scope of the present invention. An exemplary fusion protein consists an aadA coding region operably linked to a green fluorescent protein coding region. These moieties may be linked by peptide linkers such as ELVEGKLELVEGLKVA and ELAVEGKLEVA.

Plasmids for transforming the plastids of higher plants, are also included in the present invention. Exemplary plasmids are selected from the group consisting of pMSK49 and pMSK35.

Transgenic plants, both monocots and dicots harboring the plasmids set forth above are also contemplated to be within the scope of the invention.

The following definitions will facilitate the understanding of the subject matter of the present invention:
**Heteroplastomic:** refers to the presence of a mixed population of different plastid genomes within a single plastid or in a population of plastids contained in plant cells or tissues.
**Homoplastomic:** refers to a pure population of plastid genomes, either within a plastid or within a population contained in plant cells and tissues. Homoplastomic plastids, cells or tissues are genetically stable because they contain only one type of plastid genome. Hence, they remain homoplastomic even after the selection pressure has been removed, and selfed progeny are also homoplastomic. For purposes of the present invention, heteroplastomic populations of genomes that are functionally homoplastomic (i.e., contain only minor populations of wild-type DNA or transformed genomes with sequence variations) may be referred to herein as "functionally homoplastomic" or "substantially homoplastomic." These types of cells or tissues can be readily purified to a homoplastomic state by continued selection.
**Plastome:** the genome of a plastid.
**Transplastome:** a transformed plastid genome.
**Transformation of plastids:** stable integration of transforming DNA into the plastid genome that is transmitted to the seed progeny of plants containing the transformed plastids.
**Selectable marker gene**: the term "selectable marker gene" refers to a gene that upon expression confers a selective advantage to the plastids and a phenotype by which successfully transformed plastids or cells or tissues carrying the transformed plastid can be identified.
**Transforming DNA**: refers to homologous DNA, or heterologous DNA flanked by homologous DNA , which when introduced into plastids becomes part of the plastid genome by homologous recombination.
**Operably linked**: refers to two different regions or two separate genes spliced together in a construct such that both regions will function to promote gene expression and/or protein translation.
The detailed description as follows provides examples of preferred methods for making and using the DNA constructs of the present invention and for practicing the methods of the invention. Any molecular cloning and recombinant DNA techniques not specifically described are carried out by standard methods, as generally set forth, for example in Sambrook et al., "DNA Cloning, A Laboratory Manual," Cold Spring Harbor Laboratory, 1989.

### BRIEF DESCRIPTION OF THE DRAWINGS

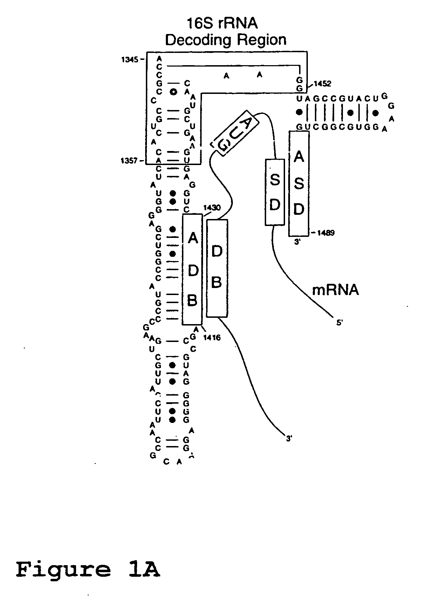

**Figure 1A****.** Plastid mRNAs and the small (16S) ribosomal RNA contain complementary sequences downstream of AUG implicating interactions between mRNA and 16S rRNA during translation initiation in plastids. Proposed model is based on data in *E. coli* (Sprengart *et al.*, 1996); for sequence of 16S rRNA see ref. (Shinozaki *et al.*, 1986b). SD, Shine-Dalgarno sequence; ASD, anti SD region; DB, downstream box; ADB, anti DB region. Watson-Crick (line) and G-U (closed circle) pairing are marked.

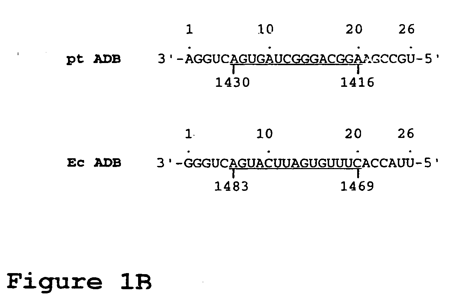

**Figure 1B****.** Sequence of the anti-downstream-box regions (ADB sequence underlined) of the 16S rRNA in plastids (pt; this application) and in *E. coli* (Ec; Sprengart et al., 1996). The *E. coli* ADB box contains sequences between nucleotides 1469-1483 of the 16S rRNA (Sprengart et al., 1996), corresponding to nucleotides 1416-1430 of the tobacco 16S rRNA (Dams et al., 1988; sequence between nucleotides 104173-104187 in Shinozaki et al., 1986).

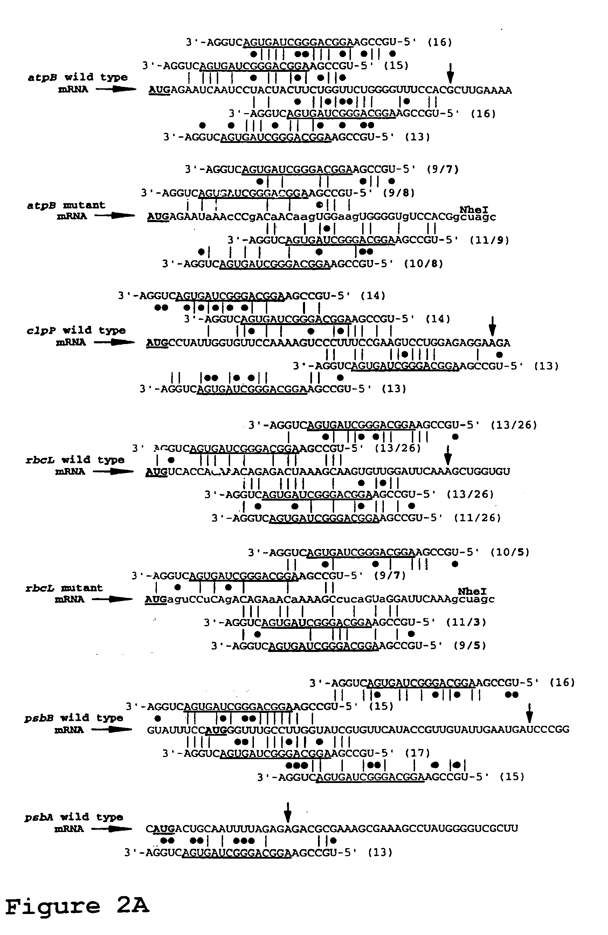

**Figure 2A****.** Base-pairing between plastid ADB and *atpB*, *clpP, rbcL, psbB* and *psbA* mRNAs (underlined). Multiple alternative DB-ADB interactions are shown. Nucleotides changed to reduce or alter mRNA-rRNA interaction are in lower case. The number of potential nucleotide pairs formed with the 26 nt ADB region is in parenthesis. The number of pairing events affected by mutagenesis is in bold.

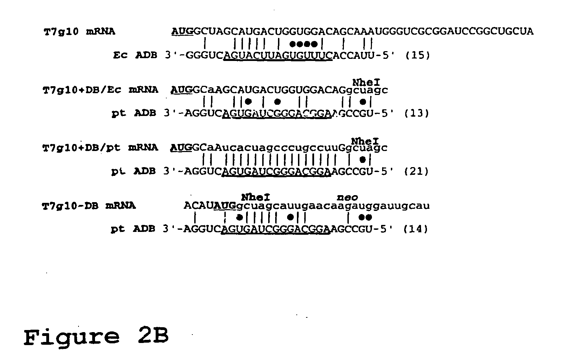

**Figure 2B****.** Complementarity of Prrn T7 phage gene 10 leader derivatives with the *E. coli* and plastid ADB sequences. Nucleotides changed to reduce or alter mRNA-rRNA interaction are in lower case. The number of potential nucleotide pairs formed with the 26 nt ADB region is in parenthesis.

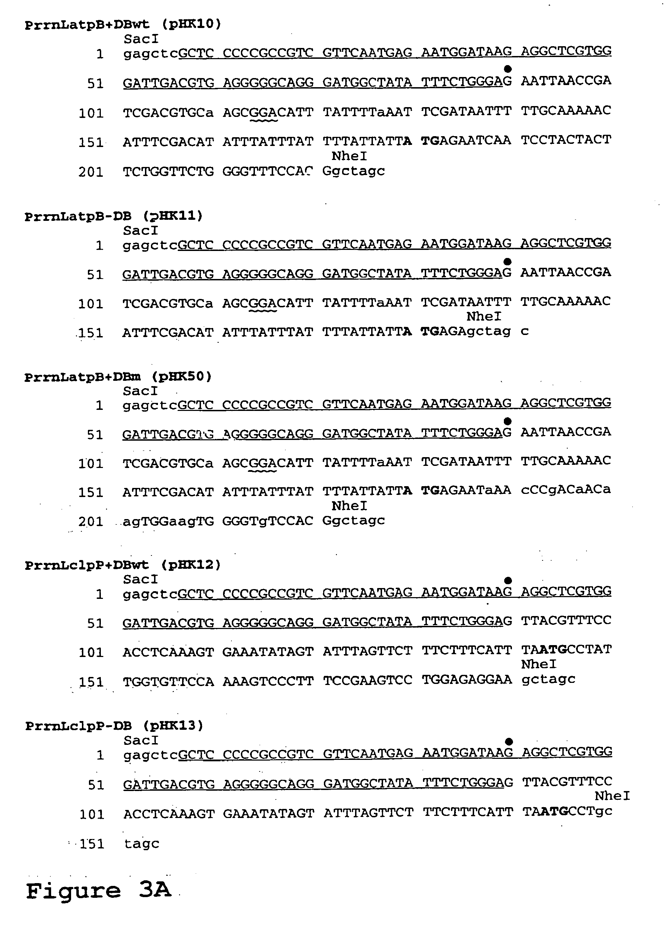

**Figure 3A****.** DNA sequence of the chimeric Prrn plastid promoter fragments with *atpB* and *clpP* translation control regions. The plasmid name that is the source of the promoter fragment is given in parenthesis. The Prrn promoter sequence is underlined; nucleotide at which transcription initiates in tobacco plastids is marked with filled circle; translational initiation codon (ATG) is in bold; SD is underlined with a wavy line; nucleotides of the 5' and 3' restriction sites and point mutations are in lower case.

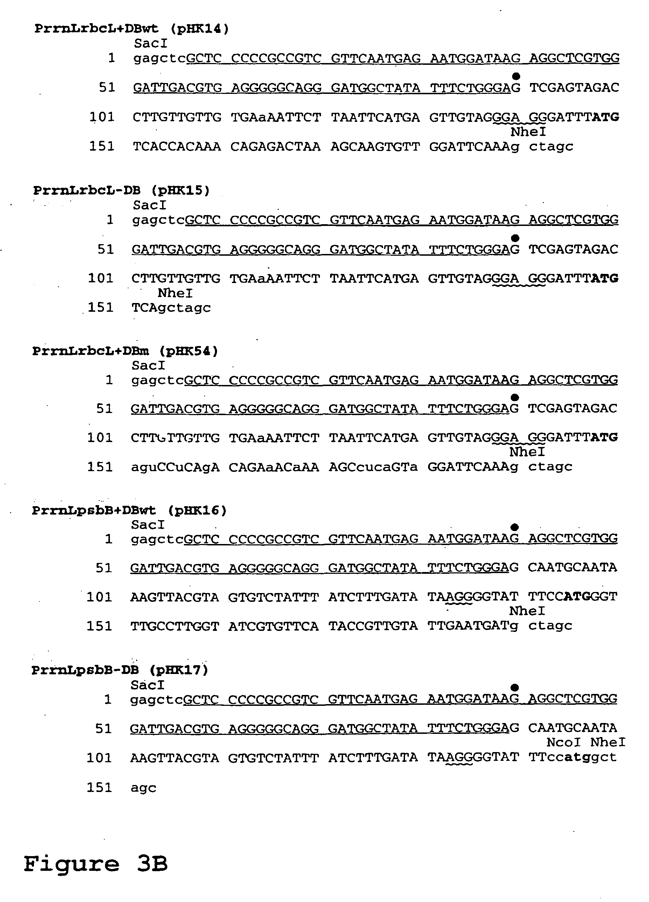

**Figure 3B****.** DNA sequence of the chimeric Prrn plastid promoter fragments with *rbcL* and *psbB* translation control regions. For details see description of Fig. 3A.

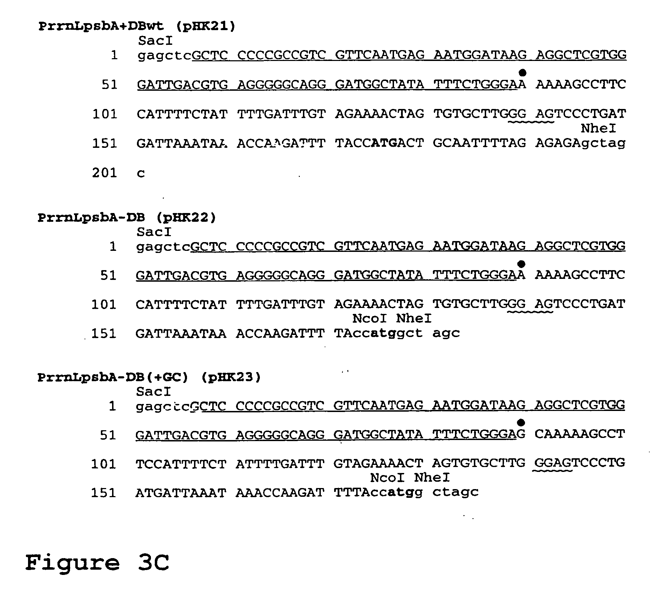

**Figure 3C****.** DNA sequence of the chimeric Prrn plastid promoter fragments with *psbA* translation control regions. For details see description of Fig. 3A.

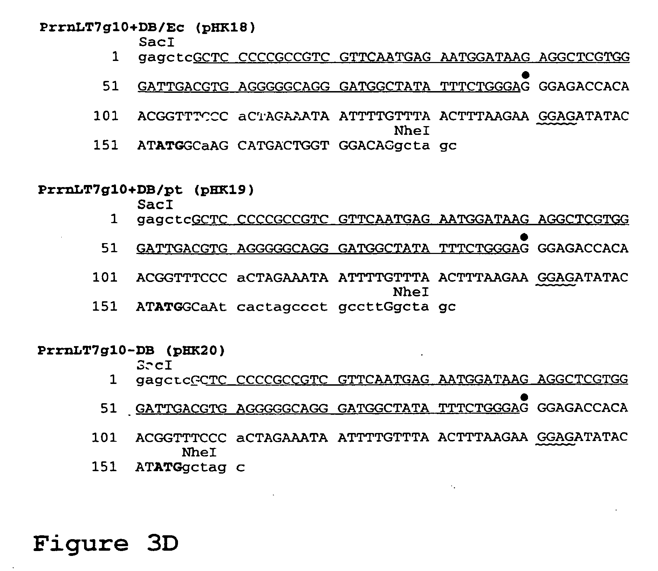

**Figure 3D****.** DNA sequence of the chimeric Prrn plastid promoter fragments with the T7 phage gene 10 (PrrnLT7g10+DB/Ec) plastid (PrrnLT7g10+DB/pt) and synthetic DB (PrrnLT7g10-DB). For details see description of Fig. 3A.

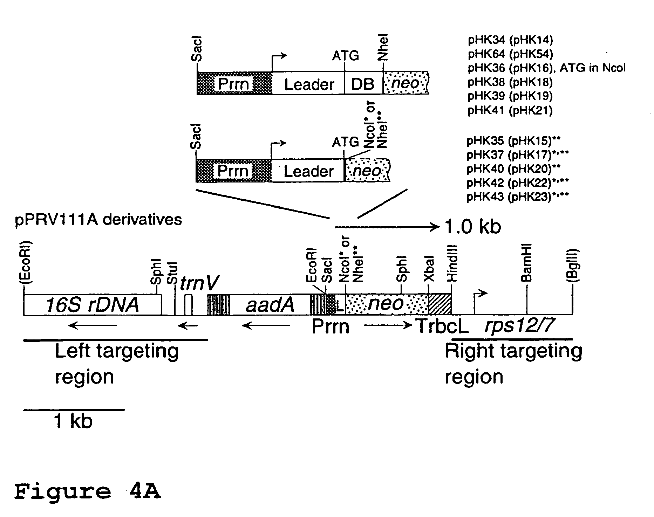

**Figure 4A****.** Plastid transformation vector pPRV111A with chimeric *neo* genes. Plasmid serial numbers, for example pHK34, designate pPRV111A plastid transformation vectors derivatives; adjacent plasmid numbers in parenthesis (e.g. pHK14) designate the source of the chimeric *neo* gene in pUC118 or pBSIIKS+ vectors. Arrows mark orientation of the selectable marker gene (*aadA*) and of the chimeric *neo* gene. Plastid targeting sequences are underlined in bold. Components of the chimeric *neo* genes are: Prrn, rRNA operon promoter fragment; L, leader sequence; DB, downstream box; *NheI* site which serves as a synthetic DB is marked by a heavy line; *neo*, neomycin phosphotransferase coding region; TrbcL, *rbcL* 3'-untranslated region. *16SrDNA, trnV*, *rps12*/*7* are plastid genes (Shinozaki et al., 1986). The restriction sites marked for: EcoRI, SphI, StuI, SacI, NheI, NcoI, XbaI, HindIII, BamHI and BglII. Restriction sites in brackets were eliminated during construction. The *neo* translation initiation in plasmid pHK36 is included in NcoI site (not marked). The presence and relative order of NheI (**) and NcoI (*) restriction sites in the plasmid pPRV111A -DB derivatives (pHK35, pHK37, pHK40, pHK42, pHK43) are marked by asterisks. The promoter sequences are shown in Figures 3B, C and D.

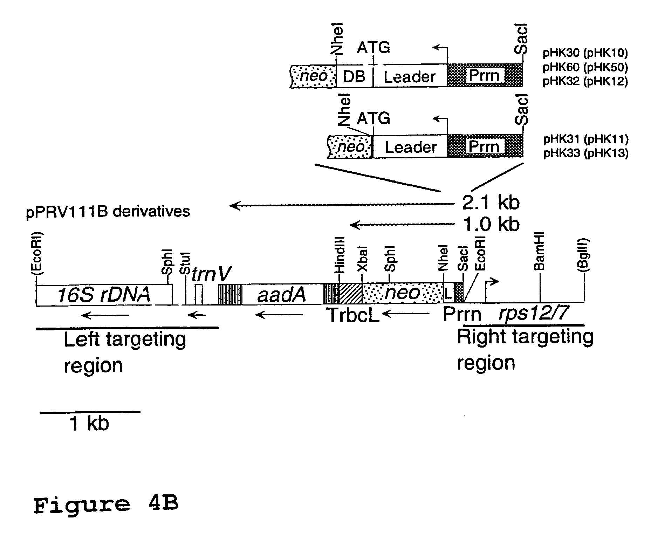

**Figure 4B****.** Plastid transformation vector pPRV111B with chimeric *neo* genes. See description of Fig. 4A. The promoter sequences are shown in Fig. 3A.

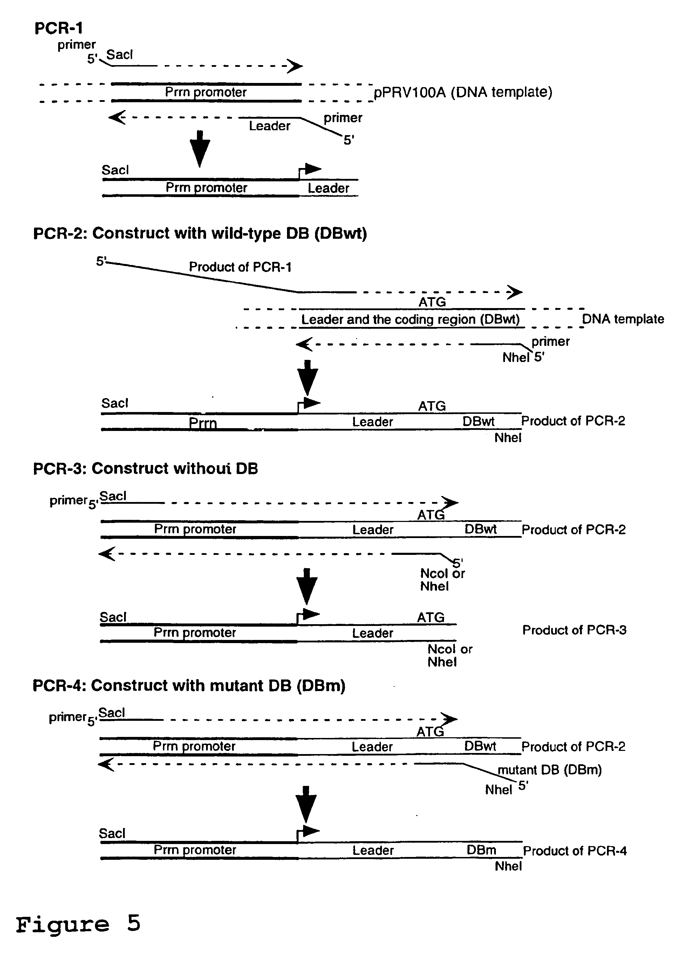

**Figure 5****.** Construction of Prrn promoter-plastid leader fragments by overlap extension PCR.

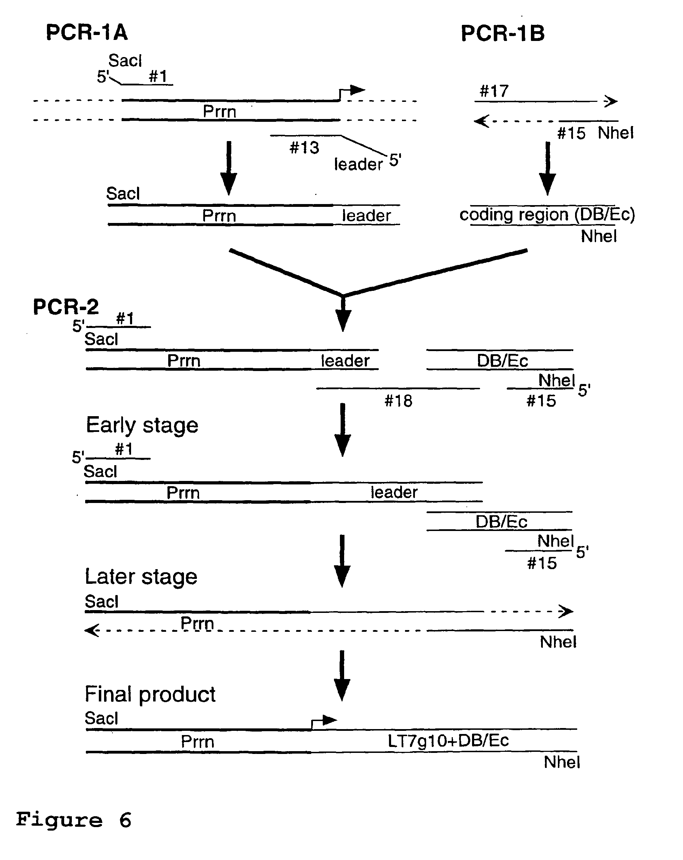

**Figure 6****.** Construction by the PCR of PrrnLT7g10+DB/Ec promoter (SacI-NheI fragment) in plasmid pHK18.

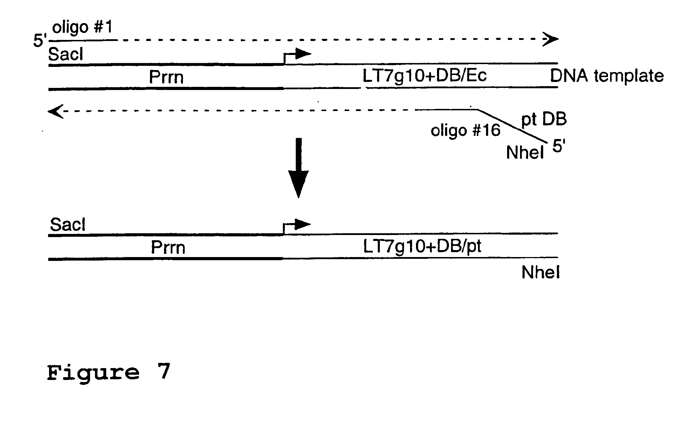

**Figure 7****.** Construction by PCR of the PrrnLT7g10+DB/pt promoter (SacI-NheI fragment) in plasmid pHK19.

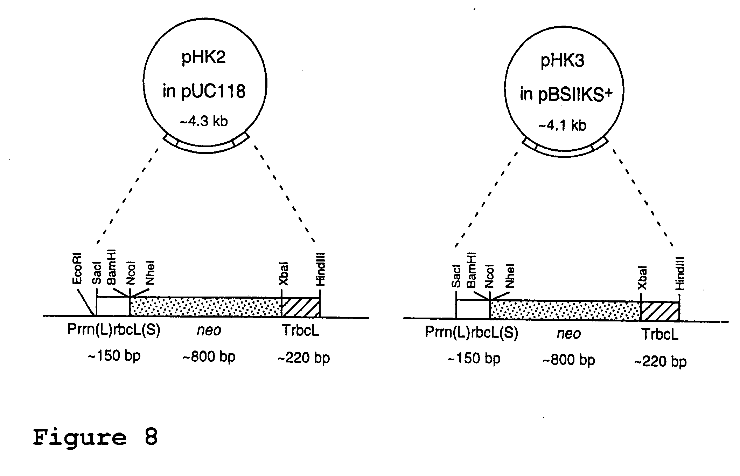

**Figure 8****.** Restriction map of plasmids pHK2 and pHK3 with the Prrn(L)rbcL(S)::*neo*::TrbcL gene. Restriction enzyme cleavage sites are marked for: BamHI, EcoRI, HindIII, NcoI, NheI, SacI, XbaI.

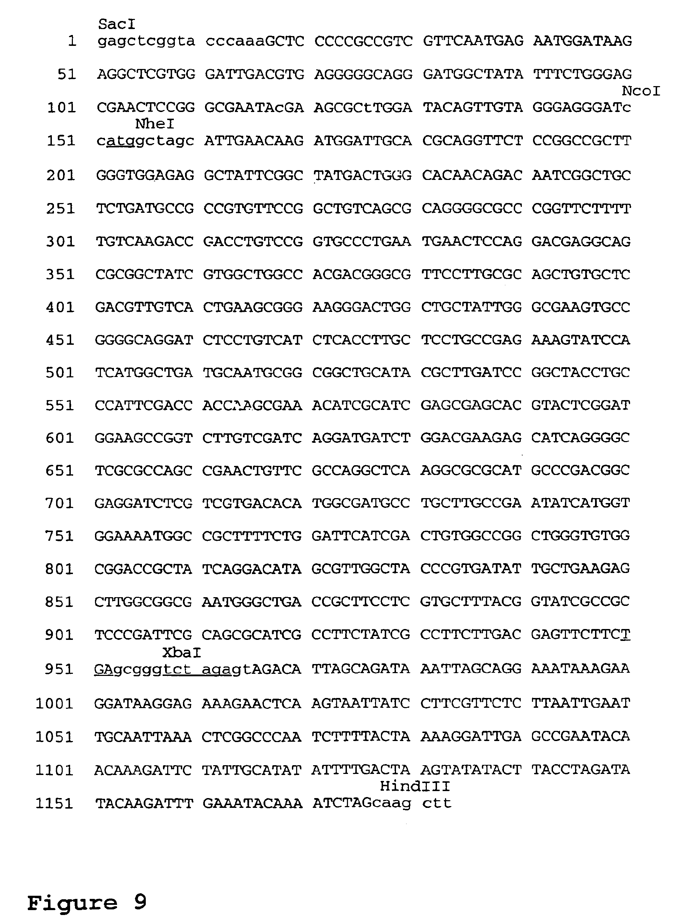

**Figure 9****.** DNA sequence of the Prrn(L)rbcL(S)::*neo*::TrbcL gene in plasmid pHK3. Plasmid pHK2 carries an identical *neo* gene, except that there is an EcoRI site upstream of the SacI site.

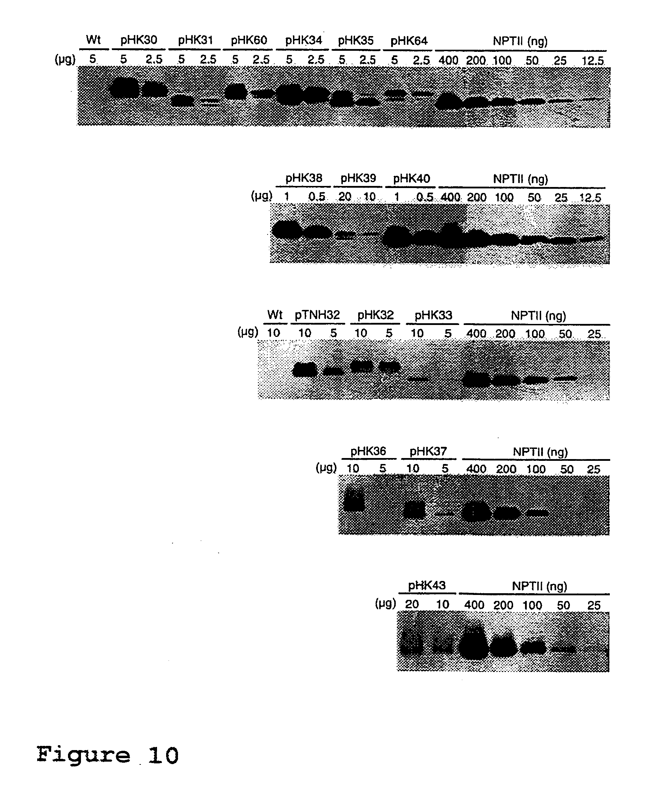

**Figure 10****.** NPTII accumulation in tobacco leaves detected by protein gel blot analysis. Amount of total soluble leaf protein (µg) loaded on SDS-PAGE gel is indicated above the lanes. Lanes are designated with plasmid used for plant transformation; µg protein loaded per lane is given below. NPTII standard and Nt-pTNH32 extracts were run as positive controls; extracts from wild-type non-transformed plants (wt) were used as negative controls.

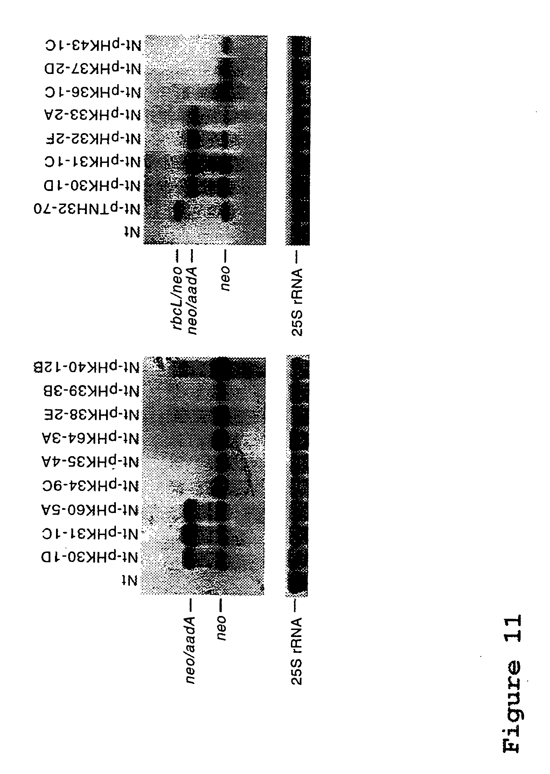

**Figure 11****.** The levels of neo mRNA in the transplastomic leaves. The blots were probed for neo (top) and cytoplasmic 25S rRNA as loading control (bottom). Positions of the monocistronic neo mRNA in vector pPRV111A (Figure 4A), the monocistronic neo and dicistronic *neo*-*aadA* transcripts in vector pPRV111B (Figure 4B) and the monocistronic *neo* and dicistronic *rbcL-neo* transcripts in pTNH32 transformed plants (Carrer et al., 1993) are marked. Lanes are designated with the transgenic plant serial number. 4 µg total cellular RNA was loaded per lane.

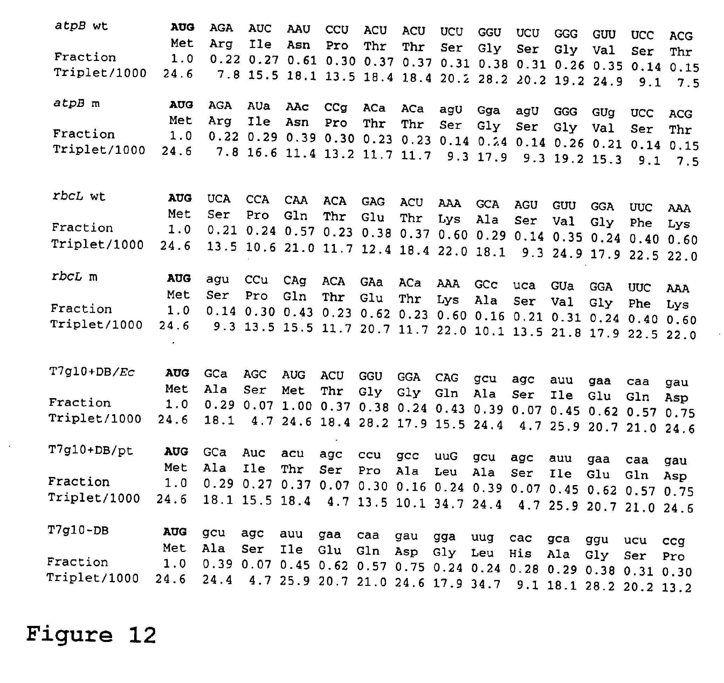

**Figure 12****.** Fraction of a codon encoding a particular amino acid and triplet frequency per 1000 codons in the mutagenized *atpB* and *rbcL* DB region. Altered nucleotides are in lower case.

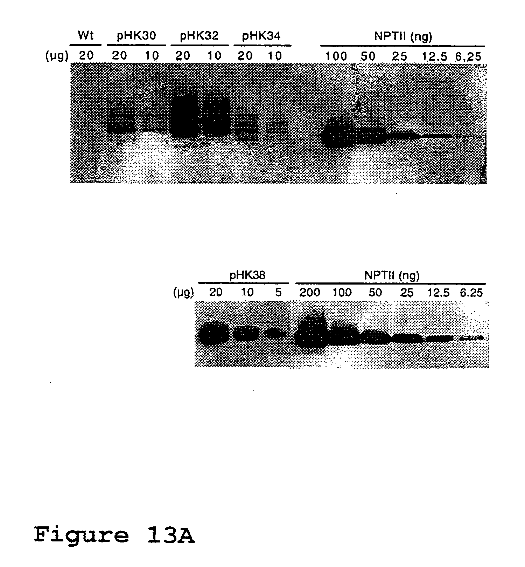

**Figure 13A****.** NPTII accumulation in tobacco roots detected by protein gel blot analysis. Lanes are designated with the plasmid used for plant transformation; µg protein loaded per lane is given below. NPTII standard was run as positive control; extracts from wild-type non-transformed plants (wt) were used as negative controls.

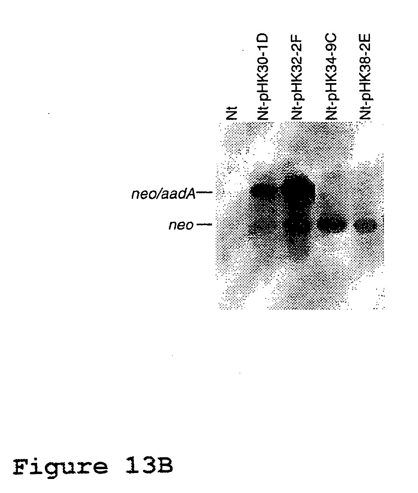

**Figure 13B****.** Steady-state levels of *neo* mRNA in tobacco roots. The *neo* probe detects a monocistronic mRNA in plants transformed with vector pPRV111A (Figure 4A), and a monocistronic *neo* and a dicistronic neo-aadA transcript in plants transformed with vector pPRV111B (Figure 4B). Lanes are designated with the transgenic plant serial number. 4 µg total cellular RNA was loaded per lane.

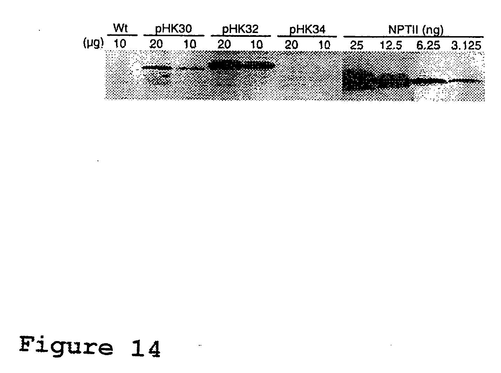

**Figure 14****.** Protein gel blot analysis to detect NPTII accumulation in tobacco seeds. Lanes are designated with plasmid used for plant transformation; µg protein loaded per lane is given below. NPTII standard was run as positive control; extracts from wild-type non-transformed plants (wt) were used as negative controls.

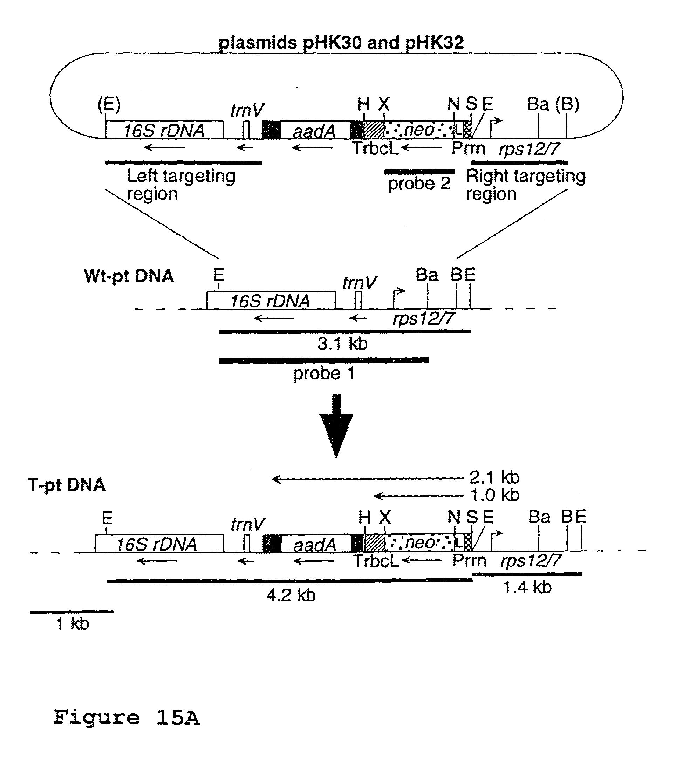

**Figure 15A****.** Diagram showing integration of the chimeric *neo* and *aadA* genes into the plastid genome by two homologous recombination events via the plastid targeting sequences (underlined). On top is shown a diagram of plasmids pHK30 and pHK32 are plastid transformation vector pPRV111B derivatives (Zoubenko et al., 1994). Horizontal arrows mark gene orientation. For description of chimeric *neo* genes, see Figure 4B. *16SrDNA, trnV, rps12*/*7* are plastid genes (Shinozaki et al., 1986). The restriction sites marked for: EcoRI (E), SacI (S), NheI (N), XbaI (X), HindIII (H), BamHI (Ba) and BglII Restriction sites in brackets were eliminated during construction. In the middle the wild-type plastid DNA region (Wt-ptDNA) targeted for insertion is shown. Lines connecting plasmids and ptDNA mark sites of homologous recombination at the end of the vector plastid-targeting regions. The transformed plastid genome segment (T-ptDNA) map is shown on the bottom.

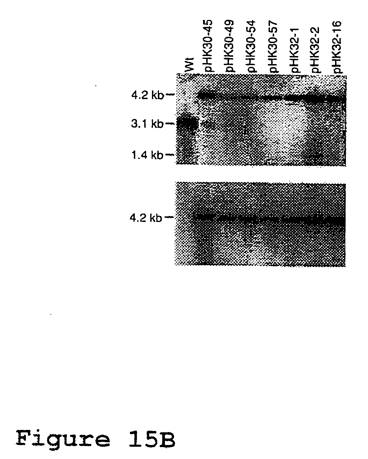

**Figure 15B****.** DNA gel blot analysis confirms of integration of the *neo* and *aadA* genes into the plastid genome. The blot on top was probed with the plastid targeting sequence (Probe 1 in Figure 15A). It lights up 4.2-kb and 1.4-kb fragments in transplastomic lines, and a 3.1-kb fragment in wild-type (see Figure 15A). Note that the 1.4-kb signal is week in most clones. The blot on the bottom was probed for neo sequences, which are present only in the transplastomic lines.

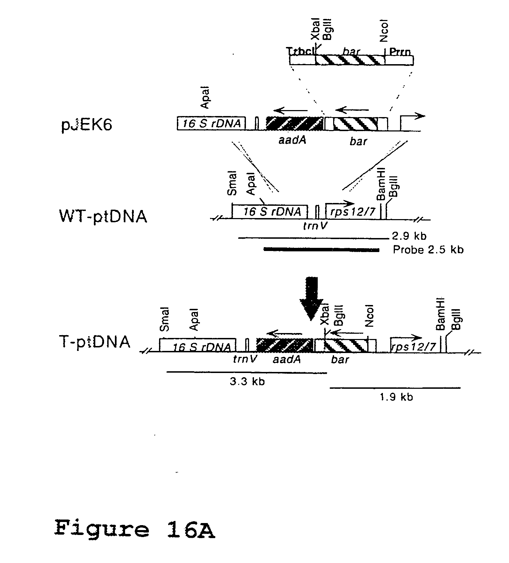

**Figure 16A****.** Diagram showing integration of the *bar* gene into the tobacco plastid genome. Map of the plastid targeting region in plasmid pJEK6 is shown on top. The targeted region of the wild-type plastid genome (wt-ptDNA) is shown in the middle. Integrated transgenes in the transplastome (T-ptDNA) are shown at the bottom. Map positions are shown for: the *bar* gene; *aadA,* the selectable spectinomycin resistance gene; *16SrDNA* and *rps12*/*7,* plastid genes (Shinozaki et al., 1986). Arrows indicate direction of transcription. Map position of the probe (2.5 kb) is marked by a heavy line; the wild-type (2.9-kb) and transgenic (3.3-kb, 1.9-kb) fragments generated by *Sma*I and *Bgl*II digestion are marked by thin lines.

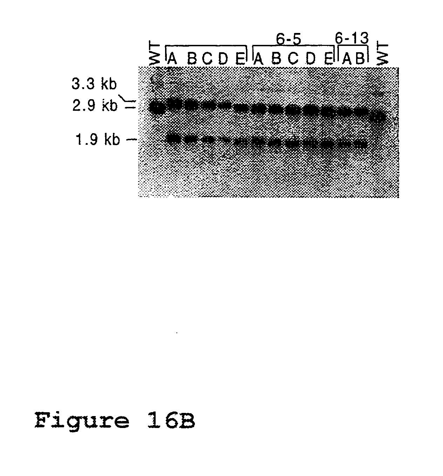

**Figure 16B****.** DNA gel blot confirms integration of bar into the tobacco plastid genome. Data are shown for transplastomic lines Nt-pJEK6-2A through E, Nt-pJEK6-5A through E and Nt-pJEK6-13A and B, and the wild-type parental line. *Sma*I-*Bgl*II digested total cellular DNA was probed with the 2.5-kb *Apa*I-*Bam*HI plastid targeting sequence marked with heavy line in Figure 16A.

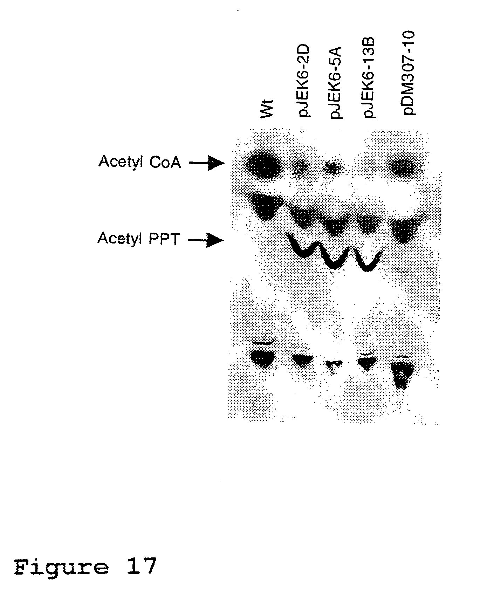

**Figure 17****.** PAT assay confirms bar expression in tobacco plastids. PAT activity was determined by conversion of PPT into acetyl-PPT using radiolabeled ¹⁴C-Acetyl-CoA. Data are shown for transplastomic lines Nt-pJEK6-2D, Nt-pJEK6-5A and Nt-pJEK6-13B, nuclear transformant Nt-pDM307-10 and wild-type (wt).

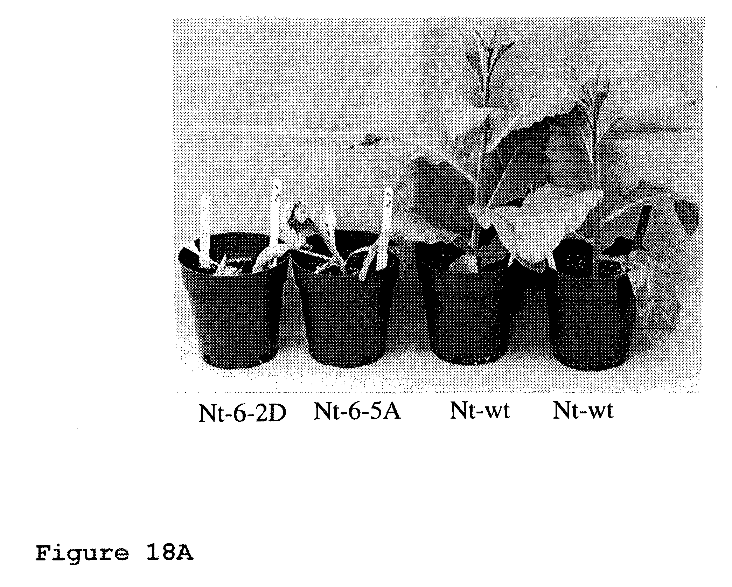

**Figure 18A****.** Transplastomic tobacco plants are herbicide resistant. Wild-type and pJEK6-transformed plants 13 days after Liberty spraying (5 ml, 2% solution).

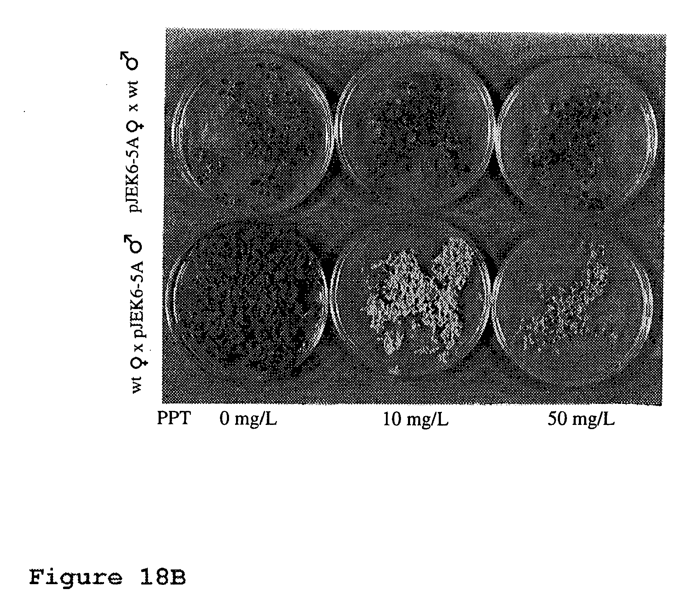

**Figure 18B****.** Maternal inheritance of PPT resistance in the seed progeny. Seeds from reciprocal crosses with Nt-pJEK6-5A plants germinated on 0, 10 and 50 mg/L PPT. wt x pJEK6-5A, transplastomic used as pollen parent; pJEK6-5A x wt , transplastomic line female parent. Resistant seedlings are green on PPT medium, sensitive seedlings are bleached.

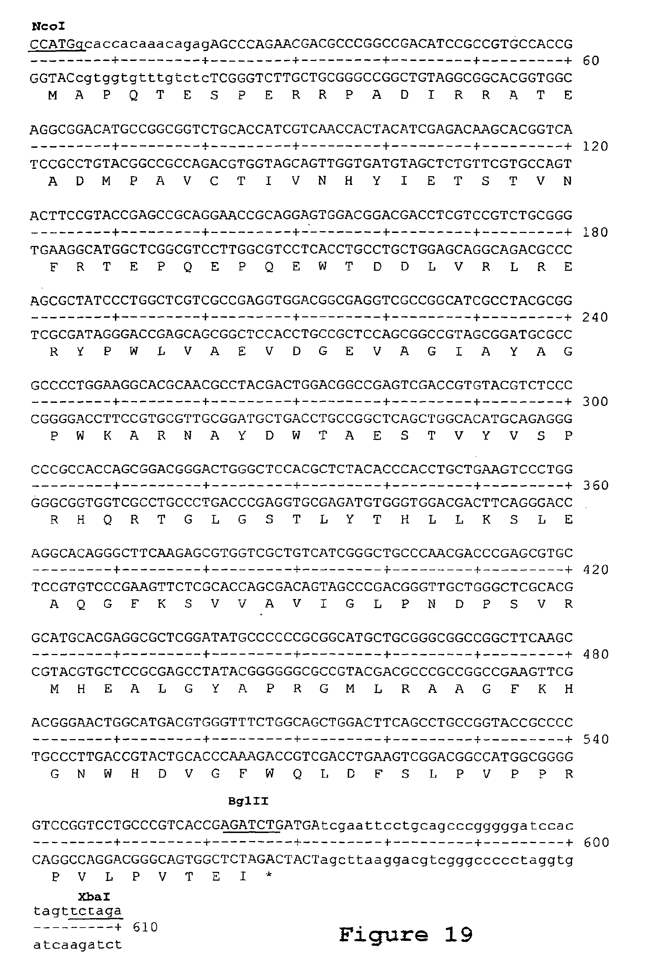

**Figure 19****.** The engineered bacterial bar coding region DNA sequence in plasmid pJEK3 and pJEK6 and encoded amino acid sequence. Nucleotides encoding the *rbcL* five N-terminal amino acids are in lower case. Nucleotides added at the 3' end during construction are also in lower case. NcoI, BglII and XbaI cloning sites are marked.

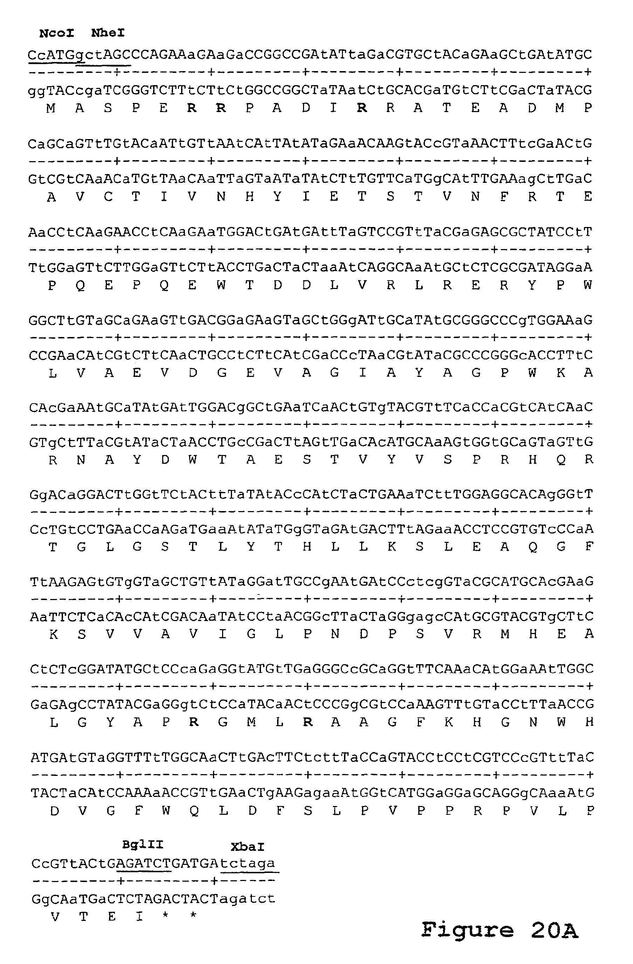

**Figure 20A****.** The synthetic bar gene DNA sequence and the encoded amino acid sequence. The arginines encoded by AGA/AGG codons are in bold. Original nucleotides are in capital letters, altered bases are in lower case. Restriction sites used for cloning are marked.

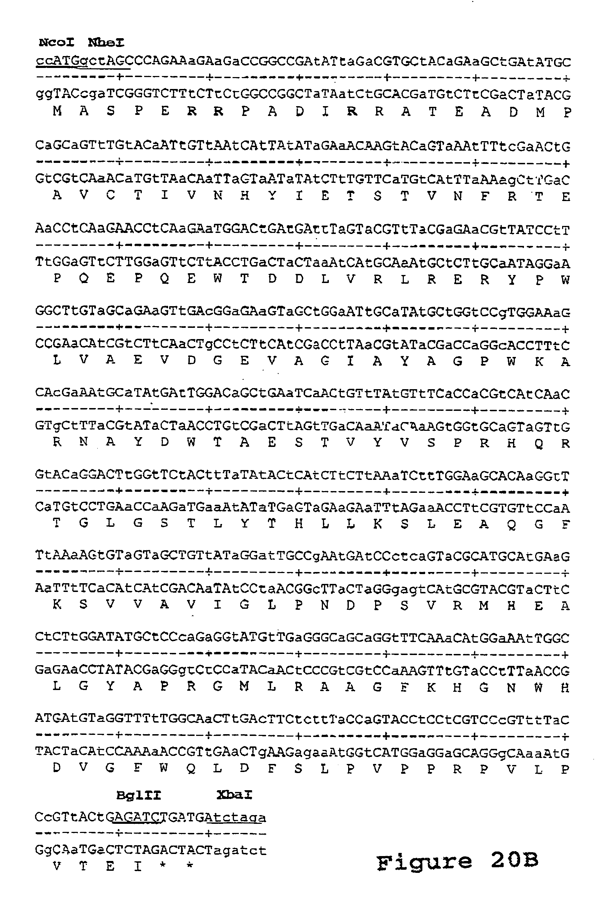

**Figure 20B****.** The synthetic *s2-bar* gene DNA sequence and the encoded amino acid sequence. The arginines encoded by AGA/AGG codons are in bold. Original nucleotides are in capital letters, altered bases are in lower case. Restriction sites used for cloning are marked.

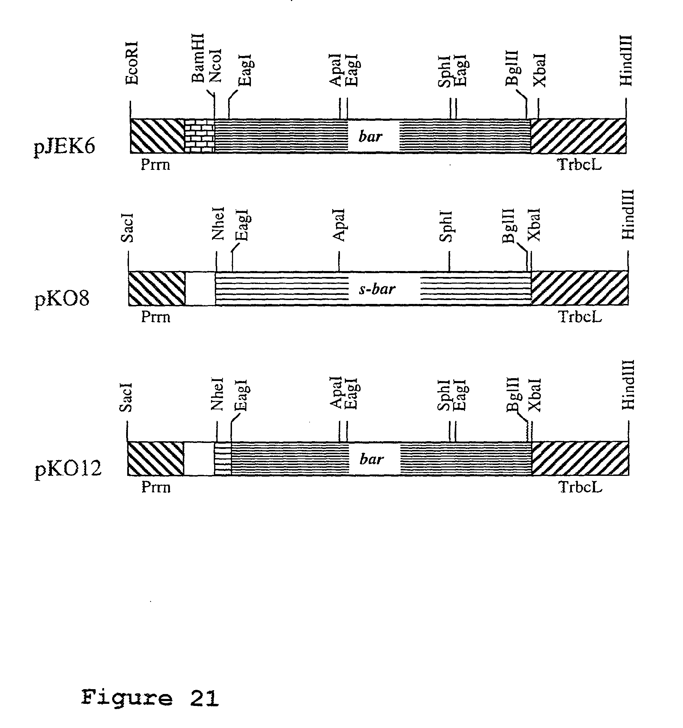

**Figure 21****.** Synthetic and bacterial bar genes. The *bar* coding region is expressed in the Prrn/TrbcL cassettes. Note that the Prrn promoters differ with respect to the translational control region.

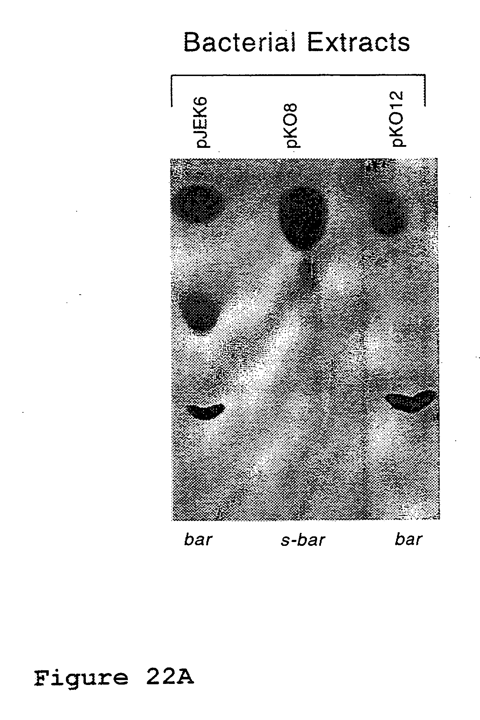

**Figure 22A****.** PAT is expressed in *E. coli* from bar, but not from *s-bar* coding region. PAT activity was determined by conversion of PPT into acetyl-PPT using radiolabeled ¹⁴C-Acetyl-CoA. Data are shown for *E. coli* transformed with plasmids pJEK6 and pKO12 carrying the *bar* gene, and pK08, carrying s-bar.

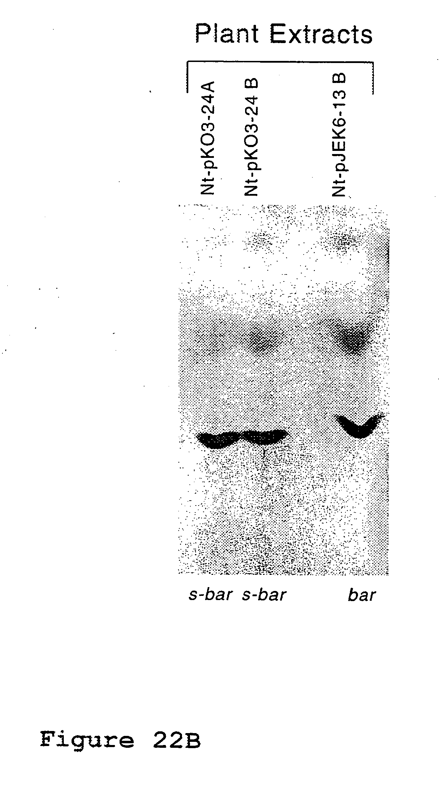

**Figure 22B****.** PAT assay confirms expression of bar and *s-bar* in tobacco plastids. PAT activity was determined by conversion of PPT into acetyl-PPT using radiolabeled ¹⁴C-Acetyl-CoA. Data are shown for transplastomic lines Nt-pJEK6-13B and Nt-pKO3-24a,B carrying *bar* and *s-bar,* respectively.

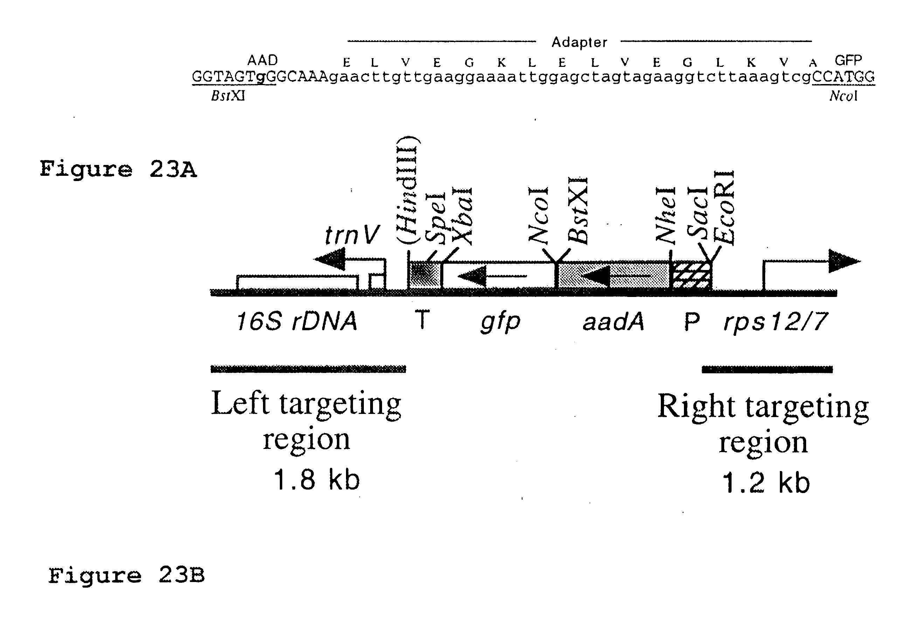

**Figure 23A****.** Plastid transformation vector with FLARE16-S as selectable marker targeting the plastid inverted repeat region. DNA and protein sequence at the *aadA-gfp* junction. Nucleotides derived from *aadA* and *gfp* are in capital, adapters sequences and the point mutation used to create the *BstXI* restriction site (bold) are in lower case.
**Figure 23B****.** Physical map of plastid transformation vector with FLARE16-S as selectable marker targeting the plastid inverted repeat region. Shown are: the promoter (P) and 3'UTR (T) of the *aadA16pt-gfp* coding region and its component parts (*aadA* and *gfp* coding regions); *rrn16* and *rps12*/*7* plastid genes; restriction endonuclease sites HindIII (removed), SpeI, XbaI, NcoI, BstXI, NheI, EcoRI. In plasmid pMSK56 *aadA16pt-gfp* is expressed from the Prrn:LatpBDB promoter and encodes FLARE16-S1. In plasmid pMSK57 *aadA16pt-gfp* is expressed from the Prrn:LrbcLDB promoter and encodes FLARE16-S2.

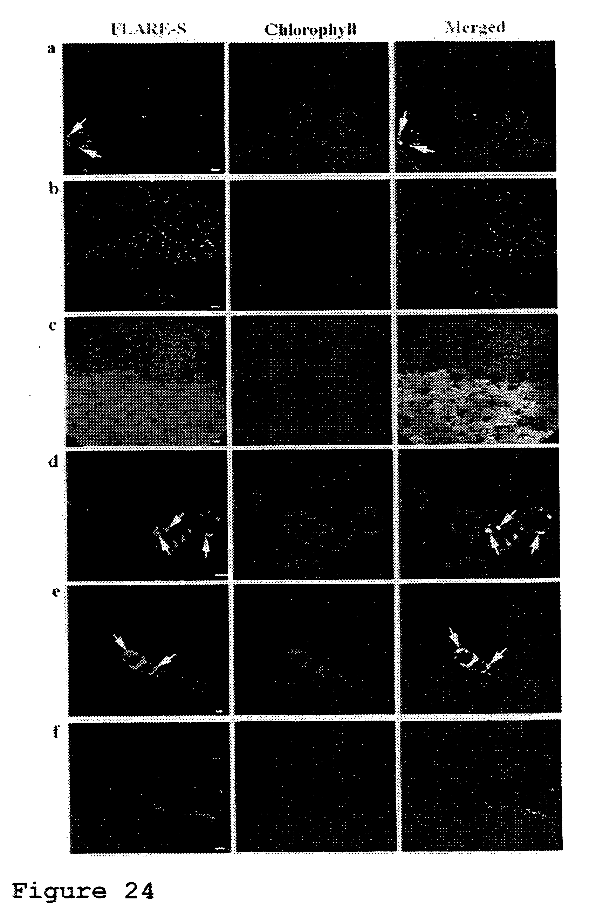

**Figure 24****.** Localization of FLARE16-S to tobacco plastids by laser scanning confocal microscopy in heteroplastomic tissue. Images were processed to detect FLARE16-S (green) and chlorophyll fluorescence (red) and both in a merged view. Sections are shown from plants expressing FLARE16-S1 (a,b) and FLARE16-S2 (3c-f). Note wild-type and transformed plastids in leaves (3a,c,d), chromoplasts of petals (3b), trichomes (3e) and non-green root plastids (f). White arrows mark transplastomic organelles. Bars represent 25 µm.

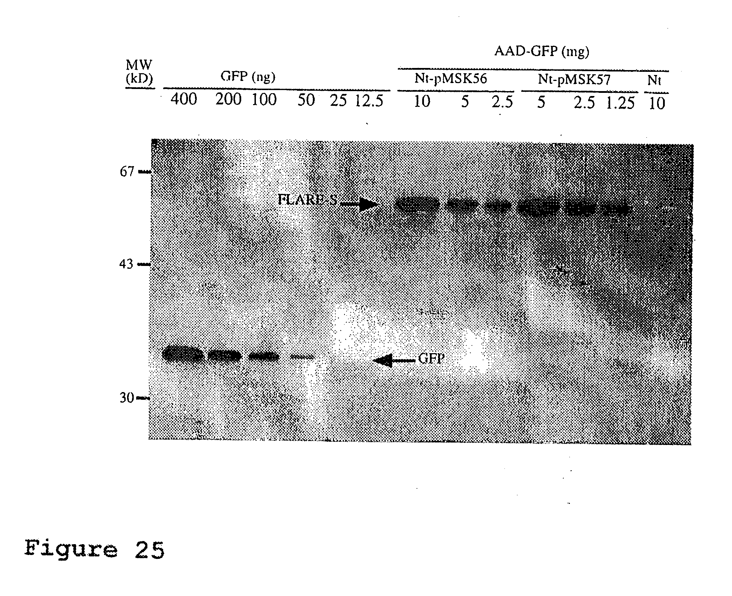

**Figure 25****.** Immunoblot analysis of FLARE16-S accumulation in chloroplasts. The amount of loaded protein (µg) is indicated above the lanes. Quantification of FLARE16-S1 (Nt-pMSK56 plants) and FLARE16-S2 (Nt-pMSK57 plants) is based on comparison with a purified GFP dilution series. Extract from a wild-type plant (Nt) was used as negative control.

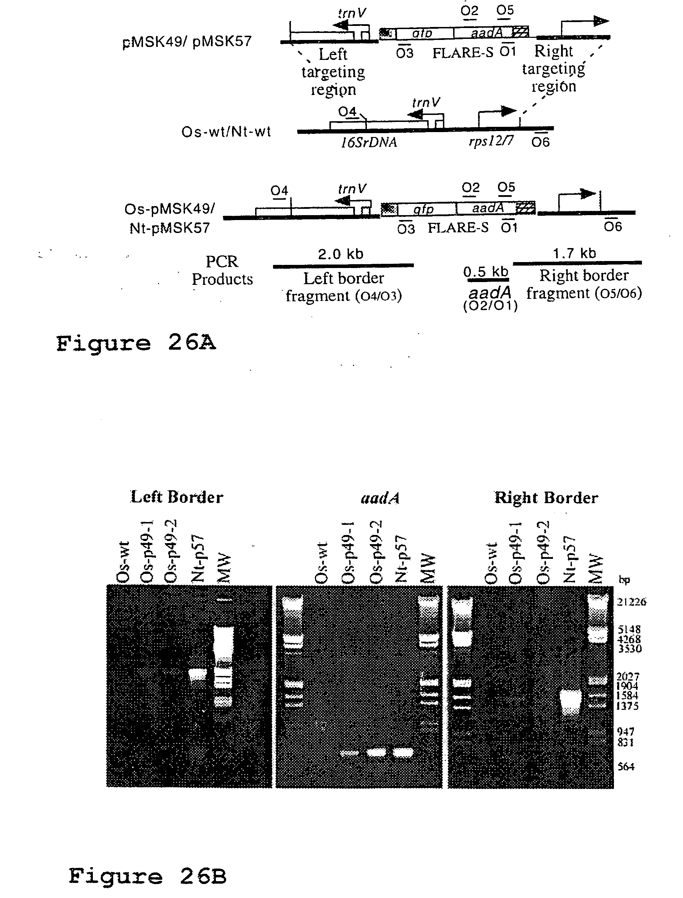

**Figure 26A****.** Amplification of border fragments confirms integration of FLARE-S genes into the plastid genome. Maps of the plastid targeting regions of the rice (pMSK49) and tobacco (pMSK57) vectors, the segment of the rice and tobacco plastid genomes targeted by the vectors (Os-wt and Nt-wt), and the same regions after integration of FLARE-S genes. The ends of plastid targeting regions are connected with cognate sequences in the wild-type plastid genome. Plastid genes *16SrDNA, trnV* and *rps12*/*7* are marked only in the wild-type plastid genomes. The position of PCR primers (01-06) and the PCR fragments generated by them are also shown.
**Figure 26B****.** Amplification of border fragments confirms integration of FLARE-S genes into the plastid genome. Gels with PCR-amplified left and right border fragments, and with *aadA* fragment. Results are shown for rice (Os-pMSK49-1 and Os-pMSK49-2) and tobacco (Nt-pMSK57) transplastomic lines and wild-type (Os-wt) rice. The molecular weight markers is EcoRI- and HindIII-digested λ DNA.

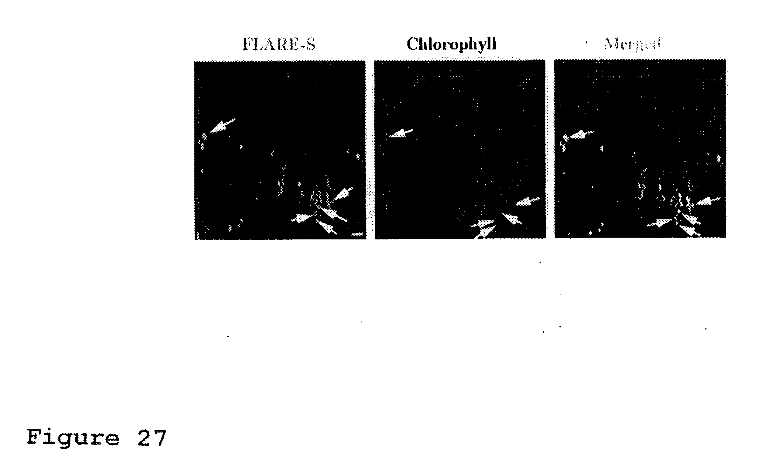

**Figure 27****.** Localization of FLARE11-S3 to rice chloroplasts in the Os-pMSK49-5 line by laser scanning confocal microscopy. Images were processed to detect FLARE11-S (green) and chlorophyll fluorescence (red) and both in a merged view. Arrows point to mixed populations of plastids in cells. Bar represents 25 µm.

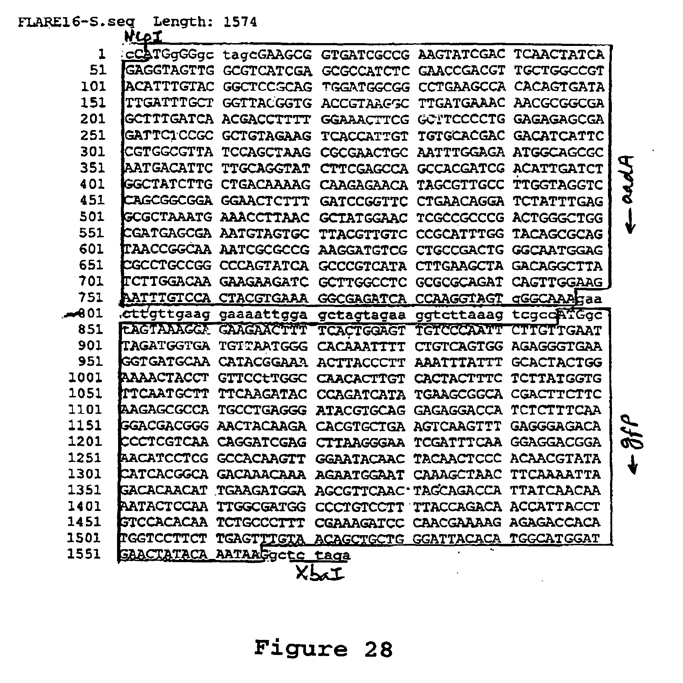

**Figure 28****.** The sequence of FLARE16-S is shown.

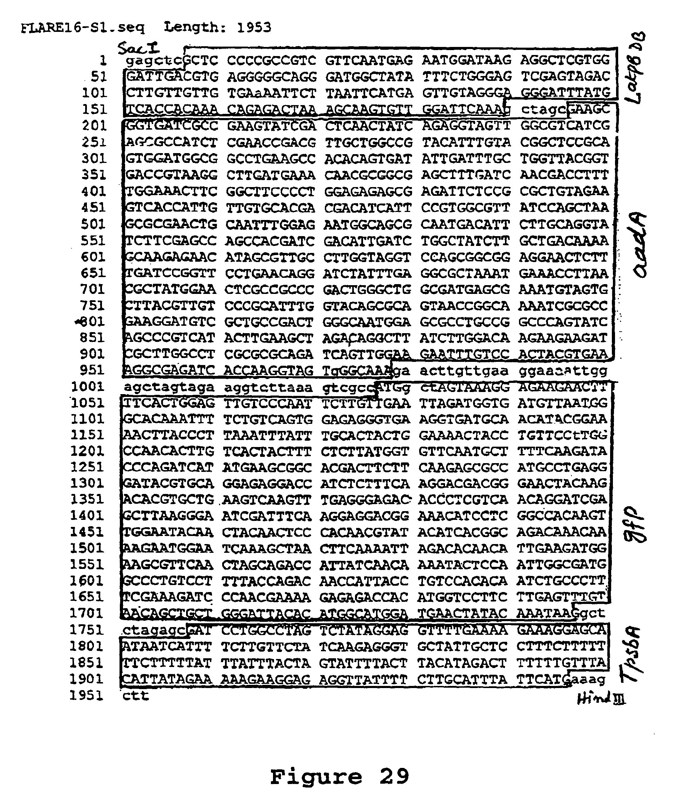

**Figure 29****.** The sequence of FLARE16-S1 is shown.

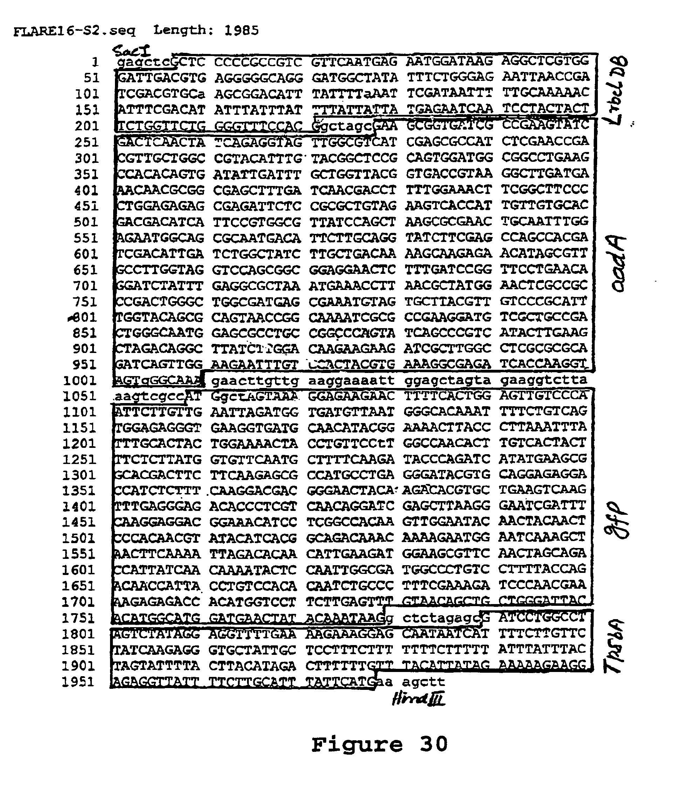

**Figure 30****.** The sequence of FLARE16-S2 is shown.

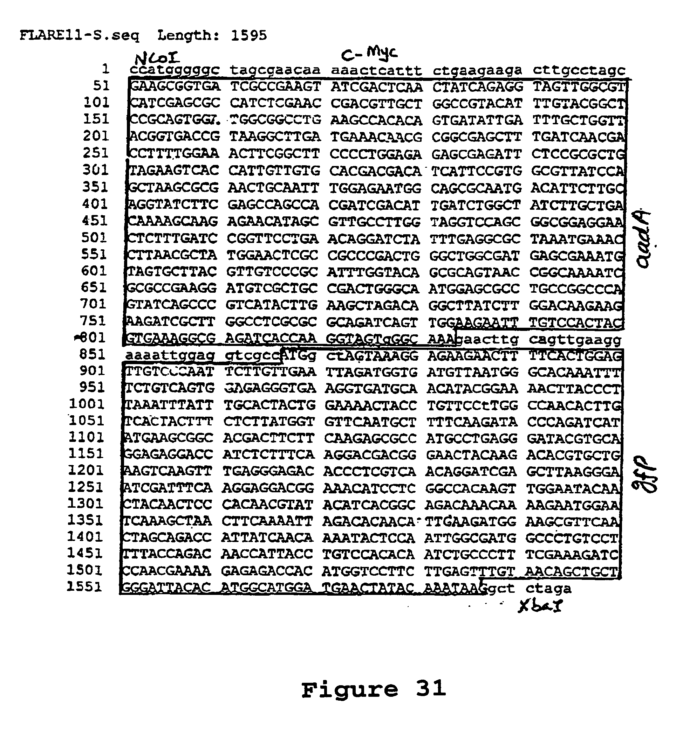

**Figure 31****.** The sequence of FLARE11-S is shown.

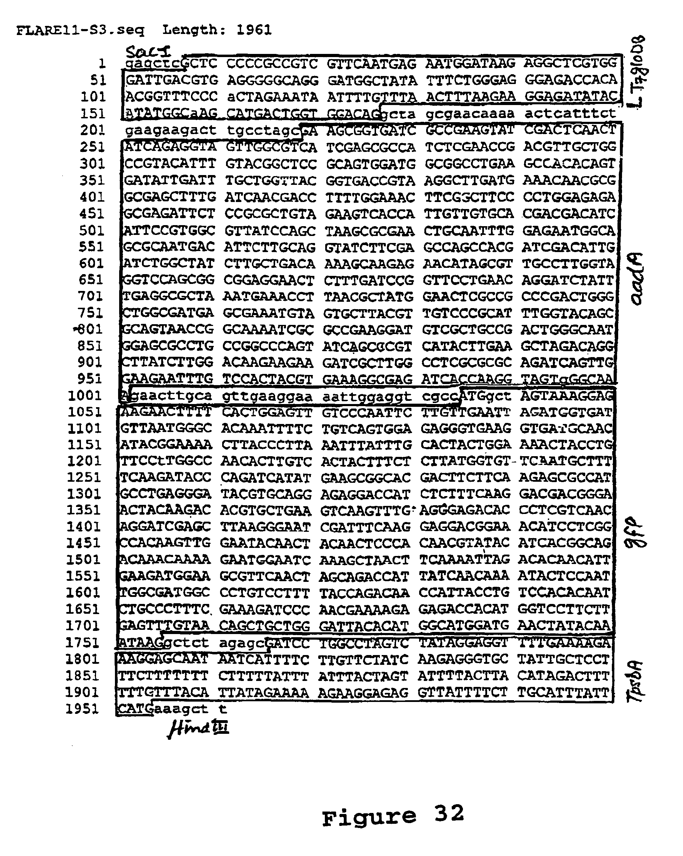

**Figure 32****.** The sequence of FLARE11-S3 is shown.

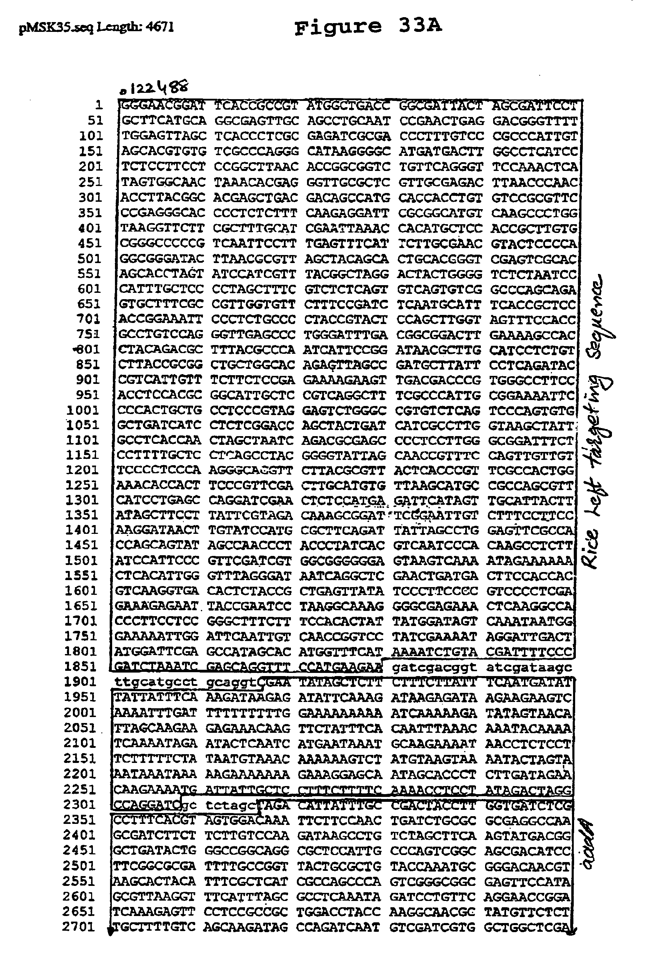

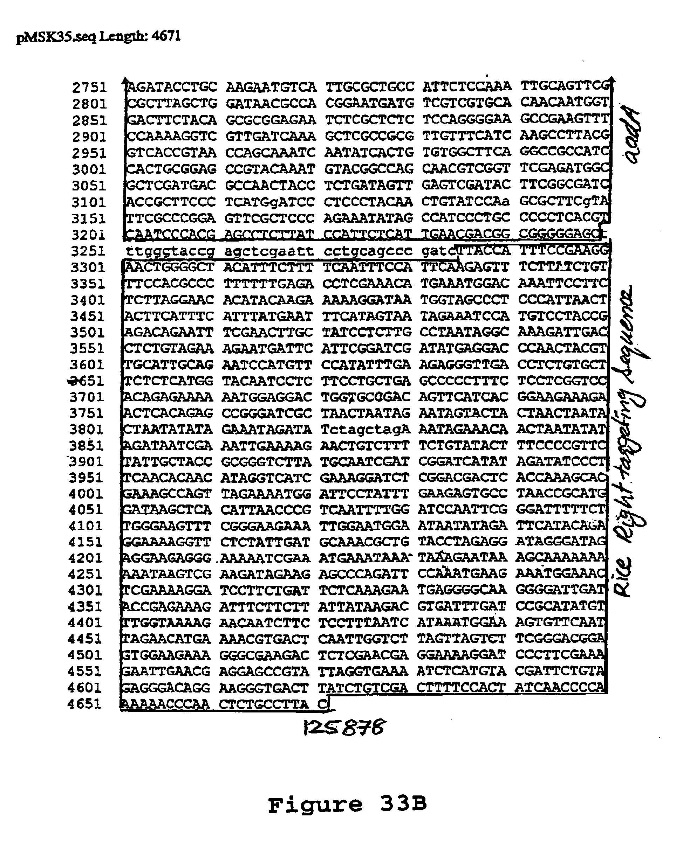

**Figures 33A** **and** **33B****.** The sequence of pMSK35 is shown.

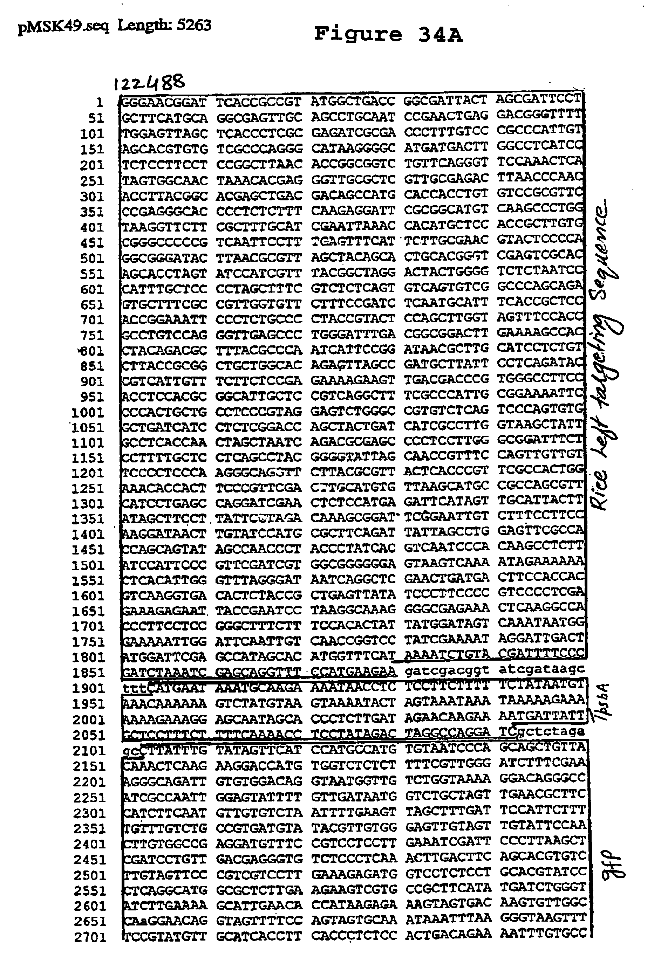

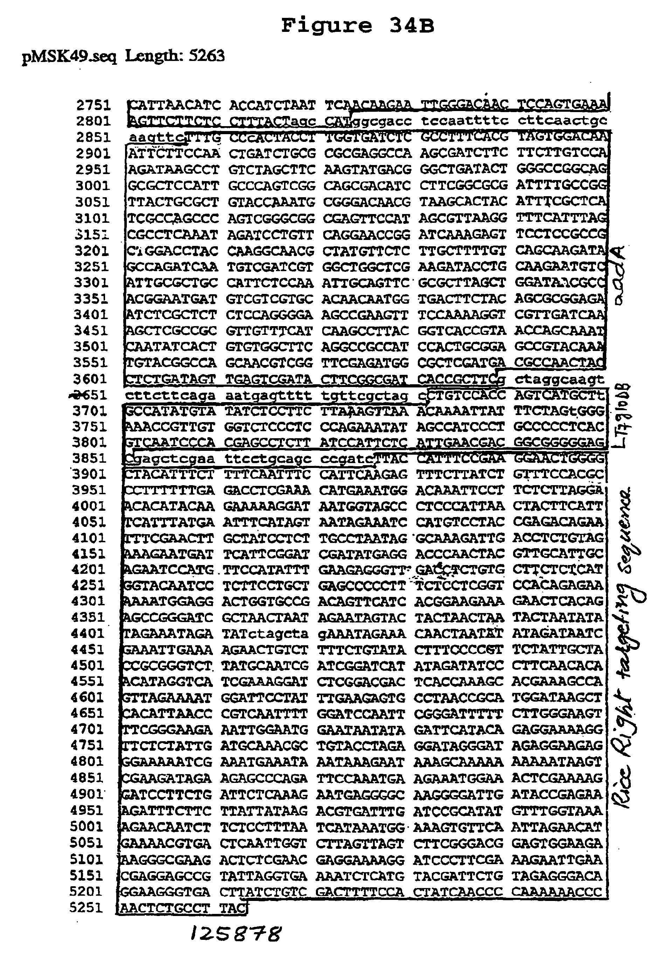

**Figures 34A** **and** **34B****.** The sequence of pMSK49 is shown.

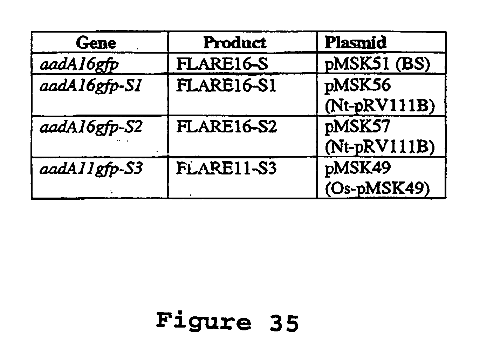

**Figure 35****.** A table describing the FLARE constructs of the invention.

### DETAILED DESCRIPTION OF THE INVENTION

DNA cassettes for high level protein expression in plastids are provided herein. Higher plant plastid mRNAs contain sequences within 50 nt downstream of AUG that are complementary to the 16S rRNA 3-region. These complementary sequences are approximately at the same position as DB sequences in *E. coli* mRNAs. See Figures 1A and 2A. Interestingly, the tentative plastid DB sequence significantly deviates from the *E. coli* DB consensus, since the tobacco plastid and *E. coli* 16S rRNA sequence in the anti-downstream-box (ADB) region is significantly different (Figure 1B). The feasibility of improving protein expression by incorporating DB sequences in plastids was assessed by constructing a series of chimeric 5' regulatory regions consisting of the plastid rRNA operon σ⁷⁰-type promoter (Prrn-114; Svab and Maliga, 1993; Vera and Sugiura, 1995) and the leader sequence of plastid mRNAs with the native DB, mutagenized DB and synthetic DB sequences. The plastid mRNA leaders differ with respect to the presence and position of the SD sequence. Translation efficiency from the chimeric promoters was determined by expressing the bacterial *neo* gene in plastids. The *neo* (or kan) gene encodes neomycin phosphotransferase (NPTII) and confers resistance to kanamycin in bacteria and plastids (Carrer et al., 1993). We have found that NPTII from the chimeric *neo* transcripts accumulates in the range of 0.2% to 23% of the total soluble leaf protein, indicating the importance of translational control signals in the mRNA 5' region for high-level protein expression.

There is great interest in producing recombinant proteins in plants plastids which, thus far have been expressed from nuclear genes only (Arntzen, 1997; Conrad and Fiedler, 1998; Kusnadi *et al.*, 1997). Protein levels produced from the PrrnLrbcL+DBwt and PrrnLT7g10 expression cassettes described here significantly exceed protein levels reported for nuclear genes. Accumulation of NPTII from nuclear genes is typically <<0.1% (Allen et al., 1996), the highest value being 0.4% of the total soluble protein (Houdt *et al.*, 1997). We reported earlier accumulation of 1% NPTII from a plastid neo transgene (Carrer *et al.*, 1993). Other examples for protein accumulation from plastid transgenes are 2.5% β-glucuronidase (GUS) (Staub and Maliga, 1993)) and 3-5% of the *Bacillus thuringiensis* (Bt) crystal toxins (McBride *et al.*, 1995). As compared to this earlier report, we have achieved a significant increase in NPTII levels, up to 23% of total soluble protein.

FLARE-S, a protein obtained by fusing an antibiotic-inactivating enzyme with the *Aequorea victoria* green fluorescence protein accumulated to 8% and 18% of total soluble protein from the PrrnLatpB+DBwt and PrrnLrbcL+DBwt cassettes provided herein. See Example 8. High-level protein accumulation from the cassettes of the present invention can be clearly attributed to engineering the translational control region (TCR) of the chimeric genes. These novel genetic elements may be used in different applications to drive expression of proteins with agronomic, industrial or pharmaceutical importance.

There is a strong demand for methods that control the flow of transgenes in field crops. Incorporation of the transgenes in the plastid genome rather than the nuclear genome results in natural transgene containment, since plastids are not transmitted via pollen in most crops (Maliga, 1993). Plastid transformation in crops has not been widely employed due to the lack of technology. Enhanced expression of selective markers should yield higher transformation efficiencies. The chimeric promoters of the present invention facilitate extension of plastid transformation to agronomically and industrially important crops. Indeed, high-level expression from the PrrnLatpB+DBwt cassette described here resulted in ∼25-fold increase in the frequency of kanamycin-resistant transplastomic tobacco lines. More importantly, high levels of marker gene expression following plastid transformation have been obtained in rice, the first cereal species in which plastid transformation has been successful. The results are set forth in Example 8.

The following examples are provided to illustrate various embodiments of the present invention. They are not intended to limit the invention in any way.

The protocols set forth below are provided to facilitate the practice of the present invention.

### PREPARATION OF CHIMERIC 5' CASSETTES FOR ELEVATED EXPRESSION OF HETEROLOGOUS PROTEINS IN PLASTIDS OF HIGHER PLANTS

### Identification of a potential downstream box in plastid mRNAs

The presence or absence of downstream box elements in mRNA molecules was determined for the following genes: *psbB* (Tanaka et al., 1987) and *psbA* (Sugita and Sugiura, 1984), photosystem II genes; *rbcL*, encoding the large subunit of ribulose-1,5-bisphosphate carboxylase/oxygenase (Shinozaki and Sugiura, 1982); *atpB*, encoding the ATPase β subunit (Orozco et al., 1990); and *clpP*, encoding the proteolytic subunit of the Clp ATP-dependent plastid protease (Hajdukiewicz et al., 1997). Interestingly, most or all of the PclpP-53 promoter is downstream of the transcription initiation site, therefore the PrrnLclpP constructs are assumed to contain two promoters: Prrn-114 and PclpP-53. Transcription initiation sites for these genes were described in references cited above; for nucleotide position of the genes in the plastid genome see Shinozaki et al., 1986.

Initially, it was assumed that the plastid ADB is similar in size and position as the *E. coli* ADB in the 16S rRNA. The *E. coli* ADB is localized on a conserved stem structure between nucleotides 1469 to 1483 (15 nt) that corresponds to nucleotides 1416 and 1430 of the plastid 16S rRNA (Dams *et al.*, 1988; Sprengart *et al.,* 1996). Although in both cases, the ADB is contained in the 16S rRNA penultimate stem, the actual ADB sequence is different in plastids and in *E. coli* (Figure 1B). The N-terminal coding regions of plastid genes *atpB, clpP, rbcL, petA, psaA, psbA, psbB, psbD* and *psbE* were searched for potential DB sequences. The homology search was carried out with a 26 nucleotide sequence centered on the tentative DB region (Figure 1B). The search revealed short stretches of imperfect homology with alternative solutions. Since the position of DB in the mRNA is quite flexible (Etchegaray and Inouye, 1999), we *show* four potential DB-ADB interactions for *atpB* and *rbcL* in Figure 2A. Two plastid mRNAs were selected to test the role of DB in the translation of plastid mRNAs: 1) *atpB* mRNA lacks a SD sequence; and 2) *rbcL* mRNA contains a SD sequence at the prokaryotic consensus. In addition, the phage T7 gene 10 (T7g10) leader was included in the study. This leader has a well-characterized *E. coli* DB sequence (Figure 2B; Sprengart *et al.,* 1996). Additional plastid mRNAs with potential DB sequences shown in Figure 2A are *clpP, psbB* and *psbA.*

### Experimental strategy to test the efficiency of leader sequences for translation

To compare the efficiency of translation from the 5'-UTR of the selected genes, the 5'-UTR was cloned downstream of the strong plastid rRNA operon σ⁷⁰-type promoter (Prrn-114) (Svab and Maliga, 1993; Allison et al., 1996), which initiates transcription from multiple adjacent nucleotides (-114, -113, -111; Sriraman et al., 1998). The promoter fragments were constructed as SacI-NheI or a SacI-NcoI fragments. Construction of the chimeric promoters using conventional molecular biological techniques is set forth in detail in the next section.

Two constructs were prepared for each 5'-UTR selected: one with (+DB) and one without (-DB) a native downstream box. It will be obvious from the forthcoming discussion, that the -DB constructs have a synthetic DB provided by the NheI restriction site. The promoters were cloned upstream of the coding region of a kanamycin resistance (*neo*) gene, which is available on an NheI-XbaI or NcoI-XbaI fragment. For the stabilization of the mRNA, the *rbcL* gene 3'-untranslated region was cloned downstream of *neo* as an XbaI-HindIII fragment. The chimeric *neo* genes can therefore be excised from the pUC118 or pBSIIKS+ plasmids as SacI-HindIII fragments. These source plasmids are listed in Table 1.

**Table 1. Salient features of chimeric promoters^{a.}**

| Source of 5'-UTR SD (nucleotides from AUG) | | | DB | Promoter fragment | pUC118(U) or pBSIIKS⁺(B) | pPRV111A, B |
|---|---|---|---|---|---|---|
| atpB | (-90/+42) | - | wt | SacI/NheI | pHK10 (U) | pHK30 (B) |
| atpB | (-90/+6) | - | s | SacI/NheI | pHK11 (U) | pHK31 (B) |
| atpB | (-90/42) | - | m | SacI/NheI | pHK50 (B) | pHK60 (B) |
| | | | | | | |
| clpP | (-53/+48) | - | wt | SacI/NheI | pHK12 (U) | pHK32 (B) |
| clpP | (-53/+6) | - | s | SacI/NheI | pHK13 (U) | pHK33 (B) |
| | | | | | | |
| rbcL | (-58/+42) | + | wt | SacI/NheI | pHK14 (B) | pHK34 (A) |
| rbcL | (-58/+6) | + | s | SacI/NheI | pHK15 (U) | pHK35 (A) |
| rbcL | (-58/+42) | + | m | SacI/NheI | pHK54 (B) | pHK64 (A) |
| | | | | | | |
| psbB | (-54/+45) | + | wt | SacI/NheI^{d} | pHK16 (U) | pHK36 (A) |
| psbB | (-54/+3) | + | s | SacI/NcoI^{d} | PHK17 (U) | pHK37(A) |
| | | | | | | |
| ^{b}T7g10+DB/Ec | (-63/+24) | + | Ec | SacI/NheI | pHK18 (B) | pHK38 (A) |
| ^{b}T7g10+DB/pt | (-63/+24) | + | pt | SacI/NheI | pHK19 (B) | pHK39 (A) |
| T7g10-DB | (-63/+9) | + | s | SacI/NheI | pHK20 (B) | pHK40 (A) |
| | | | | | | |
| psbA | (-85/+21) | - | wt | SacI/NheI | pHK21 (U) | pHK41 (A) |
| psbA | (-85/+3) | - | s | SacI/NcoI^{e} | pHK22 (U) | pHK42 (A) |
| ^{c}psbA(+GC) | (-85/+3) | | - | sSacI/NcoI^{e} | pHK23 (U) | pHK43(A) |

| | | | | | | |
|---|---|---|---|---|---|---|
| ^{a}SD+, SD at prokaryotic consensus position; SD-, no SD at prokaryotic consensus position; DB wt, wild-type; m, mutants; s, NheI site as synthetic DB. ^{b}Ec or pt refers to construct with *E. coli* or plastid DB sequence. ^{c}psbA(+GC) indicates addition of GC to the wild-type A at the mRNA 5'-end. ^{d}In source gene psbB translation initiation codon is within NcoI site; therefor +DB construct pHK16 has this NcoI site upstream of the NheI site; see Figure 9. ^{e}Translation initiation codon is included in NcoI site; NheI site is directly downstream in *kan* coding region; see Figure 8. | | | | | | |

The Prrn promoter fragment is available in plasmid pPRV100A (Zoubenko et al., 1994). The promoters were designed to include sequences between -197 nt and -114 nt upstream of the mature 16S rRNA 5' end. Nucleotide - 197 is the 5'-end of the Prrn promoter constructs utilized for these and other studies (Svab and Maliga. 1993; -1 is the first nucleotide upstream of the mature 16S rRNA). The G at the -114 position is one of three transcription initiation sites; the other two are the adjacent C (-113) and A (-111) nucleotides (Allison et al., 1996, Sriraman et al., 1998). The nucleotide at which Prrn transcription would initiate is marked by a filled circle in Figure 3A-D. In most constructs, this is a G (-114) as in the native promoter. In two constructs the G was replaced by an A, as in the *psbA* promoter which is the source of the leader sequence (pHK21, pHK22; see below).

### DESIGN OF THE 5' LEADER FROM atpB

For the *atpB* gene, multiple mRNA 5'-ends were mapped in tobacco leaves including at least four primary transcripts indicating transcription from four promoters and a processed 5'-end 90 nucleotides upstream of the translation initiation codon (Orozco et al., 1990). The terminal nucleotide of the processed *atpB* 5'-end is a G. Therefore, the chimeric PrrnLatpB promoters were designed to initiate transcription at a G, anticipating that the leader sequence of the chimeric transcript will be a perfect reproduction of the processed *atpB* mRNA 5'-end. Out of the *atpB* coding region, 42 and 6 nucleotides are included in the +DBwt and -DB constructs, respectively. The 42 nucleotides include four potential DB sequences shown in Figure 2A. Two point mutations in the leader sequence were designed to eliminate NheI (T to A) and EcoRI (G to A) restriction sites without affecting the predicted mRNA 5' secondary structure. In the -DB constructs, two codons (6 nucleotides) were retained from the native coding region upstream of the NheI restriction site (GCTAGC sequence) in which the stop codon is out-of-frame (Figure 3A). Eleven silent point mutations were introduced in the DB region of the PrrnLatpB+DBm construct to either minimize the number of base pairs, or to change the nature of base pairing (for example G-C to G-U) (Figure 2A; Figure 3A) .

### DESIGN OF THE 5' LEADER FROM clpP

Two major mRNA 5'-ends of the *clpP* gene were mapped in tobacco leaves (Hajdukiewicz et al., 1997). The terminal nucleotide of the proximal primary transcript is a G. Therefore, the chimeric PrrnLclpP promoters were designed to initiate transcription at a G, anticipating that the leader sequence of the chimeric transcript will be a perfect reproduction of the leader transcribed from the Pclp-53 NEP promoter. Out of the *clpP* coding region, 48 and 6 nucleotides are retained in the +DBwt and -DB constructs, respectively. The 48 nucleotides include four potential DB sequences as shown in Figure 2A. In the -DB constructs, two codons (6 nucleotides) were retained from the native coding region upstream of the NheI restriction site (GCTAGC sequence) in which the stop codon is out-of-frame.

### DESIGN OF THE 5' LEADER FROM rbcL

One primary and one processed mRNA 5'-end were mapped in tobacco leaves for the *rbcL* gene (Shinozaki and Sugiura, 1982). The terminal nucleotide of the processed 5' end is a T. The chimeric PrrnLrbcL promoters were designed to initiate transcription at a G, one nucleotide downstream of the terminal T. Forty-two and 6 nucleotides out of the *rbcL* coding region are included in the +DB and -DB constructs, respectively. The 42 nucleotides include four potential DB sequences as shown in Figure 2A. The one point mutation (G to A) in the leader sequence was designed to eliminate an EcoRI restriction site without affecting the predicted mRNA 5' secondary structure. In the -DB constructs, two codons (6 nucleotides) were retained from the native coding region upstream of the NheI restriction site (GCTAGC sequence) in which the stop codon is out-of-frame. Twelve silent point mutations were introduced into the DB region of the PrrnLrbcL+DBm construct to either minimize the number of base pairs, or to change the nature of base pairing (for example G-C to G-U) (Figure 2A, Figure 3B).

### DESIGN OF THE 5' LEADER FROM psbB

One primary and one processed mRNA 5'-end for the *psbB* gene were tentatively identified in tobacco leaves (Tanaka et al., 1987). The leader sequence was designed to initiate transcription from the G (-114) of the Prrn promoter, and include the intact secondary (stem) structure assumed to be involved in stabilizing the mRNA. Forty-five and 3 nucleotides out of the *psbB* coding region are included in the +DB and -DB constructs, respectively. The 45 nucleotides include four potential DB sequences shown in Figure 2A. Since the ATG is naturally included in an NcoI site that is used to fuse the *neo* coding region with the *psbB* leader, no amino acid from the *psbB* coding region is added in the -DB construct.

### DESIGN OF THE 5' LEADER FROM psbA

One mRNA 5'-end was mapped for the *psbA* gene in tobacco leaves (Sugita and Sugiura, 1984). The terminal nucleotide of the primary transcript is an A. Therefore, the chimeric PrrnLpsbA promoters were designed to initiate transcription at an A, anticipating that the leader sequence of the chimeric transcript will be a perfect reproduction of the leader transcribed from the *psbA* promoter. Twenty-one and 3 nucleotides out of the *psbA* coding region are included in the +DB and -DB constructs, respectively. The 21 nucleotides include the potential DB sequence as shown in Figure 2A. Since the neo coding region was linked to the chimeric promoter via an NcoI site which includes the translation initiation codon (ATG), no amino acid from the *psbA* coding region is added in the -DB constructs. This is true of a second -DB promoter, in plasmid PHK23, in which transcription is designed to initiate from the Prrn G (-114) and C (-113) (Figure 3C).

### DESIGN OF THE T7 PHAGE GENE 10 LEADER

The T7 phage gene 10 leader (63 nucleotides) was shown to promote efficient translation initiation in *E. coli* (Olins et al., 1988). This leader is used in the *E. coli* pET expression vectors (Studier et al., 1990; Novagen Inc.). The terminal nucleotide at the 5'-end is a G. Therefore, the chimeric PrrnT7g10L promoters were designed to initiate transcription at a G, anticipating that the leader sequence of the chimeric transcript will be a reproduction of the T7 phage gene 10 mRNA, with the exception of a T to A mutation which was introduced to eliminate an XbaI site. Twenty-four and 9 nucleotides from the T7 phage gene 10 coding region are included in the +DB/Ec (with *E. coli* DB sequence) and -DB constructs, respectively. To compare the efficiency of *E. coli* and plastid DB sequences in plastids, a second +DB promoter was constructed with the tobacco DB sequence (PrrnT7g10L+DB/pt). The native T7g10 leader has an NheI site directly downstream of the translation initiation codon. This NheI site was removed by a T to A point mutation in the +DB constructs (Figure 3D).

For introduction into the plastid genome, the chimeric neo genes were cloned into plastid transformation vector pPRV111A or pPRV111B. See U.S. Patent 5,877,402. The pPRV111 vectors target insertions into the inverted repeat region of the tobacco plastid genome, and carry a selectable spectinomcyin (*aadA*) resistance gene. The sequences of the vectors have been deposited in GenBank (U12812, U12813). The chimeric *neo* gene in vector pPRV111B is in tandem with the *aadA* gene, whereas in vector pPRV111A the chimeric neo is oriented divergently. The general outline of the plastid transformation vector with the chimeric neo genes is shown in Figures 4A and 4B.

### CONSTRUCTION OF CHIMERIC Prnn PROMOTERS WITH PLASTID MRNA LEADERS

The chimeric Prrn promoter/leader fragments were constructed as a SacI-NheI or SacI-NcoI fragments (Table 1, below) by overlap extension PCR (SOE-PCR), essentially as described in Lefebvre et al., (1995). Construction of the Prrn-plastid leader segments is schematically shown in Figure 5. The objective of the PCR-1 step is to 1) amplify the Prrn promoter fragment while 2) adding a SacI site upstream and a seam-less overlap with the specific downstream leader sequence. The reaction contains: 1) a primer (oligonucleotide) to add a SacI site at the 5'-end of the fragment; 2) a suitable template containing the Prrn promoter sequence in plasmid pPRV100A (Zoubenko et al., 1994); and 3) a primer to add on the overlap with the leader sequence at the 3' of the amplified product. The objective of the PCR-2 step is to create the chimeric promoter with DB sequence using: 1) the product of PCR-1 step as a primer; 2) a suitable DNA template containing the specific leader sequence; and 3) primer (oligonucleotide) to include NheI restriction site at the 3'-end of the amplification product. The product of the PCR-2 is the SacI-NheI chimeric Prrn promoter fragment with DB sequence. The objective of the PCR-3 step is to remove the DB sequence while introducing a suitable NheI or NcoI restriction site. The product of PCR-3 is the SacI-NheI or SacI-NcoI chimeric Prrn promoter fragment in which the DB sequence is replaced with the NheI site. The objective of the PCR-4 step is to replace the wild-type DB with a mutant DB. The product of PCR-4 is a SacI-NheI Prrn promoter fragment.

The primers (oligonucleotides) used for the construction of chimeric promoters are listed in Table 2. The chimeric promoters were obtained by overlap extension PCR using oligonucleotides and DNA templates schematically shown in Figure 5.

**Table 2.**

| **Oligonucleotides used for the construction of chimeric promoters.** | |
|---|---|
| #1: | 5'-CCCGAGCTCGCTCCCCCGCCGTCGTTC-3' |
| #2: | 5'- CGAATTTAAAATAAATGTCCGCTTGCACGTCGATCGGTTAATTCTCCCAGAAATATAGCCATCC-3' |
| #3: | 5'-CCCGCTAGCCGTGGAAACCCCAGAACC-3' |
| #4: | 5'-CCCGCTAGCTCTCATAATAATAAAATAAATAAATATGTC-3' |
| #5: | 5'-TCACTTTGAGGTGGAAACGTAACTCCCAGAAATATAGCCATCC-3' |
| #6: | 5'-CCCGCTAGCTTCCTCTCCAGGACTTCG-3' |
| #7: | 5'-CCCGCTAGCAGGCATTAAATGAAAGAAAGAAC-3' |
| #8: | 5'-TAAGAATTTTCACAACAACAAGGTCTACTCGACTCCCAGAAATATAGCCATCC-3' |
| #9: | 5'-CCCGCTAGCTTTGAATCCAACACTTGCTTTAG-3' |
| #10: | 5'-CCCGCTAGCTGACATAAATCCCTCCCTAC-3' |
| #11: | 5'-CAAAGATAAATAGACACTACGTAACTTTATTGCATTGCTCCCAGAAATATAGCCATCC- 3' |
| #12: | 5'-CCCGCTAGCATCATTCAATACAACGGTATGAACACG-3' |
| #13: | 5'-TTCTAGTGGGAAACCGTTGTGGTCTCCCTCCCAGAAATATAGCCATCC-3' |
| #14: | 5'-CCCGCTAGCCATATGTATATCTCCTTCTTAAAG-3' |
| #15: | 5'-CCCGCTAGCCTGTCCACCAGTCATGCTTGCCATA-3' |
| #16: | 5'-CCCGCTAGCCAAGGCAGGGCTAGTGATTGCCATATGTATATCTCCTTC-3' |
| #17: | 5'-TTTGTTTAACTTTAAGAAGGAGATATACATATGGCAAGCATGACTGGTGG-3' |
| #18: | 5'-CTCCTTCTTAAAGTTAAACAAAATTATTTCTAGTGGGAAACCGTTGT-3' |
| #19: | 5'-CAAAATAGAAAATGGAAGGCTTTTTGCTCCCAGAAATATAGCCATCCC-3' |
| #20: | 5'-CAAAATAGAAAATGGAAGGCTTTTTTCCCAGAAATATAGCCATCCC-3 3 |
| #21: | 5'-GGGCCATGGTAAAATCTTGGTTTATTTAATC-3' |
| #22: | 5'-GGGGCTAGCTCTCTCTAAAATTGCAGT-3' |
| #23: | 5'-GAATAGCCTCTCCACCCA-3' |
| #24: | 5'-CCCGCTAGCCGTGGACACCCCACTTCCACTTGTTGTCGGGTTTATTCTCAT-3' |
| #25: | 5'-CCCGCTAGCTTTGAATCCTACTGAGGCTTTTGTTTCTGTTTGAGGACTCAT-3' |

### CONSTRUCTION OF CHIMERIC Prnn PROMOTER/atpB LEADER SEGMENTS

PrrnLatpB+DBwt in plasmid pHK10 (Product of PCR-2)
PrrnLatpB-DB in plasmid pHK11 (Product of PCR-3)
PrrnLatpB+DBm in plasmid pHK50 (Product of PCR-4)
PCR-1: Oligonucleotides #1, #2 as primers; plasmid pPRV100A (Zoubenko et al., 1994) DNA as template.
PCR-2: Product of PCR-1 step, Oligonucleotide #3 as primers; plasmid pIK79 (see below) DNA as template.
PCR-3: Oligonucleotide #1, #4 as primers; Product of PCR-2 step as template.
PCR-4: Oligonucleotide #1, #24 as primers; Product of PCR-2 step as template.
Plasmid pIK79 is a Bluescript BS+ phagemid derivative which carries a PvuII/XhoI tobacco plastid DNA fragment between nucleotides 55147-60484 containing the rbcL-atpB intergenic region with divergent promoters for these genes (Shinozaki et al., 1986).

### CONSTRUCTION OF CHIMERIC Prnn PROMOTER/clpP LEADER SEGMENTS

PrrnLclpP+DBwt in plasmid pHK12 (Product of PCR-2)
PrrnLclpP-DB in plasmid pHK13 (Product of PCR-3)
PCR-1: Oligonucleotides #1, #5 as primers; plasmid pPRV100A (Zoubenko et al., 1994) DNA as template.
PCR-2: Product of PCR-1 step, Oligo #6 as primers; tobacco Sa18 ptDNA fragment (Shinozaki et al., 1986) as template.
PCR-3: Oligonucleotide #1, #7 as primers; Product of PCR-2 step as template.

### CONSTRUCTION OF CHIMERIC Prnn PROMOTER/rbcL LEADER SEGMENTS

PrrnLrbcL+DBwt in plasmid pHK14 (Product of PCR-2)
PrrnLrbcL-DB in plasmid pHK15 (Product of PCR-3)
PrrnLrbcL+DBm in plasmid pHK54 (Product of PCR-4)
PCR-1: Oligonucleotides #1, #8 as primers; plasmid pPRV100A (Zoubenko et al., 1994) DNA as template.
PCR-2: Product of PCR-1 step, Oligonucleotide #9 as primers; plasmid pIK79 DNA (see description of pHK10 above) as template.
PCR-3: Oligonucleotide #1, #10 as primers; Product of PCR-2 step as template.
PCR-4: Oligonucleotide #1, #25 as primers; Product of PCR-2 step as template.

### CONSTRUCTION OF CHIMERIC Prnn PROMOTER/psbB LEADER SEGMENTS

PrrnLpsbB+DBwt in plasmid pHK16 (Product of PCR-2)
PrrnLpsbB-DB in plasmid pHK17 (Promoter from pHK16, digested with SacI/NcoI)
PCR-1: Oligonucleotides #1, #11 as primers; plasmid pPRV100A (Zoubenko et al., 1994) DNA as template.
PCR-2: Product of PCR-1 step, Oligo #12 as primers; tobacco Sa18 ptDNA fragment (Shinozaki et al., 1986) as template.
PCR-3 was not necessary, since the psbB translation initiation codon is naturally included in an NcoI site. Therefore, the -DB derivative could be obtained by SacI/NcoI digestion of the PCR-2 step.

### CONSTRUCTION OF CHIMERIC Prnn PROMOTER/psbA LEADER SEGMENTS

PrrnLpsbA+DBwt in plasmid pHK21 (Product of PCR-2)
PrrnLpsbA -DB in plasmid pHK22 (Product of PCR-3)
PCR-1: Oligonucleotides #1, #20 as primers; plasmid pPRV100A (Zoubenko et al., 1994) DNA as template.
PCR-2: Product of PCR-1 step, Oligo #22 as primers; tobacco Sa13 ptDNA fragment (Shinozaki et al., 1986) as template.
PCR-3: Oligonucleotide #1, #21 as primers; Product of PCR-2 step as template.

PrrnLpsbA(GC) -DB in plasmid pHK23 (Product of PCR-2)
PCR-1: Oligonucleotides #1, #19 as primers; plasmid pPRV100A (Zoubenko et al., 1994) DNA as template.
PCR-2: Product of PCR-1 step, Oligo #21 as primers; tobacco Sal3 ptDNA fragment (Shinozaki et al., 1986) as template.

In all of the above, PCR amplification was carried out with AmpliTaq DNA polymerase (Perkin Elmer) or Pfu DNA polymerase (Stratagene) and "stepdown" PCR that utilizes gradually decreasing annealing temperatures was performed (Hecker and Roux, 1996). The exact amplification conditions for the chimeric Prrn::LatpB promoters are given below. The amplification conditions for the remaining chimeric Prrn - plastid leader promoters were calculated according to Hecker and Roux (1996), and differ only in the annealing temperatures. Description of PCR conditions for the construction of the chimeric Prrn promoters with plastid mRNA leaders is given below; for interpretation of individual steps see scheme in Figure 5.

**PCR-1 Program: 50 picomoles of both primers per 100 µl**

| | | | |
|---|---|---|---|
| 1.1 | Denature | 5 min. at 94 °C | |
| 2.1 | Denature | 1 min. at 94 °C | |
| 2.2 | Annealing | 0.5 min. at 72 °C | 3 cycles |
| 2.3 | Extension | 0.5 min. at 72 °C | |
| 3.1 | Denature | 1 min. at 94 °C | |
| 3.2 | Annealing | 0.5 min. at 69 °C | 3 cycles |
| 3.3 | Extension | 0.5 min. at 72 °C | |
| 4.1 | Denature | 1 min. at 94 °C | |
| 4.2 | Annealing | 0.5 min. at 66 °C | 3 cycles |
| 4.3 | Extension | 0.5 min. at 72 °C | |
| 5.1 | Denature | 1 min. at 94 °C | |
| 5.2 | Annealing | 0.5 min. at 63 °C | 3 cycles |
| 5.3 | Extension | 0.5 min. at 72 °C | |
| 6.1 | Denature | 1 min. at 94 °C | |
| 6.2 | Annealing | 0.5 min. at 60 °C | 3 cycles |
| 6.3 | Extension | 0.5 min. at 72 °C | |
| 7.1 | Denature | 1 min. at 94 °C | |
| 7.2 | Annealing | 0.5 min. at 57 °C | 20 cycles |
| 7.3 | Extension | 0.5 min. at 72 °C | |
| 8.1 | Extension | 10 min. at 72 °C | |
| 8.2 | | 1 min. at 30 °C | |

The PCR-2 program was essentially identical to the PCR1 program set forth above with the following modifications: 1) Primers in 100 µl were the products of lst PCR reaction, 50 picomoles of the oligonucleotide primer were used; and 2) the annealing temperature in stepdown PCR was from 67 °C to 52 °C. Accordingly, the following annealing temperatures were used: Step 2.2, 67 °C; Step 3.2, 64 °C; Step 4.2, 61 °C; Step 5.2, 58 °C; Step 6.2, 55 °C; Step 7.2, 52 °C.
The PCR-3 and PCR-4 programs were essentially identical to the PCR1 program with the following modification: 1) The annealing temperature in stepdown PCR was from 69 °C to 44 °C. Accordingly, the following annealing temperatures were used: Step 2.2, 69 °C; Step 3.2, 64 °C; Step 4.2, 59 °C; Step 5.2, 54 °C; Step 6.2, 49 °C; Step 7.2, 44 °C. In cases where the yield of the final PCR reaction was too low for efficient cloning, final product was amplified using primers which were used to generate the ends. The final PCR products were digested with the appropriate restriction enzymes (SacI and NheI or SacI and NcoI) and cloned in plasmids pHK2 or pHK3 (see below).

### CONSTRUCTION OF CHIMERIC PROMOTERS WITH T7 PHAGE GENE 10 mRNA LEADER SEGMENT

The chimeric Prrn promoter/T7gene10 leader (PrrnLT7g10) fragments were constructed as SacI-NheI fragments (Table 1, below).

### PrrnLT7g10+DB/Ec promoter in plasmid pHK18

In the absence of a proper DNA template, the PrrnLT7g10+DB/Ec was constructed by employing a modified polymerase chain reaction (Uchida, 1992) in two PCR steps, as schematically shown in Figure 6. The PCR-1A and PCR1B steps generate two fragments in two separate reactions (A and B). The objective of the PCR-1A step is to amplify Prrn promoter fragment while: 1) adding a SacI site upstream (Oligonucleotide #1 in Table 2); and 2) a seam-less overlap with the specific downstream leader sequence (Oligonucleotide #13 in Table 2) using plasmid pPRV100A (Zoubenko et al., 1994) as DNA template. The objective of the PCR-1B step is to amplify part of the T7g10 leader sequence using overlapping oligonucleotides #15 and #17 in Table 2. The NheI site is introduced in oligonucleotide #15. Both PCR-1A and PCR-1B reactions were carried out by stepdown PCR as described above for the construction of the chimeric Prrn promoters.

PCR-2 reaction generating this chimeric promoter contained:
a) The products of the PCR-1A and PCR-1B reactions as DNA templates;
b) Oligonucleotide #18 (0.5 picomole; Table 2) to generate overlapping fragments with products of the PCR-1A and PCR-1B reactions;
c) Oligonucleotides #1 and #15 (Table 2) for amplification of the final product, 50 picomoles each in 100 µl final volume.

Promoter was amplified by stepdown PCR, as described for the chimeric Prrn promoters above; the annealing temperatures were between 72 °C to 57 °C.

### PrrnLT7g10+DB/pt promoter in plasmid pHK19

The promoter fragment was obtained in one PCR step as shown in Figure 7. The reaction contained:
a) The product of the PCR-2 reaction generating promoter PrrnLT7g10+DB/Ec in plasmid pHK18 as DNA template; and
b) Oligonucleotides #1 and #16 (Table 2), 50 picomoles each in 100 µl final volume.

Promoter was amplified by stepdown PCR, as described for the construction of chimeric Prrn promoters above; the annealing temperatures were between 72 °C to 52 °C.

### PrrnLT7g10-DB promoter in plasmid pHK20

The promoter fragment was obtained in one PCR step, which is similar to the PCR-3 step in Figure 5. The reaction contained:
a) The product of the PCR-2 reaction generating promoter PrrnLT7g10+DB/Ec in plasmid pHK18 as DNA template; and
b) Oligonucleotides #1 and #14 (Table 2), 50 picomoles each in 100 µl final volume.

Promoter was amplified by stepdown PCR, as described for the chimeric Prrn promoters above; the annealing temperatures were between 72 °C to 52 °C.

The final PCR products were digested with the SacI and NheI restriction enzymes and cloned in plasmid pHK3 to obtain plasmids pHK18, pHK19, pHK20.

### Construction of chimeric neo genes

Construction of the chimeric promoters was described in the preceding sections. For determining effects on levels of protein accumulation, the promoters were cloned upstream of a kanamycin-resistance encoding construct, consisting of the *neo* coding region and the 3'-UTR of the plastid *rbcL* gene. Such constructs are available in plasmids pHK2 and pHK3, which carry the same Prrn(L)rbcL(S)::*neo*::TrbcL gene as a SacI-HindIII fragment. Plasmid pHK2 is a pUC118 vector derivative; pHK3 is a pBSIIKS+ derivative. Plasmid maps with relevant restriction sites are shown in Figure 8. DNA sequence of the neo gene in plasmids pHK2 and pHK3 is shown in Figure 9. Note, that in plasmid pHK2 the neo gene has an EcoRI site upstream of the SacI site (Figure 8). Prrn and TrbcL have been described by Staub and Maliga, 1994; the *neo* gene derives from plasmid pSC1 (Chaudhuri and Maliga, 1996). The pUC118 and pBSIIKS+ plasmid derivatives which carry the various promoter constructs are listed in Table 1.

To determine the DNA sequence of the promoter fragments, the plasmids were purified with the QIAGEN Plasmid Purification Kit following the manufacturer's recommendations. DNA sequencing was carried out using a T7 DNA sequencing kit (version 2.0 DNA , Amersham Cat. No. US70770) and primer No. #23 in Table 2, which is complementary to the neo coding sequence. These promoter sequences are shown in Figure 3A-D.

### Introduction of chimeric neo genes into the tobacco plastid genome

Suitable vectors are available for the introduction of foreign genes into the tobacco plastid genome. Such vectors are pPRV111A and pPRV111B, which carry a selectable spectinomycin-resistance (*aadA*) gene and target insertions into the repeated region of the plastid genome (Zoubenko et al., 1994). The chimeric neo genes were cloned into one of these plastid transformation vectors (Table 1) and introduced into the tobacco plastid genome by the biolistic process. From the transformed cells plants were regenerated by standard protocols (Svab and Maliga, 1993). A uniform population of transformed plastid genome copies was confirmed by Southern analysis.

For Southern analysis, total cellular DNA was prepared by the CTAB method (Saghai-Maroof et al., 1984). Two leaves of each transformed plant were homogenized and incubated at 60°C for 30 minutes in a buffer containing 2% CTAB (tetradecyl-trimethyl-ammonium bromide), 1.4 M NaCl, 20 mM EDTA (pH 8.0), 1 mM Tris/HCl (pH 8.0) and 100 mM β-mercaptoethanol. After chloroform extraction, the DNA was precipitated with isopropyl alcohol and dissolved in water or in TE buffer (10 mM Tris, 1 mM EDTA, pH 8.0). DNA digested with an appropriate restriction enzyme was electrophoresed on 0.8% agarose gel and transferred to nylon membrane using PosiBlot Transfer apparatus (Stratagene). The blots were probed using Rapid Hybridization Buffer and plastid targeting sequences as a probe labeled with random priming (³²P, Boehringer Mannheim Cat No. 1004760).

Plastid transformation was achieved with each of the plasmids listed in Table 1. Exceptions were plasmids pHK41 and pHK42. It appears that NPTII expression with the psbA leader derivatives was so high that the plants were not viable. It follows that these same leaders may be used to advantage when fused with weaker promoters.

Transplastomic lines are designated by Nt (N. tabacum, the species), the plasmid name (for example pHK30) and an individual line number and a letter identifying regenerated plants. For example, the Nt-pHK30-1D and Nt-pHK30-1C plants were both obtained by transformation with plasmid pHK30, are derived from the same transformation event and were regenerated from the same culture. Nt-pHK30-2 plants are derived from an independent transformation event. Normally, several transformed lines per construct were obtained. However, data are shown here only for one: Nt-pHK30-1D, Nt-pHK31-1C, Nt-pHK60-5A, Nt-pHK32-2F, Nt-pHK33-2A, Nt-pHK34-9C, Nt-pHK35-4A, Nt-pHK64-3A, Nt-pHK36-1C, Nt-pHK37-2D, Nt-pHK38-2E, Nt-pHK39-3B, Nt-pHK40-12B and Nt-pHK43-1C.

### Testing mRNA accumulation by RNA gel blot (Northern) analysis

RNA gel blot analysis was performed to determine steady-state levels of chimeric mRNA in the transplastomic lines. Total leaf RNA was prepared from the leaves and roots of plants grown in sterile culture according to Stiekema et al (1988). RNA (4 µg per lane) was electrophoresed on 1% agarose gel and transferred to nylon membranes using the PosiBlot Transfer apparatus (Stratagene). The blots were probed using Rapid Hybridization Buffer Amersham) with a ³²P-labeled neo probe (Pharmacia, Ready-To-Go Random Priming Kit). The *neo* probe was obtained by isolating the NheI/XbaI fragment from plasmid pHK2. The template for probing the tobacco cytoplasmic 25S rRNA was a fragment which was PCR amplified from total tobacco cellular DNA with primers 5'-TCACCTGCCGAATCAACTAGC-3' and 5'-GACTTCCCTTGCCTACATTG-3'. RNA hybridization signals were quantified using a Molecular Dynamics PhosphorImager, and normalized to the 25S rRNA signal.

### Testing NPTII accumulation by protein gel blot (Western) analysis

Total soluble protein was extracted from the leaves, roots or seeds of transgenic tobacco plants grown in sterile culture. In case of leaves grown in sterile culture, about 200 mg leaf tissue was homogenized in 1 ml of buffer containing 50 mM Hepes/KOH (pH 7.5), 1 mM EDTA, 10 mM potassium acetate, 5 mM magnesium acetate, 1 mM dithiothreitol and 2 mM PMSF. The homogenate was centrifuged twice at 4 °C to remove insoluble material. Protein concentration was determined using the Biorad Protein Assay reagent kit. Transgenic tobacco plants expressing *neo* in the plastid genome (Nt-pTNH32-70, Carrer et al., 1993) and wild type plants were used as positive and negative controls, respectively. Proteins were separated in SDS polyacrylamide gels (SDS-PAGE; 15% acrylamide, 6 M urea) and transferred to nitrocellulose membranes using a semi-dry transfer apparatus (Bio-Rad). After blocking non-specific binding sites, the membrane was incubated with 4,000-fold diluted polyclonal rabbit antiserum raised against NPTII (5Prime→3Prime Inc.). HRP-conjugated secondary antibody, diluted 20,000 fold, and ECL chemiluminescence were used for immunoblot detection on X-ray film. NPTII was quantified on the immunoblots by comparison of the experimental samples with a dilution series of commercial NPTII (SPrime→3Prime).

### EXAMPLE 1

### DB sequences enhance protein accumulation from rbcL leader; protein accumulation from the atpB translation control signals is high but DB-independent

The role of DB sequences in mRNA translation was tested using *neo* as the reporter gene. The *neo* gene encodes the bacterial enzyme neomycin phosphotransferase (NPTII) (Beck *et al.,* 1982). The tested *neo* genes have the same promoter (Prrn) and transcription terminator (TrbcL), and differ only with respect to the translation control region (TCR) comprising the 5' untranslated region of the mRNA and the coding region N-terminus. Two constructs were prepared with the *atpB* and *rbcL* TCRs. One construct contained the wild-type TCR, including the processed 5' untranslated region and 42 nucleotides of the coding region N-terminus (PrrnLatpB+DBwt, plasmid pHK30, Figure 4B; PrrnLrbcL+DBwt, plasmid pHK34, Figure 4A). The second construct contained silent mutations in the 42-nucleotide segment of the *atpB* and *rbcL* N-terminal coding regions to either eliminate or alter mRNA and rRNA base pairing (PrrnLatpB+DBm plasmids pHK60, Figure 2A and Figure 4B; PrrnLrbcL+DBm, pHK64, Figure 2A and Figure. 4A). The silent mutations altered the mRNA sequence without effecting the amino acid sequence. For example, 13 potential base pairs may form between the wild-type *atpB* mRNA and the ADB sequence shown at the bottom in Figure 2A. The 11 silent mutations affect eight base-paring events for this particular ADB-DB interaction. After mutagenesis, there is a possibility for ten base pairing events, most of which are new. The chimeric neo genes were introduced into the tobacco plastid genome by homologous targeting using the biolistic approach (Svab and Maliga, 1993; Zoubenko *et al.*, 1994). NPTII and neo mRNA levels were then assessed in the leaves of transplastomic plants. Since NPTII in wild-type DB-containing and mutant DB-containing plants has the exact same protein sequence, protein levels in the plants directly reflect the efficiency of mRNA translation. In case of the *atpB* TCR, mutagenesis of DB reduced protein accumulation to ~4% instead of ∼7% (Figure 10 and Table 3). In contrast, mutagenesis of *rbcL* DB had a dramatic effect, reducing NPTII accumulation 35-fold. Thus, DB-ADB interaction is very important for translation of the plastid *rbcL* mRNA, but is less important for translation of the *atpB* mRNA.

We also prepared a third construct set with the *atpB* and *rbcL* leaders, but without the native DB (PrrnLatpB-DB, plasmid pHK31, Figure 4B; PrrnLrbcL-DB, plasmid pHK35, Figure 4A). The *neo* coding region in these constructs is directly linked to the Prrn promoter via a synthetic *Nhe*I restriction site. The *Nhe*I restriction site (GCTAGC) is fully complementary to the ADB region (Figure 2B), therefore it was hoped that it would function as a DB sequence. Utility of *Nhe*I site as an alternative DB could be best judged by NPTII accumulation from the *rbcL* leader, which is highly dependent on DB. High levels of NPTII from the *Nhe*I construct (4.7%) relative to the mutant DB (0.3%) indicate, that linking the coding region via an *Nhe*I site provides a suitable DB for expressing foreign polypeptides (Figure 10, Table 3).

**TABLE 3**

| **Levels of NPTII and *neo* mRNA in tobacco leaves** | | | | | |
|---|---|---|---|---|---|
| | SD | DB | NPTII (%) | neo mRNA | NPTII/neo mRNA |
| Nt-pTNH32-70 | + | - | 2.10±0.33 | 41.5 | 5.06 |
| | | | | | |
| Nt-pHK30-1D | (+) | wt | 7.02±0.82 | 70.05±12.33 | 8.85 |
| Nt-pHK31-1C | (+) | s | 2.52±0.79 | 100 | 2.52 |
| Nt-pHK60-5A | (+) | m | 4.03±1.45 | 91.57±12.76 | 4.40 |
| | | | | | |
| Nt-pHK32-2F | - | wt | 1.17±0.05 | 49.33±7.76 | 2.37 |
| Nt-pHK33-2A | - | s | 0.21±0.05 | 49.55±6.67 | 0.42 |
| | | | | | |
| Nt-pHK34-9C | + | wt | 10.83±3.84 | 48.91±22.65 | 22.14 |
| Nt-pHK35-4A | + | s | 4.68±1.84 | 21.41±7.88 | 21.86 |
| Nt-pHK64-3A | + | m | 0.31±0.15 | 52.47±4.29 | 0.59 |
| | | | | | |
| Nt-pHK36-1C | + | wt | 2.17±70.97 | 68.8 | 3.15 |
| Nt-pHK37-2D | + | s | 2.35±0.05 | 42.3 | 5.56 |
| Nt-pHK38-2E | + | Ec | 16.39±3.42 | 47.59±19.06 | 34.44 |
| Nt-pHK39-3B | + | pt | 0.16±0.13 | 13.12±1.27 | 1.22 |
| Nt-pHK40-12B | + | s | 23.00±5.40 | 90.27±31.83 | 25.48 |
| | | | | | |
| Nt-pHK43-1C | (+) | s | 0.65±0.28 | 13.2 | 4.92 |

### DISCUSSION

In bacteria, mutagenesis or deletion of the DB reduces translation 2- to 34-fold, depending on the individual mRNA (Etchegaray and Inouye, 1999; Faxen *et al.,* 1991; Ito *et al.*, 1993; Mitta *et al.,* 1997; Sprengart *et al.*, 1996). Furthermore, reliance on the DB increases when the SD sequence is removed (Sprengart *et al.*, 1996; Wu and Janssen, 1996). In our experiments, no variation was made in the *atpB* or *rbcL* 5'UTR, only sequences downstream of the AUG were altered. Mutagenesis of the *atpB* DB region reduced protein levels ∼2-fold. Although the *atpB* mRNA does not have a SD directly upstream of AUG, we speculate that it probably has an alternate mechanism for translation initiation that reduces its dependence on the DB. Alternatively translation initiation may be facilitated by activator proteins as described for *Chlamydomonas* chloroplasts (Rochaix, 1996; Stern *et al.*, 1997). The consequence of DB mutagenesis on *rbcL* translation was a dramatic 35-fold drop in NPTII levels. Accordingly, efficient *rbcL* translation is highly dependent on DB-ADB interactions. Genes in both prokaryotes and eukaryotes show biases in the usage of the 61 amino acid codons and have a tRNA population closely matched to the overall codon bias of the resident mRNA population. Incorporation of synonymous minor codons in the coding region may dramatically reduce translation (Makrides, 1996) and destabilize the mRNA (Deana et al., 1998). A well-characterized example for minor codons causing reduced expression in *E. coli* are the AGA/AGG arginine codons recognized by the same tRNA which are present at the frequency of 2.6 and 1.6 per thousand codons. Therefore, we have compared codon usage bias and frequency of triplets per 1000 nucleotides in the wild-type and mutagenized atpB and rbcL DB regions. Since we studied NPTII accumulation in leaves, the values shown in Figure 12 were calculated for the highly expressed rbcL, psaA, psaB, psaC, psbA, psbB, psbC, psbD, psbE and psbF photosynthetic genes using the Genetics Computer Group (GCG; Madison Wisconsin) codon frequency program. Codon usage bias and triplet frequency is comparable in the wild-type and mutant DB regions of both atpB and rbcL. In addition, the mRNAs for the wild-type and mutant DB constructs accumulate at similar levels. Therefore, the dramatic change in NPTII acccumulation from the PrrnLrbcL+DBm promoter in the Nt-pHK64 line can not be attributed to incorporation of a rare codon in the mutant DB region.

We have shown here that sequences downstream of the translation initiation codon may dramatically affect mRNA translation. Therefore, silent mutations in the DB region of heterologous proteins may significantly improve expression in chloroplasts by increasing complementarity of the mRNA with the plastid rRNA penultimate stem structure.

There are significant differences in NPTII accumulation from *neo* transgenes with different leaders and the same synthetic DB (Table 3). This indicates that the 5'UTR is an important determinant of translation efficiency. Many data are available supporting the importance of 5'UTR as a target for translational control in higher plants (Hirose and Sugiura, 1996; Staub and Maliga, 1993; Staub and Maliga, 1994b) and the unicellular alga *Chlamydomonas* (Mayfield *et al*., 1994; Nickelsen *et al.,* 1999; Sakamoto *et al.,* 1993; Zerges *et al.*, 1997). The data presented herein demonstrate that translation efficiency in plastids is determined by sequences both upstream and downstream of the AUG.

### EXAMPLE 2

### Study of phage T7g10 translation control sequences indicates that the efficient DB in plastids has loose complementarity to ADB

Since the actual ADB sequence is different in plastids and *E. coli,* we anticipated (Sprengart *et al.*, 1996; Etchegaray & Inoyue, 1999) that replacement of the *E. coli* DB with a perfect plastid DB (100% DB-ADB complementarity) would enhance translation in plastids. We choose the phage T7g10 translational control region for the study since it has a well-characterized *E. coli* DB. Three Prrn promoter derivatives were constructed. Cassette PrrnLT7g10+DB/Ec consists of Prrn fused with the native T7g10 TCR containing the *E. coli* DB (plasmid pHK38; Figure 2B, Figure 4A). Cassette PrrnLT7g10+DB/pt consists of the Prrn promoter, T7g10 leader and the perfect tobacco DB (pHK39; Figure 2B, Figure 4A). Cassette PrrnLT7g10-DB has the Prrn promoter and T7g10 leader, but lacks the T7g10 DB sequence (pHK40; Figure 2B, Figure 4A). The *neo* coding region in these constructs is directly linked to the Prrn promoter via a synthetic *Nhe*I restriction site. The *neo* genes in the three expression cassettes were introduced into tobacco plastids by transformation (Svab and Maliga, 1993; Zoubenko *et al.*, 1994) and the leaves of transplastomic tobacco were tested for NPTII accumulation and mRNA levels (Figures 10, 11; Table 3).

Surprisingly, NPTII levels from the heterologous T7g10 TCR were higher (Nt-pHK38; ~16%) than the levels obtained from the *rbcL* TCR (Nt-pHK34; ~11%). We expected that incorporation of the plastid DB with 100% complementarity would further enhance NPTII levels. Instead, we found that plants transformed with the construct having the perfect plastid DB (Nt-pHK39) contained NPTII levels 100-fold lower than the plants expressing NPTII from the *E. coli* TCR (Nt-pHK38; Figures 10; Table 3). This result suggests that, unlike in *E. coli*, 100% complementarity reduces, rather than enhances translation efficiency. Indeed, none of the highly expressed plastid genes have a perfect DB sequence (Figure 2A). RNA gel blots shown in Figure 11 indicate that Nt-pHK39 plants with the perfect DB contain -3-fold less *neo* mRNA. Therefore, a contributing factor to lower NPTII levels in these plants appears to be a faster mRNA turnover rate. Furthermore, NPTII expressed from the PrrnLT7g10 derivatives differ by the DB-encoded amino acids at the N-terminus. Therefore, differential protein turnover rates may be part of the reason for differences in NPTII accumulation. The highest yield of NPTII (23%) was obtained with the synthetic, NheI-containing DB cassette.

### DISCUSSION

This example utilizing the *rbcL* translation control regions reveals that sequences downstream of the translation initiation codon may dramatically affect mRNA translation. Therefore, silent mutations in the DB region of heterologous proteins may significantly improve expression in chloroplasts by increasing complementarity of the mRNA with the plastid rRNA penultimate stem structure. However, it appears that perfect complementarity is undesirable, as it may accelerate mRNA turnover and reduce the rate of translation. This finding highlights differences in the translation machinery of plastids and *E. coli*, in which perfect complementarity enhances translation (Etchegaray and Inouye, 1999; Sprengart *et al.*, 1996). It is possible, however, that shifting the region of complementarity relative to AUG or targeting a slightly different region of the penultimate stem may facilitate highly efficient translation of mRNAs with a perfectly matched DB.

The T7g10 constructs have one or two relatively rare AGC serine codons (4.7 per 1000, Figure 12), one of which is encoded in the NheI site. This codon is present in the Nt-pHK38 and Nt-pHK40 plants, which contain the highest levels of NPTII. Further improvement may be expected by replacing the AGC with an AGT serine codon.

### EXAMPLE 3

### The clpP, psbB and psbA TCRs have distinct expression characteristics

NPTII accumulation was studied in transplastomic tobacco carrying the PrrnLclpP promoter derivatives. The PrrnLclpP+DBwt (Nt-pHK32-2F) and PrrnLclpP-DB (Nt-pHK33-2A) plants accumulate 1.2% and 0.2% NPTII in their leaves (Figure 10; Table 3). We have found that over-expression of *clpP* 5'-UTR causes a mutant phenotype manifested as pale green leaf color and slower growth. This phenotype is normalized in older plants. We assume that the primary cause of this mutant phenotype is the lack of ClpP protein, the *clpP* gene product. This mutant phenotype is absent in plants transformed with other 5'UTRs. Therefore we believe, that the mutant phenotype is attributable to competition for a *clpP*-specific nuclear factor. The *clpP* gene has two introns. Preliminary RNA gel blot analysis reveals reduced levels of mature, monocistronic *clpP* mRNA (~30% of wild-type) and accumulation of intron I-containing *clpP* pre-mRNA in the pale-green leaves. Normalization of phenotype coincides with increase of translatable monocistronic *clpP* mRNA to wild type levels. Over-expression of clpP 5'UTR therefore may interfere with splicing of *clpP* pre-mRNA.

NPTII accumulation was also studied in transplastomic tobacco carrying the PrrnLpsbB promoter derivatives. The PrrnL psbB+DBwt (Nt-pHK36-1C) and PrrnL psbB -DB (Nt-pHK37-2D) plants accumulate 2.2% and 2.4% NPTII in their leaves (Figure 10; Table 3). Thus, the synthetic DB sequence in case of the *psbB* TCR efficiently replaces the native DB sequence.
Conversely, it may rely on an alternative mechanism for translation initiation.

The Prrn promoter constructs with the *psbA* leader were obtained as described. However, we have been able to introduce only one of them, PrrnLpsbA-DB(+GC) into tobacco plastids in line Nt-pHK43-1C. The Nt-pHK43-1C plants accumulate NPTII at a relatively low level (0.6%; Figure 10, Table 3). It is conceivable that the lack of success in introducing the +DB construct is due to the dramatically elevated expression level of NPTII which is toxic to the plants.

### DISCUSSION

NPTII levels obtained from PrrnLclpP+DBwt (Nt-pHK32-2F) promoter are relatively low, only 1.2% of the total soluble protein. However, this promoter is desirable for driving expression of selectable marker genes, as the recovery of transplastomic clones is relatively efficient when the *neo* gene is expressed from this promoter, as shown in Example 4. Expression of *neo* from the PrrnLclpP+DBwt promoter does not cause a mutant phenotype in tissue culture. Thus, it is suitable to drive the expression of marker genes, so long as the marker gene is subsequently removed. It appears that competition for a nuclear-encoded factor required for processing the *clpP* introns gives rise to the reduced expression observed. This intron is absent in the *clpP* genes in the monocots rice (Hiratsuka et al., 1989) and maize (Maier et al., 1995). The PrrnLclpP+DBwt promoter therefore may be used to advantage in the transformation of monocots. Furthermore, the level of the trans-factor required for clpP intron processing is likely to be expressed at different levels in dicots. We anticipate therefore, that expression of the clpP TCR will have no undesirable consequences in other dicot species. It is also possible that the phenotypic consequences of expressing the clpP TCR in plastids is a property of the tobacco line, *N. tabacum* cv. Petit Havana utilized herein and is absent in other tobacco lines. This would make the clpP gene TCR a desirable expression tool in both monocots and dicots.

Both *psbB* leader derivatives accumulate NPTII at comparable levels (2.2% and 2.4%, respectively; Table 3). This 5' regulatory region is a good alternative to the most commonly used rbcL leader when protein accumulation is required in the -2% range.

In the past, the *psbA* promoter and leader construct yielded relatively high levels of expression in leaves (2.5% GUS; Staub and Maliga, 1993). Yet these constructs did not contain psbA DB elements. The present invention describes the generation of chimeric promoters that are suitable to obtain high-level protein expression while elucidating the regulatory role played by DB sequences. Prrn is the strongest known promoter in plastids and consequently provides for high levels of NPTII translation. These elevated levels of NPTII can be toxic to the plant and therefore it is difficult to obtain transplastomic lines with the highest prospective levels of NPTII. An alternative approach involves operably linking the *psbA* leader to a relatively weak promoter. This approach may generate cassettes which are suitable for obtaining relatively high levels of protein accumulation from relatively low levels of mRNA.

### EXAMPLE 4

### NPTII accumulation in roots and seeds

Posttranscriptional regulation is an important mechanism of plastid gene expression (Rochaix, 1996; Stem et al., 1997). Therefore, we expected that NPTII accumulation may be tissue-specific due to regulation of gene expression at the level of mRNA translation. Thus, NPTII accumulation was tested in roots and seeds.

Testing of NPTII accumulation in roots was carried out with a subset of transplastomic lines (Table 4). Roots for protein extraction were collected from plants grown in liquid MS salt medium (3% sucrose) in sterile cultures incubated on a shaker to facilitate aeration. Protein was extracted from the roots with the leaf protocol and tested for NPTII accumulation (Figure 13A). The highest level of NPTII, 0.75%, is found in the roots of plants expressing NPTII from the *clpP* TCR (PrrnLclpP+DBwt construct; pHK32). The second highest value, 0.3%, was found in the roots of plants transformed with plasmid pHK38 expressing NPTII from the T7g10 TCR (PrnnLT7g10+DB/Ec promoter). The level of NPTII was about the same, approximately 0.1 %, in roots expressing the recombinant protein from the *atpB* and *rbcL* TCR in pHK30- and pHK34- transformed plants.

Since plastids in the roots are smaller than in leaves, we expected lower levels of NPTII accumulation in the roots than in the leaves. This was true for all the tested roots, except those of the Nt-pHK32 plants. Interestingly, NPTII from the *clpP* TCR accumulated at almost the same level in the roots (0.75%,, Table 4) as in the leaves (approximately 1%, Table 3). This is likely attributable to high levels of the *neo* mRNA in the roots (Figure 13B). Since the *clpP* leader includes the minimal PclpP-53 promoter (Sriraman et al., 1998a; NAR 26: 4874) we speculate, that the relatively high mRNA levels are due to activation of PclpP-53 in roots. High levels of expression make the clpP leader a desirable TCR for protein expression in roots.

The T7g10 leader (pHK38) was the most efficient in roots from which the most NPTII accumulated relative to the mRNA (Table 4). Although in the Nt-pHK38 plants, the neo mRNA was 7-times less than in the Nt-pHK32 plants, NPTII levels were almost as high (approximately 0.30% compared to 0.75%) as in the plastids with the clpP TCR (pHK32). High level NPTII accumulation from the T7g10 TCR in leaves (pHK38, pHK40; Table 3) and in roots (pHK38; Table 4) indicates the general utility of the phage T7g10 translation control region for protein expression in plastids.

Protein accumulation was also studied in seeds harvested from the transgenic plants (Figure 14). Protein levels were 0.05% in plants transformed with pHK32 (clpP TCR), and approximately 0.01% in plants transformed with plasmid pHK30 (atpB TCR). No NPTII was detectable in plants in which *neo* was introduced in the rbcL TCR-construct (plasmid pHK34), indicating differential protein accumulation which is dependent on the choice of the TCR.

**Table 4.**

| **Levels of NPTII and *neo* mRNA in tobacco roots** | | | |
|---|---|---|---|
| Strain | NPTII (%) | *neo* mRNA (%) | NPTII/*neo* mRNAx10³ |
| Nt-pHK30-1D | 0.14±0.05 | 33.7 | 4.2 |
| Nt-pHK32-2F | 0.75±0.35 | 100 | 7.5 |
| Nt-pHK34-9C | 0.12±0.03 | 23.5 | 5.1 |
| Nt-pHK38-2E | 0.31±0.04 | 13.4 | 23.1 |

### EXAMPLE 5

### High-level NPTII expression facilitates efficient recovery of transplastomic lines by selection for kanamycin resistance

The plastid genome of higher plants is a 120-kb to 160-kb double-stranded DNA which is present in 1,900 to 50,000 copies per leaf cell (Bendich, 1987). To obtain genetically stable transplastomic lines every one of the plastid genome copies (ptDNA) should be uniformly altered in a plant. Since integration of foreign DNA always occurs by homologous recombination, plastid transformation vectors contain segments of the plastid genome to target insertions at specific locations. Useful, non-selectable genes are cloned next to the selectable marker genes, which are then introduced into the plastid genome by linkage to the selectable marker gene (Maliga, 1993). Transforming DNA is introduced into plastids by the biolistic process (Svab et al., 1990; Svab and Maliga, 1993) or PEG treatment (Golds et al., 1993; O'Neil et al., 1993). Elimination of wild-type genome copies occurs during repeated cell divisions on a selective medium. The success of transformation depends on the success of selective amplification of the few initially transformed genome copies. Therefore the choice of the antibiotic used for the selective amplification of transformed genome copies and the mechanism by which the plant cells are protected from antibiotic action is a critical parameter to be considered for successful generation of homoplasmic plants.

The most commonly used antibiotic for the selection of transplastomic lines is spectinomycin, an inhibitor of protein synthesis on plastid ribosomes. Initially, plastid transformation in tobacco was carried out by selection for resistance based on mutations in the plastid 16S rRNA (Svab et al., 1990). Selection was inefficient, yielding about one transplastomic clone per 50 bombarded samples, probably because the 16S rRNA based mutation in recessive. Recovery of transplastomic lines was enhanced -100-fold by selection for a dominant marker, spectinomycin resistance based on inactivation by aminoglycoside 3" adenyltransferase encoded in a chimeric *aadA* gene (Svab and Maliga, 1993). In addition to tobacco, selection for spectinomycin resistance (*aadA*) could be applied to recover transplastomic lines in *Arabidopsis* and potato. The *aadA* gene in plants confers resistance to both spectinomycin and streptomycin. Selection for streptomycin resistance was used for plastid transformation in rice, a species resistant to spectinomycin, after bombardment with a chimeric *aadA* gene. See Example 8.

The need for an alternative marker gene for plastid manipulation has led to testing kanamycin resistance as a selective marker. A chimeric *neo* (*kan*) gene, encoding neomycin phosphotransferase, was suitable to recover transplastomic tobacco lines. However, recovery of transplastomic lines was relatively inefficient, yielding only one transplastomic line in -25 bombarded leaf samples. Furthermore, for every plastid transformation event -25 to 50 kanamycin resistant lines were obtained in which integration of the plastid *neo* construct into the nuclear genome resulted in kanamycin resistance (Carrer *et al.*, 1993). We report here that the efficiency of recovering transplastomic clones is significantly improved when transforming tobacco chloroplasts with a new *neo* gene expressed from a promoter with the *atpB* and clpP translation control region. The number of nuclear transformation events is reduced using the cassettes of the present invention. These improvements make the new *neo* gene a practical tool for plastid genome manipulations.

### DISCUSSION

The chimeric *neo* genes described in Examples 1-4 were introduced into plastids by selection for the linked spectinomycin resistance (*aadA*) gene as their suitability for directly selecting transplastomic lines was unknown. The transplastomic lines listed in Table 3 were then tested for resistance to kanamycin by their ability to proliferate on a medium containing 50 mg/L kanamycin. The RMOP meduim used for testing induces formation of green callus and shoot regeneration in the absence of kanamycin. The tissue culture procedures utilized for this example are described in references Carrer et al., 1993 and Carrer and Maliga, 1995.

On the selctive kanamycin medium only scanty, white callus forms from wild-type leaf section. Formation of green callus and shoots from leaf section of plants transformed with pHK plasmids in Table 3 indicates that accumulation of NPTII confers kanamycin resistance. We set out to test if transplastomic clones can be directly selected by kanamycin resistance after bombardment with plasmids pHK30 and pHK32. The results are summarized in Table 5.

Bombardment of 25 tobacco leaves with plasmid pHK30 yielded 45 kanamycin resistant lines on a medium containing 50 mg/L kanamycin. Transplastomic *neo* lines are expected to be resistant to much higher levels, 500 mg/L of kanamycin (Carrer et al., 1993). In addition, in plasmid pHK30 the *neo* gene is physically linked to a spectinomycin resistance (*aadA*) gene. Spectinomycin resistance is manifested as kanamycin resistance: sensitive leaf sections form white callus and no shoots whereas resistant leaf sections form green callus and shoots on a selective medium (500 mg/L) RMOP medium. We assumed therefore, that all transplastomic lines should be resistant to both 500 mg/L of kanamycin and 500 mg/L spectinomycin (Carrer and Maliga, 1995). When applying this test we found that 22 of the 45 lines meet these criteria. Digestion of the plastid DNA with the EcoRI restriction enzyme and probing with the plastid targeting region should detect 3.1-kb fragment in the wild-type and a 4.2-kb and 1.2-kb fragment in transplastomic lines (Figure 15A). DNA gel blot analysis of seven of the kanamycin-spectinomycin resistant lines confirmed integration of both transgenes into the plastid genome (Figure 15B). Therefore, we assume that all 22 kanamycin-spectinomycin lines are transplastomic (Table 5).

Bombardment of 30 tobacco leaves with plasmid pHK32 yielded 28 kanamycin resistant lines on a medium containing 50 mg/L kanamycin. We have identified 11 double-resistant lines by testing these on a medium containing 500 mg/L of kanamycin and 500 mg/L spectinomycin. All six tested were transplastomic by DNA gel bloc analysis (Figure 15B), therefore we believe that all eleven are transplastomic (Table 5).

**TABLE 5**

| **SELECTION OF TRANSPLASTOMIC TOBACCO CLONES BY KANAMYCIN RESISTANCE** | | | | | |
|---|---|---|---|---|---|
| Vector | No. leaves | Kan. Res. 50 mg/L | Kan. Res. 500 mg/L | Kan. Res. 500 mg/L Spec. Res. 500 mg/L | Transplastomic |
| pTNH32 | 29 | 59 | 7 | | 0 |
| | 50^{a} | 52 | | | 2 |
| | 25^{a} | 47 | 4 | | 1 |
| pHK30 | 25 | 45 | | 22 | 22 |
| pHK32 | 30 | 28 | | 11 | 11 |

| | | | | | |
|---|---|---|---|---|---|
| (^{a}Carrer et al., 1993) | | | | | |

### DISCUSSION

Plastid transformation efficiency should be comparable, if we target the same region of the plastid genome for insertion, use similar size targeting sequences and the same method of DNA delivery.
Therefore, lower transformation efficiencies obtained by selection for kanamycin resistance with the old chimeric *neo* genes was likely due to the lack of recovery of tranplastomic clones by selection. We have found that transformation with neo genes expressed from the PrrnLatpB+DBwt and PrrnLclpP+DBwt promoters is as efficient as with the aadA gene. This is a significant technical advance, and will facilitate plastid transformation in crops, in which the regenerable tissues contain non-green plastids. Most important targets are the non-green plastids of cereal crops. Kanamycin selection is widely used to obtain transgenic lines after transformation with chimeric neo genes in dicots. However, kanamycin is an undesirable selective agent in monocots such as cereal tissue cultures. However, NPTII also inactivates paromomycin, which may be used to recover nuclear gene transformants at an extremely high efficiency in cereals. See for example, PCT application WO99/05296.

### EXAMPLE 6

### Bacterial bar gene expression in tobacco plastids confers resistance to the herbicide phosphinothricin

Bialaphos, a non-selective herbicide, is a tripeptide composed of two L-alanine residues and an analog of glutamic acid known as phosphinothricin (PPT). While PPT is an inhibitor of glutamine synthetase in both plants and bacteria, the intact tripeptide has little or no inhibitory effect in vitro. Bialaphos is toxic for bacteria and plants, as intracellular peptidases remove the alanine residues and release active PPT. Bialaphos is produced by *Streptomyces hygroscopicus.* The bacterium is protected from phosphinothricin toxicity by phosphinothricin acetyltransferase (PAT), the bar gene product. This enzyme acetylates phosphinothricin or demethylphosphinothricin (Thompson et al., 1987). PPT resistant crops have been obtained by expressing the *S*. *hygroscopicus* bar gene in the plant nucleus. Herbicide resistant lines were obtained by direct selection for PPT resistance in culture after *Agrobacterium tumefaciens*-mediated DNA delivery in tobacco, potato, Brassica napus and *Brassica oleracea* (De Block et al., 1987, 1989). Biolistic DNA delivery of chimeric bar genes has been employed to obtain PPT resistant maize (Spencer et al., 1990), rice (Cao, et al, 1992) and Arabidopsis thaliana (Sawaskaki et al., 1994). Construction of transplastomic tobacco plants, in which PPT resistance is based on the expression of bar from S. hygroscopicus in plastids is described in the present example. The vectors utilized to express the bar gene contain an exemplary chimeric 5' regulatory region as set forth in the previous examples. The following material and methods facilitate the practice of this aspect of the present invention.

### Construction of plastid bar gene

A NcoI/XbaI bar gene fragment was generated by PCR amplification using plasmid of pDM302 (Cao et al., 1992) with the following primers:
P1, 5'-AAACCATGGCACCACAAACAGAGAGCCCAGAACGACGCCC-3';
P2, 5'-AAAATCTAGATCATCAGATCTCGGTGACG-3'.

The ends of the PCR fragment were blunt ended by treatment with the Klenow Fragment of DNA polymerase I. The fragment was then ligated into the EcoRV site of pBluescript II KS+ (Stratagene, La Jolla, CA) to create plasmid pJEK3. Sequence analysis of pJEK3 plasmid DNA revealed that the XbaI site we intended to create through PCR amplification of pDM302 is absent. See Figure 19. The bar gene has the two translation termination codons followed by vector sequences. The last 20 bp of pJEK3 are:
CCCGTCACCGAGATCTGATGAtcgaattcctgcagcccgggggatccactagttct aga. The bar sequences are in capital (stop codons underlined), the vector sequences are in lower case (XbaI site underlined). Since there is an XbaI site present in the vector 40 bp from the intended XbaI site, it was not necessary to repair this error. The NcoI-XbaI fragment from plasmid pJEK3 was ligated into NcoI-XbaI digested pGS104 plasmid (Serino and Maliga, 1997) to generate plasmid pJEK6. Plasmid pGS104 carries a PrrnTrbcL expression cassette in a pPRV111B plastid transformation vector. A map of the plastid targeting region of plasmid pJEK6 is shown in Figure 16A.

### Plastid transformation and plant regeneration

Tobacco (*Nicotiana tabacum cv.* Petit Havana) plants were grown aseptically on agar-solidified medium containing MS salts (Murashige and Skoog, 1962) and sucrose (30g/1). Leaves were placed abaxial side up on RMOP media for bombardment. The RMOP medium consists of MS salts, N6-benzyladenine (1mg/l), 1-naphthaleneacetic acid (0.1 mg/l), thymine (1mg/l), inositol (100 mg/l), agar (6g/l), pH 5.8, and sucrose (30g/l). The DNA was introduced into chloroplasts on the surface of 1µm tungsten particles using the DuPont PDS1000He Biolistic gun (Maliga 1995). Spectinomycin resistant clones were selected on RMOP medium containing 500 µg/ml spectinomycin dihydrochloride. Resistant shoots were regenerated on the same selective medium and rooted on MS agar medium (Svab and Maliga, 1993). The independently transformed lines are designated by the transforming plasmid (pJEK6) and a serial number, for example pJEK6-2, pJEK6-5. Plants regenerated from the same transformed line are distinguished by letters, for example pJEK6-2A, pJEK6-2B.

### Southern Blot Analysis

Total cellular DNA was isolated from wild-type and transgenic spectinomycin resistant plants with CTAB (Saghai-Maroof et al., 1984). The DNA was digested with the Sma I and BglII restriction endonucleases, separated on a 0.7% agarose gel and blotted onto a Hybond-N nylon membrane (Amersham, Arlington Heights, IL) by a pressure blotter: The membrane was hybridized overnight with an ApaI/ BamHI fragment labeled with (α- ³²P )dCTP using a dCTP DNA Labeling Beads Kit (Pharmacia Inc, Piscataway, NJ). The membrane was washed 2 times with 0.1X SSPE, 0.2X SDS at 55°C for 30 minutes. Film was exposed to the membrane for 30 minutes at room temperature.

### PAT Assay

The PAT assay was performed as described by Spencer et. al. (1990). Leaf tissue (100 mg) from wild type tobacco (wt), transgenic Nt-pDM307-10 tobacco (a line transformed with the nuclear bar gene in plasmid pDM307; Cao et al., 1992), and plastid bar gene transformants was homogenized in 1 volume of extraction buffer (10 mM Na₂HPO₄, 10 mM NaCl). The supernatant was collected after spinning in a microfuge for 10 minutes. Protein (25 mg) was added to 1 mg/ml PPT and ¹⁴C-labeled Acetyl CoA. The reaction was incubated at 37°C for 30 minutes and the entire reaction was spotted onto a TLC plate. Ascending chromatography was performed in a 3:2 mixture of 1-propanol and NH₄OH. Film was exposed to the TLC plate overnight at room temperature.

### Herbicide Application

Wild type and transgenic plants were sprayed with 5 ml of a 2% solution of Liberty (AgrEvo, Wilmington, DE) with an aerosol sprayer.

### RESULTS AND DISCUSSION

First the bacterial bar gene was converted into a plastid gene by cloning the bar coding region into a plastid expression cassette. This cassette consists of an engineered plastid rRNA operon promoter (Prrn) and TrbcL and the 3' UTR of the plastid rbcL gene for stabilization of the mRNA. The plastid bar gene was then cloned into the plastid transformation vector to yield plasmid pJEK6, and introduced into plastids on the surface of microscopic tungsten particles. The bar gene integrated into the plastid genome by two homologous recombination events via the plastid targeting sequences, as shown in Figure 16A. Selection for the linked aadA (spectinomycin resistance) gene on spectinomycin-containing medium eventually yielded cells which carried a uniformly transformed plastid genome population, which were then regenerated into plants.

Integration of bar and aadA was verified by DNA gel blot analysis. Total cellular DNA of wild-type and transplastomic plants was digested with the SmaI and BglII restriction enzymes and probed with the 2.9-kb ApaI-BamHI plastid targeting fragment of *N. tabacum* (Figure 16B). The two fragments that were expected for the transgenic plants, 3.3 kb and 1.9 kb, were present in each of the transplastomic samples shown in Figure 16B. Absence of the 2.9 kb wild type fragment indicated, that by the time these plants have been regenerated, the wild-type plastid genome copies have been diluted out on the selective medium.

To determine if the plastid bar gene has been expressed, leaf extracts were assayed for phosphinothricin acetyltransferase (PAT) activity. Conversion of PPT into acetyl-PPT indicated PAT activity in each of the tested transplastomic lines. Data in Figure 17 are shown for the transplastomic lines Nt-pJEK6-2D, Nt-pJEK6-5A and Nt-pJEK6-13B. Interestingly, PAT activity was significantly (>>10-fold) higher when bar was expressed in the plastids, as compared to the bar gene expressed from the cauliflower mosaic virus 35S promoter in the nucleus of the Nt-pDM307-10 plant.

PAT expression confers resistance to PPT in tissue culture and in the greenhouse. When wild type leaf sections are grown in tissue culture, 10 mg/L PPT completely blocks callus proliferation. This same PPT concentration is suitable for the selection of nuclear transformants after bombardment with the nuclear bar construct in plasmid pDM307. Leaf sections of plants expressing bar in plastids show resistance in the presence of up to 100 mg/L PPT in the culture medium. We have tested PPT resistance in the greenhouse, spraying wild-type and transplastomic plants with Liberty, a commercial formulation of PPT, at the recommended field dose of 2%. As shown in Figure 18A, 13 days after the treatment, the wild type plants were dead while the transgenic plants thrived. Since then the sprayed plants have flowered and set seed. Figure 18B shows maternal inheritance of PPT resistance. Lack of plastid pollen transmission results in a lack of herbicide resistance in progeny pollinated with transgenic pollen. The bacterial bar gene has a high G + C content (68.3%; Genbank Accession No. X17220), while plastid genes have a relatively high A + T content; for example the G + C content of the highly expressed psbA and rbcL genes is 42.7% and 43.7%, respectively (Genbank Accession No. Z00044). Differences in the G + C content are also reflected in the codon usage biases. Interestingly, data presented here indicate that expression of bar from *S. hygroscopicus* is sufficiently high to confer resistance to field levels of the non-selective herbicide PPT. Furthermore, the PAT enzyme levels obtained in the transplastomic lines are significantly higher than those observed in the nuclear transformant. Therefore, further improvement of the expression levels may be obtained by optimizing the codon usage for plastids as set forth in Example 7.

Advantages of incorporating bar in the plastid genome are containment of herbicide resistance due to the lack of pollen transmission in most crops. Furthermore, the lack of genetic segregation would simplify back-crossing for the introduction of herbicide resistance into additional breeding lines.

### EXAMPLE 7

### A Synthetic bar gene Improves Containment and Enhances Expression in Plastids

The bacterial *bar* gene was introduced into the tobacco plastid genome by transformation with plasmid pJEK6, as described above in Example 6. In plasmid pJEK6 *bar* is expressed in a cassette consisting of the Prrn(L)rbcL(S) promoter and TrbcL transcription terminator. This plasmid conferred PPT resistance to plants grown in the presence of PPT in the tissue culture medium, but direct selection for transformed lines was not possible. Although the PAT levels in homoplastomic leaves was high, the amount of PAT produced by the few pJEK6 bar copies during the early stage of plastid transformation was probably insufficient to protect the entire cell.

To improve bar expression in plastids a synthetic gene was created. The codon usage was modified to mimic that of the average tobacco photosynthetic plastid gene. Changing the codon usage lead to a lowered GC content characteristic of higher plant plastid genes. To assist with cloning, restriction enzyme recognition sequences were removed and added as necessary. Codon usage frequency in bacteria reflects relative tRNA abundance: frequent use of codons for rare tRNAs may significantly reduce translation efficiency. We hoped that differential codon usage in plastids and bacteria would reduce or prevent expression of the synthetic gene in bacteria, thereby reducing the danger of horizontal gene transfer to microorganisms. We also hoped that improved bar expression in our novel promoter cassettes will allow direct selection of plastid transformants on PPT-containing medium.

### Materials and Methods for Example 7

Codon comparisons of photosynthetic (*rbcL, psaA, psaB, psaC, psbA, psbB, psbC, psbD, psbE, psbF*) plastid genes were compiled using GCG (Genetics Computer Group, Madison, WI). DNA mutations were then introduced into the bacterial bar gene making its codon usage more similar to plastid genes, while removing several restriction enzyme sites that could interfere with cloning. See Figure 28. The synthetic bar gene (*s-bar*) was obtained by single-step assembly of the entire *s-bar* gene from 28 oligonucleotides (one 44 nt primer, one 30 nt primer and twenty-six 40 nt primers) using PCR (Stemmer et al., 1995). The top and bottom strands of the primers overlap with each other by 20 nucleotides. *Nco*I and *Nhe*I sites were added at the 5' end and a *Xba*I site was added at the 3' end through PCR amplification. To obtain the complete *s-bar* gene, a small aliquot of the assembly PCR product was amplified using primers 1A and 14B. Unchanged nucleotides are in upper case, altered nucleotides are in lower case in the primers listed below.

| | |
|---|---|
| Primer 1A | ccATGgctAGCCCAGAAaGAaGaCCGGCCGAtATtaGaCG |
| Primer 1B | GCATaTCaGCtTCtGTaGCACGtCtaATaTCGGCCGGtCt |
| Primer 2A | TGCtACaGAaGCtGAtATGCCaGCaGTtTGtACaATCGTt |
| Primer 2B | CTTGTtTCtATaTAaTGGTTaACGATtGTaCAaACtGCtG |
| Primer 3A | AACCAtTAtATaGAaACAAGtACaGTaAACTTtaGaACtG |
| Primer 3B | tTCtTGaGGTTCtTGaGGtTCaGTtCtaAAGTTtACtGTa |
| Primer 4A | AaCCtCAaGAACCtCAaGAaTGGACtGAtGAtCTaGTCCG |
| Primer 4B | AaGGATAGCGCTCtCGtAGACGGACtAGaTCaTCaGTCCA |
| Primer 5A | TCTaCGaGAGCGCTATCCtTGGCTtGTaGCaGAaGTtGAC |
| Primer 5B | GCGATaCCaGCtACtTCaCCGTCaACtTCtGCtACaAGCC |
| Primer 6A | GGtGAaGTaGCtGGtATCGCaTAtGCGGGCCCtTGGAAGG |
| Primer 6B | CCAaTCaTAtGCaTTtCtTGCCTTCCAaGGGCCCGCaTAt |
| Primer 7A | CAaGaAAtGCaTAtGAtTGGACaGCtGAaTCaACtGTtTA |
| Primer 7B | GtTGaTGaCGtGGtGAaACGTAaACaGTtGAtTCaGCtGT |
| Primer 8A | CGTtTCaCCaCGtCAtCAaCGtACaGGACTtGGtTCtACt |
| Primer 8B | TTCAGtAGaTGtGTaTAtAGaGTaGAaCCaAGtCCtGTaC |
| Primer 9A | CTaTAtACaCAtCTaCTGAAaTCttTGGAGGCACAaGGtT |
| Primer 9B | aACAGCtACaACaCTCTTaAAaCCtTGTGCCTCCAaaGAt |
| Primer10A | TtAAGAGtGTtGTaGCTGTtATaGGatTGCCtAAtGAtCC |
| Primer10B | CtTCaTGCATGCGtACaCtTGGaTCaTTaGGCAatCCtAT |
| Primer11A | aAGtGTaCGCATGCAtGAaGCtCTaGGATATGCtCCaaGa |
| Primer11B | CCtGCaGCCCtCAaCATaCCtCttGGaGCATATCCtAGaG |
| Primer12A | GGtATGtTGaGGGCtGCaGGtTTCAAaCAtGGaAACTGGC |
| Primer12B | tTGCCAaAAACCtACaTCATGCCAGTTtCCaTGtTTGAAa |
| Primer13A | ATGAtGTaGGTTTtTGGCAaCTtGAtTTCAGtCTaCCaGT |
| Primer13B | GtAGaACtGGACGaGGaGGTACtGGtAGaCTGAAaTCaAG |
| Primer14A | ACCtCCtCGTCCaGTtCTaCCaGTtACtGAGATCTGATGA |
| Primer14B | tctagaTCATCAGATCTCaGTaACtG |

The amplified *s-bar* coding region was then cloned into a pBSIIKS+ plasmid (Stratagene, La Jolla, CA) and sequenced (Figure 20A). The *s-bar* gene was cloned into cassettes with the chimeric PrrnLatpB+DBwt, PrrnLrbcL+DBwt and PrrnLT7g10+DB/Ec promoters. Table 6 sets forth the plasmids used in the practice of this example.

**Table6. Plasmids with bar genes.**

| **Plasmid** | **Promoter** | **bar** | **3'UTR** | **Vector** |
|---|---|---|---|---|
| pKO5 | | synthetic (*s-bar*) | | pBSIIKS+ |
| pK03 | PrrnLatpB+DBwt | synthetic (*s-bar*) | TrbcL | pPRV111B |
| pKO8 | PrrnLrbcL+DBwt | synthetic (*s-bar*) | TrbcL | pPRV111A |
| pKO17 | PrrnLT7g10+DB/ Ec | synthetic (*s-bar*) | TrbcL | pPRV111B |
| pKO12 | PrrnLrbcL+DBwt | bacterial (*bar*) | TrbcL | pPRV111A |

To provide a suitable cloning site at 3'-end of the bacterial bar gene, the EagI/BglII fragment of *s-bar* was replaced with the cognate fragment of the bacterial *bar* coding region. Such a bacterial *bar* gene is incorporated in plasmid pKO12 (Figure 21). In plasmid pKO12 the first 22 nucleotides of the bacterial *bar* coding region are replaced with nucleotides from the *s-bar.*

### RESULTS

The engineered bacterial *bar* gene in pJEK6 is expressed both in *E. coli* and plants, as shown in the previous example. We were interested to test if modification of the codon affects expression of the *s-bar* gene in plastids and in *E. coli.* In *E. coli, s-bar* expression was determined by measuring PAT activity. Extracts were prepared from bacteria carrying plasmids pKO3 and pKO8 expressing *s-bar* from the PrrnLatpB+DBwt and PrrnLrbcL+DBwt promoters, respectively. The radioactive assay did not detect any activity, although extracts from bacteria transformed with plasmids pJEK6 and pKO12 carrying the bacterial *bar* genes gave strong signals (Figure 22A). In plasmid pKO12 the first 22 nucleotides of the bacterial *bar* coding region are replaced with nucleotides from the *s-bar*. Therefore, lack of expression from the *s-bar* in *E. coli* is not due to changes within the first 22 nucleotides.

The *s-bar* was also introduced into plastids by transformation with vector pKO3. Extracts were prepared from pKO3- and pJEK6-transformed tobacco plants, which carry the *s-bar* and bar genes, respectively. Extracts from both types of plants contained significant PAT activity (Figure 22B). Therefore, the synthetic *bar* is expressed in plastids but not in *E. coli.*

Changing the *bar* gene codon usage abrogated expression of the gene in *E. coli.* This is likely due to the introduction of the rare AGA and AGG arginine codons in the *s-bar* coding region. The triplet frequency per thousand nucleotides for AGA and AGG is the lowest in *E. coli*, reflecting low abundance of the tRNA required for translation of these codons. The minor arginine tRNA^{Arg(AGG/AGA)} has been shown to be a limiting factor in the bacterial expression of several mammalian genes. The coexpression of *ArgU* (*dnaY*) gene that encodes for tRNA^{Arg(AGG/AG)} resulted in high level production of the target protein (Makrides 1996). The bacterial *bar* gene has 14 arginine codons, none of which are the rare AGA/AGG codons. The *s-bar* gene has five of them, three of which are located within the first 25 codons. Therefore, the likely explanation for the lack of *s-bar* expression in *E. coli* is introduction of the rare AGA and AGG arginine codons in the *s-bar* coding region.

There are proteins, which are toxic to *E. coli* but their expression is desirable in plastid to which it is not toxic. Engineering of these proteins in *E. coli* poses a problem, since the commonly used PEP plastid promoters are active in *E. coli,* thus the gene will be transcribed and the mRNA translated. Incorporation of minor codons in the coding region will prevent translation of these proteins in *E. coli.* Particularly useful in this regard is conversion of arginine codons to AGA/AGG. If no arginine is present in the N-terminal region, an N-terminal fusion may be designed containing multiple AGA/AGG codons to prevent translation of the mRNA.

Plants under field conditions are associated with microbes living in the soil, on the leaves and inside the plants. Gene flow from plastids to these microorganisms has not been shown. However, it would be an added safety measure to incorporate codons in plastid genes, which are rare in the target microorganisms, but are efficiently translated in plastids. Incorporation of AGA/AGG codons into the selective marker genes and the genes of interest will prevent transfer of genes from plants to microbes, which lack the capacity to efficiently translate the AGA/AGG codons. In case of specific plant-microbe associations, based on differences in codon usage preferences genes could be designed which would be expressed in plastids but not in microbes.

Attempts to directly select transplastomic clones after bombardment with the *s-bar* constructs so far has failed. The *s-bar* coding region in Figure 20A contains frequent and rare codons in proportions characteristic of plastid genes. It is possible, that relatively rare codons in a specific context at a critical stage will prevent recovery of plastid transformation events. Examples for tissue-specific translation of mRNAs dependent on tRNA availability are known (Zhou et al., 1999). Therefore, we designed a second synthetic bar gene, *S2*-*bar*, containing only frequent codons (Figure 20B). Plastid transformation with the s2-bar will enable direct selection of plastid transformation events by PPT resistance.

### EXAMPLE 8

### FLUORESCENT ANTIBIOTIC RESISTANCE MARKER FOR FACILE IDENTIFICATION OF TRANSPLASTOMIC CLONES IN TOBACCO AND RICE

Plastid transformation in higher plants is accomplished through a gradual process, during which all the 300-10,000 plastid genome copies are uniformly altered. Antibiotic resistance genes incorporated in the plastid genome facilitate maintenance of transplastomes during this process. Given the high number of plastid genome copies in a cell, transformation unavoidably yields chimeric tissues, in which the transplastomic cells need to be identified and regenerated into plants. In chimeric tissue, antibiotic resistance is not cell autonomous: transplastomic and wild-type sectors both are green due to phenotypic masking by the transgenic cells. Novel genes encoding FLARE-S, a fluorescent antibiotic resistance enzyme conferring resistance to spectinomycin and streptomycin, which were obtained by translationally fusing aminoglycoside 3" - adenylyltransferase [AAD] with the *Aequorea victoria* green fluorescent protein (GFP) are provided in the present example. FLARE-S facilitates distinction of transplastomic and wild-type sectors in the chimeric tissue, thereby significantly reducing the time and effort required to obtain genetically stable transplastomic lines. The utility of FLARE-S to select for plastid transformation events was shown by tracking segregation of transplastomic and wild-type plastids in tobacco and rice plants after transformation with FLARE-S plastid vectors and selection for resistance to spectinomycin and streptomycin, respectively.

Plastid transformation vectors contain a selectable marker gene and passenger gene(s) flanked by homologous plastid targeting sequences (Zoubenko *et al.,* 1994), and are introduced into plastids by biolistic DNA delivery (Svab *et al.*, 1990; Svab and Maliga, 1993) or PEG treatment (Golds *et al.,* 1993; Koop *et al.,* 1996; O'Neill *et al.*, 1993). The selectable marker genes may encode resistance to spectinomycin, streptomycin or kanamycin. Resistance to the drugs is conferred by the expression of chimeric *aadA* (Svab and Maliga, 1993) and *neo* (*kan*) (Carrer *et al.,* 1993) genes in plastids. These drugs inhibit chlorophyll accumulation and shoot formation on plant regeneration media. The transplastomic lines are identified by the ability to form green shoots on bleached wild-type leaf sections. Obtaining a genetically stable transplastomic line involves cultivation of the cells on a selective medium, during which the cells divide at least 16 to 17 times (Moll *et al.,* 1990). During this time wild type and transformed plastids and plastid genome copies gradually sort out. The extended period of genome and organellar sorting yields chimeric plants consisting of sectors of wild-type and transgenic cells (Maliga, 1993). In the chimeric tissue antibiotic resistance conferred by *aadA* or *neo* is not cell autonomous: transplastomic and wild-type sectors are both green due to phenotypic masking by the transgenic tissue. Chimerism necessitates a second cycle of plant regeneration on a selective medium. In the absence of a visual marker this is an inefficient process, involving antibiotic selection and identification of transplastomic plants by PCR or Southern probing. The feasibility of visual identification of transformed sectors greatly reduces the effort required to obtain homoplastomic clones.

The *Aequorea victoria* green fluorescent protein (GFP) is a visual marker, allowing direct imaging of the fluorescent gene product in living cells without the need for prolonged and lethal histochemical staining procedures. Its chromophore forms autocatalytically in the presence of oxygen and fluoresces green when absorbing blue or UV light (Prasher *et al.*, 1992; Chalfie *et al.,* 1994; Heim *et al*., 1994) (reviewed in ref. Prasher, 1995; Cubitt *et al*., 1995; Misteli and Spector, 1997). The *gfp* gene was modified for expression in the plant nucleus by removing a cryptic intron, introducing mutations to enhance brightness and to improve GFP solubility (Pang *et al*., 1996; Reichel *et al*., 1996; Rouwendal *et al*., 1997; Haseloff *et al.,* 1997; Davis and Vierstra, 1998). GFP was used to monitor protein targeting to nucleus, cytoplasm and plastids from nuclear genes (Sheen *et al.*, 1995; Chiu *et al.*, 1996; K 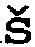 hler *et al.*, 1997), and to follow virus movement in plants (Baulcombe *et al.*, 1995; Epel *et al.*, 1996). GFP has also been used to detect transient gene expression in plastids (Hibberd *et al*., 1998).

The expression of GFP by directly incorporating the *gfp* gene in the plastid genome is described herein. Incorporation of a visual marker, the GFP protein, in the plastid transformation vectors of the present invention facilitates distinction of spontaneous antibiotic resistant mutants and plastid transformants (Svab *et al.*, 1990). Furthermore, transplastomic sectors in the chimeric tissue can be visually identified, significantly reducing the time and effort required for obtaining genetically stable transplastomic lines. The utility of the GFP marker described here is further enhanced by its fusion with the enzyme aminoglycoside 3"-adenylyltransferase [AAD] conferring spectinomycin and streptomycin resistance to plants. Using a marker gene encoding a bifunctional protein, FLARE-S (fluorescent antibiotic resistance enzyme, spectinomycin and streptomycin), prevents physical separation of the two genes and simplifies engineering. Furthermore, fluorescent antibiotic resistance genes enables extension of plastid transformation to cereal crops, in which plastid transformation is not associated with a readily identifiable tissue culture phenotype.

The following protocols are provided to facilitate the practice of the present example.

**Construction of tobacco plastid vectors**. The *aadA16gfp* gene encodes FLARE16-S fusion protein, and can be excised as an NheI-XbaI fragment from plasmid pMSK51, a pBSKSII+ derivative (Genbank Accesssion No. Not yet assigned. The fusion protein was obtained by cloning *gfp* (from plasmid pCD3-326F) downstream of *aadA* (in plasmid pMSK38), digesting the resulting plasmid with BstXI (at the 3' end of the *aadA* coding region) and NcoI (including the *gfp* translation initiation codon) and linking the two coding regions by a BstXI-NcoI compatible adapter. The adapter was obtained by annealing oligonucleotides 5'-GTGGGCAAAGAACTTGTTGAA GGAAAATTGGAGCTAGTAGAAGGTCTTAAAGTCGC-3' and 5'-CATGGCGACTTTAAGACCTTCTACTAGCTCCAATTTTCCTTCAACAAGTTCTTTGC CCACTACC-3'. The adapter connects AAD and GFP with a peptide of 16 amino acid residues (ELVEGKLELVEGLKVA).

The engineered *aadA* gene (Chinault et al., 1986) in plasmid pMSK38 (pBSIIKS+ derivative) has NcoI and NheI sites at the 5' end and BstXI and XbaI sites at the 3' end of the gene. The NcoI site includes the translation initiation codon; the NheI and BstXI sites are in the coding region close to the 5' and 3' ends, respectively; the XbaI site is downstream of stop codon. The mutations were introduced by PCR using oligonucleotides 5'-GGCCATGGGGGCTAGCGAAGCGGTGATCGCCGAAGTATCG-3' and 5'-CGAATTCTAGACATTATTTGCCCACTACCTTGGTGATCTC-3'.

The *gfp* gene in plasmid CD3-326F is the derivative of plasmid psmGFP, encoding the soluble modified version of GFP (accession number U70495) obtained under order number CD3-326 from the Arabidopsis Biological Resource Center, Columbus, OH (Davis and Vierstra, 1998). The *gfp* gene in plasmid CD3-326F is expressed in the PpsbA /TpsbA expression cassette. The *gfp* gene in plasmid CD3-326F was obtained through the following steps. The BamHI-SacI fragment from CD3-326 was cloned into pBSKS+ vector to yield plasmid CD3-326A. The SacI site downstream of the coding region was converted into an XbaI site by blunting and linker ligation (5'-GCTCTAGAGC; plasmid CD3-326B). An NcoI site was created to include the translation initiation codon and at the same time the internal NcoI site was removed by PCR amplification of the coding region N-terminus with primers 5'-CCGGATCCAAGGAGATATAACACCATGGCTAGTAAAGGAGAAGAACTTTTC-3' and 5'-GTGTTGGCCAAGGAACAGGTAGTTTTCC-3'. The PCR-amplified fragment was digested with BamHI and MscI restriction enzymes, and the resulting fragment was used to replace the BamHI-MscI fragment in plasmid CD3-326B to yield plasmid CD3-326C. The *gfp* coding region was excised from plasmid CD3-326C as an NcoI-XbaI fragment and cloned into a *psbA* cassette to yield plasmid CD3-326D. PpsbA and TpsbA are the *psbA* gene promoter and 3'- untranslated region derived from plasmids pJS25 (Staub and Maliga, 1993). TpsbA has been truncated by inserting a HindIII linker downstream of the modified BspHI site (Peter Hajdukiewcz, unpublished). The PpsbA::gfp::TpsbA gene was excised as an EcoRI-HindIII fragment and cloned into EcoRI and HindIII digested pPRV111A, to yield plasmid CD3-326F.

The *aadA16gfp* coding region from plasmid pMSK51 was introduced into two expression cassettes. In plasmid pMSK53 the *aadA16gfp* coding region is expressed in the PrrnLrbcL+DBwt/TpsbA cassette, and encodes the FLARE16-S2 protein (fluorescent antibiotic resistance enzyme, spectinomcyin). PrrnLrbcL+DBwt is described in the previous examples and derives from plasmid pHK14. The construct contains a chimeric promoter composed of the *rrn* operon promoter, the *rbcL* gene leader and downstream box sequence. TpsbA is the *psbA* gene 3' untranslated region, and functions to stabilize the chimeric mRNA. In plasmid pMSK54 the *aadA16gfp* coding region is expressed in the PrrnLatpB+DBwt/TpsbA cassette, and encodes the FLARE16-S1 protein. PrrnLatpB+DBwt derives from plasmid pHK10, and is a chimeric promoter composed of the *rrn* operon promoter, the *atpB* leader and downstream box sequence. See Examples 1-4.

The chimeric *aadA16gfp* genes were introduced into the tobacco plastid transformation vector pPRV111B (Zoubenko *et al.,* 1994). The *aadA* gene was excised from plasmid pPRV111B with EcoRI and SpeI restriction enzymes, and replaced with the EcoRI-SpeI fragment from plasmids pMSK53 and pMSK54 to generate plasmids pMSK57 *(aadA16gfp-S2)* and pMSK56 (*aadA16gfp-S1*).

**Construction of rice plastid vectors**. Plasmid pMSK49 is a rice-specific plastid transformation vector which carries the *aadA11gfp-S3* gene as the selective marker in the *trnV*/*rps12*/*7* intergenic region (GenBank Accession Number: Not yet assigned). Plasmid pMSK49 carries the rice SmaI-SnaBI plastid fragment (restriction sites at nucleotides 122488 and 125 878 in the genome Hiratsuka *et al.,* 1989) cloned into a pBSKSII+ (Stratagene) vector after blunting the SacI and KpnI restriction sites. The XbaI site present in the rice plastid DNA fragment (position at nucleotide 125032 in the genome (Hiratsuka *et al.,* 1989) was removed by filling in and religation. Prior to cloning the selective marker the progenitor plasmid was digested with the BglII restriction enzyme giving rise to a deletion of 119 nucleotides between two proximal BglII sites (positions at 124367 and 124491). The *aadA11gfp-S3* gene was then cloned in the blunted BglII sites.

The *aadA* gene in plasmid pMSK49 was obtained by modifying the *aadA* gene in plasmid pMSK38 (above) to obtain plasmid pMSK39. The modification involved translationally fusing the *aadA* gene product at its N-terminus with an epitope of the human c-Myc protein (amino acids 410-419; EQKLISEEDL Kolodziej and Young, 1991). The genetic engineering was performed by ligating an adapter obtained by annealing complementary oligonucleotides with appropriate overhangs into NcoI-NheI digested pMSK38 plasmid. The oligonucleotides were:
5'-CATGGGGGCTAGCGAACAAAAACTCATTTCTGAAGAAGACTTGc-3' and
5'-CTAGGCAAGTCTTCTTCAGAAATGAGTTTTTGTTCGCTAGCCCC-3'.

The *aadA11gfp* gene encoding FLARE11-S was obtained by linking AAD and GFP with the 11-mer peptide ELAVEGKLEVA. To clone *aadA* and *gfp* in the same polycloning site, *gfp* (EcoRI-HindIII fragment; from plasmid CD3-326F) was cloned downstream of *aadA* in plasmid pMSK39 to obtain plasmid pMSK41. The two genes were excised together as an NheI-HindIII fragment, and cloned into plasmid pMSK45 to replace a kanamycin-resistance gene yielding plasmid pMSK48. Plasmid pMSK45 is a derivative of plasmid pMSK35 which carries the PrrnLT7g10+DB/Ec promoter. The promoter consists of the plastid rRNA operon promoter and the leader sequence of the T7 phage gene 10 leader. In plasmid pMSK48, *aadA* is expressed from the PrrnLT7g10+DB/Ec promoter. The *aadA* and *gfp* genes were then translationally fused with an BstXI-NcoI adapter that links the AAD and GFP with an 11-mer peptide. The adapter was obtained by annealing oligonucleotides 5'-GTGGGCAAAGAACTTGCAGTTGAAGGAAAATTGGAGGTCGC-3' and 5'-CATGGCGACCTCCAATTTTCCTTCAACTGCAAGTTCTTTGCCCACTACC-3', which was ligated into BstXI/NcoI digested pMSK48 plasmid DNA to yield plasmid pMSK49. Plasmid pMSK49 has the rice plastid targeting sequences present in plasmid pMSK35.

**Tobacco plastid transformation**. Tobacco leaves from 4 to 6 weeks old plants were bombarded with DNA-coated tungsten particles using the Dupont PDS1000He Biolistic gun (1100 psi). Transplastomic clones were identified as green shoots regenerating on bleached leaf sections on RMOP medium containing 500mg/L spectinomycin dihydrochloride (Svab abd Maliga, 1993). The spectinomycin resistant shoots were illuminated with UV light (Model B 100AP, UV Products, Upland, California, USA). Shoots emitting green light were transferred to spectinomycin free MS medium (Murashige and Skoog, 1962) (3% sucrose) on which fluorescent (transplastomic) and non- fluorescent (wild-type) sectors formed. Fluorescent sectors were excised, and transferred to selective (500 mg/L spectinomycin) shoot regeneration (RMOP) medium. Regenerated shoots were tested for uniform transformation by Southern analysis.

**Rice plastid transformation**. Callus formation from mature *Oryza* sativa cv. Taipei 309 seeds was induced on a modified CIM medium (Tompson *et al.,* 1986), containing MS salts and vitamins (2 mg/L glycine, 0.5 mg/L nicotinic acid, 0.5 mg/L pyridoxine and 0.1 mg/L thiamine), 2 mg/L 2,4D, 1 mg/L kinetin and 300 mg/L casein enzymatic hydrolysate Type III (Sigma C-1026) and sucrose (30g/L). Embryogenic suspensions from the proliferating embryogenic calli were obtained on the AA medium (Muller and Grafe, 1978). For plastid transformation by the biolistic process rice embryogenic cells were plated on a filter paper on non-selective modified CIM medium (Tompson *et al.,* 1986). The bombarded cells were incubated for 48 hours, transferred to selective liquid AA medium (Muller and Grafe, 1978) (one to two weeks), and then to solid modified RRM regeneration medium (Zhang and Wu, 1988) containing MS salts and vitamins, 100 mg/L myo-inositol, 4 mg/L BAP, 0.5 mg/L IAA, 0.5 mg/L NAA, 30 g/L sucrose and 40 g/L maltose and 100 mg/L streptomycin sulfate on which green shoots appeared in two to three weeks. The shoots were rooted on a selective MS salt medium (Murashige and Skoog, 1962) containing 30 g/L sucrose and 100 mg/L streptomycin sulfate. Leaf samples for PCR analysis and confocal microscopy were taken from plants on selective medium.

**PCR amplification of border fragments**. Total cellular DNA was extracted according to Mettler (Mettler, 1987). The PCR analysis was carried out with a 9:1 mixture of AmpliTaq (Stratagene) and Vent (New England Biolabs) DNA polymerases in the Vent buffer following the manufacturer's recommendations. The left border fragment was amplified with primers 03 (5'-ATGGATGAACTATACAAATAAG-3'and 04 (5'-GCTCCTATAGTGTGACG-3'). The right border fragment was amplified with primers 05 (5'-ACTACCTCTGATAGTTGAGTCG-3') and 06 (5'-AGAGGTTAATCGTACTCTGG-3'). The aadA part of FLARE-S genes was amplified with primers O1 (5'-GGCTCCGCAGTGGATGGCGGCCTG-3') and 02 (5'-GGGCTGATACTGGGCCGGCAGG-3'). Primer positions are shown in Fig. 5A. Note that the same primers can be used in transplastomic tobacco and rice plants expressing FLARE-S.

**Detection of FLARE-S by fluorescence**. FLARE-S expressing sectors in the leaves were visualized by an Olympus SZX stereo microscope equipped for GFP detection with a CCD camera system. Subcellular localization of GFP was verified by laser-scanning confocal microscopy (Sarastro 2000 Confocal Image System, Molecular Dynamics, Sunnyvale, CA). This system includes an argon mixed gas laser with lines at 488 and 568 nm and detector channels. The channels are adjusted for fluorescein and rhodamine images. GFP fluorescence was detected in the FITC channel (488-514 nm). Chlorophyll fluorescence was detected in the TRITC channel (560-580 nm). The images produced by GFP and chlorophyll fluorescence were viewed on a computer screen attached to the microscope and processed using the Adobe PhotoShop software.

**Immunoblot analysis**. Leaves (0.5 g) collected from plants in sterile culture were frozen in liquid nitrogen and ground to a fine powder in a mortar with a pestle. For protein extraction the powder was transferred to a centrifuge tube containing 1 ml buffer [50 mM Hepes/KOH (pH 7.5), 1 mM EDTA, 10 mM potassium acetate, 5 mM magnesium acetate, 1 mM dithiothreitol and 2 mM PMSF] and mixed by flicking. The insoluble material was removed by centrifugation at 4°C for 5 min at 11,600 g. Protein concentration in the supernatant was determined using the Biorad protein assay reagent kit. Proteins (20 µl per lane) were separated in 12% SDS-PAGE (Laemmli, 1970). Proteins separated by SDS-PAGE were transferred to a Protran nitrocellulose membrane (Schleicher and Schuell) using a semi-dry electroblotting apparatus (Bio-Rad). The membrane was incubated with Living Colors Peptide Antibody (Clontech) diluted 1 to 200. FLARE-S was visualized using ECL chemilluminescence immunoblot detection on X-ray film. FLARE-S on the blots was quantified by comparison with a dilution series of commercially available purified wild-type GFP (Clontech).

### RESULTS AND DISCUSSION

### Tobacco plastid vectors with FLARE-S as the selectable marker.

Two FLARE-S fusion proteins were tested in *E. coli.* In one, the AAD and GFP were linked by an 11-mer (ELAVEGKLEVA), in the second by a 16-mer (ELVEGKLELVEGLKVA) linker. For transformation in tobacco, the *aadA16gfp* coding region (16-mer linker) was expressed in two cassettes known to mediate high levels of protein accumulation in plastids. Both utilize the strongest known plastid promoter driving the expression of the ribosomal RNA operon (Prrn), and the 3'-UTR of the highly expressed psbA gene (TpsbA) for the stabilization of the chimeric mRNAs. The PrrnLatpB+wtDB (plasmid pMSK56) and PrrnLrbcL+DBwt (plasmid pMSK57) promoters utilize the *atpB* or *rbcL* gene leader sequences and the coding region N-termini with the downstream box (DB) sequence, respectively. Due to inclusion of the DB sequence in the chimeric genes, the proteins encoded by the two genes are slightly different, having 14 amino acids of the ATP-ase β subunit (*atpB* gene products) or ribulose 1,5-bisphosphate carboxylase/oxygenase (*rbcL* gene product) translationally fused with FLARE16-S (FLARE16-S1 and FLARE16-S2, respectively). To obtain a plastid transformation vector with the fluorescent spectinomycin resistance genes, the chimeric genes were cloned into the *trnV*/*rps12*/*7* plastid intergenic region in plastid vector pPRV111B. Plasmids pMSK56 and pMSK57 (Fig. 23) express FLARE16-S1 and FLARE16-S2, respectively, as markers.

**Identification of transplastomic tobacco clones by fluorescence**. Transformation was carried out by biolistic delivery of pMSK56 and pMSK57 plasmid DNA into chloroplast. The bombarded leaves were transferred onto selective (500 mg/L spectinomycin) shoot regeneration medium. Wild-type leaves on this medium bleach and form white callus. Cells with transformed plastids regenerate green shoots. The leaves on the selective medium were regularly inspected with a hand-held long-wave UV lamp for FLARE-S fluorescence.

No fluorescence could be detected in young shoots (3 to 5 mm in size) developing on pMSK56-bombarded leaves. However, formation of bright sectors in the leaves was observed, when these small shoots were transferred onto non-selective plant maintenance medium. In contrast, cultures bombarded with plasmid pMSK57 yielded small fluorescent shoots at an early stage. These fluorescent shoots, and some of the non-fluorescent ones, developed into plants with bright sectors on non-selective plant maintenance medium. Therefore, FLARE16-S2 is useful for early detection of plastid transformation events. FLARE16-S2 fluorescence in young shoots on a selective medium should be due to relatively high levels of FLARE16-S2. Higher levels of FLARE16-S2 are also indicated by the brighter sectors in variegated leaves expressing FLARE16-S2 as compared to FLARE16-S1.

The size of sectors was different in individual shoots. FLARE-S expression in different leaf layers was also obvious. With the traditional selection for spectinomycin resistance, the transplastomic and wild-type sectors are not visible. Regeneration of plants with uniformly transformed plastid genomes was greatly facilitated by the fluorescing sectors expressing FLARE-S, which could be readily identified in UV light, dissected, and transferred for a second cycle of plant regeneration on spectinomycin-containing (500 mg/L) selective medium.

Given the high levels of FLARE-S accumulation we were interested to find out, if FLARE-S is toxic to plants. We expected that toxicity should be manifested as lower transformation efficiencies. Bombardment of 30 tobacco leaves with plasmids pMSK56 and pMSK57 yielded 71 and 89 spectinomycin resistant clones, respectively. Out of these, 61 and 77 lines were verified as transplastomic by fluorescence. Plastid transformation in a subset of these was confirmed by confocal laser scanning microscopy (7 clones each; see below) and Southern analysis (4 clones). The frequency of plastid transformation events with the FLARE-S -expressing genes was slightly higher (-2 instead of -1 per bombardment) than reported earlier with a chimeric *aadA* gene at the same insertion site (Svab and Maliga, 1993). Therefore, we assume that accumulation of FLARE-S at high levels is not detrimental. Lack of toxicity is also supported by the apparently normal phenotype of the plants in the greenhouse (not shown).

**Localization of FLARE-S to tobacco plastids by confocal microscopy**. Due to phenotypic masking, transplastomic and wild type sectors in a chimeric leaf are both green on a selective medium. However, we have found that in chimeric leaf sectors in the same cell some plastids express FLARE-S while others do not, when observed by confocal microscopy (Fig. 24). FLARE-S and chlorophyll fluorescence were detected separately in the fluorescein and rhodamine channels, respectively. The two images were then overlaid confirming that FLARE-S fluorescence derives from chloroplasts.

Expression of FLARE-S was also studied in non-green plastid types including the chromoplasts in petals and the non-green plastids in root cells (Fig. 24b,f). These studies were carried out in plants, which were homoplastomic for the transgenomes. Homoplastomic state was important, since in non-green tissues chlorophyll could not be used for confirmation of the organelles as plastids. Since FLARE-S expression could be readily detected in chloroplasts as well as non-green plastids, the plastid rRNA operon promoter is apparently active in all plastid types.

### FLARE-S accumulation in tobacco leaves.

Accumulation of FLARE-S in homoplastomic leaves was tested using the commercially available GFP antibody, recognizing the GFP portion (239 amino acid residues) of FLARE16-S (520 amino acids). FLARE16-S1 (532 amino acids) was -8 %, whereas FLARE16-S2 (532 amino acids) was -18 % of total soluble leaf protein (Fig. 25). To calculate FLARE16-S concentrations, a GFP dilution series was used as a reference, and the values were than increased by 2.6 to correct for the larger size of the FLARE16-S1 and -S2 proteins.

**Tracking plastid transformation in rice by FLARE-S expression**. In rice, plant regeneration is from non-green embryogenic cells. Encouraged by FLARE-S expression in non-green tobacco plastids, we attempted to transform the non-green plastids of embryogenic rice tissue-culture cells. Plastid transformation was carried out using a rice-specific vector expressing FLARE11-S3 and targeting insertion of the *aadA11gfp-S3* gene in the *trnV*/*rps12*/*7* intergenic region. The location of the insertion site and the size of plastid targeting sequences in the rice vector are similar to the tobacco vectors shown in Fig. 23.

Plastid transformation in rice was carried out by bombardment of embryogenic rice suspension culture cells using gold particles coated with plasmid pMSK49 DNA. Rice cells, as most cereals, are naturally resistant to spectinomycin (Fromm *et al.,* 1987). FLARE-S, however, confers resistance to streptomycin as well (Svab and Maliga, 1993). Therefore, selection for transplastomic lines was carried out on selective streptomycin medium (100 mg/L). Streptomycin at this concentration inhibits the growth of embryogenic rice cells. After bombardment, the rice cells were first selected in liquid embryogenic AA medium, then on the solid plant regeneration medium, on which the surviving resistant cells regenerated green shoots (12 in 25 bombarded plates). These shoots were rooted, and grown into plants. PCR amplification of border fragments in DNA isolated from the leaves of these plants confirmed integration of *aadA11gfp-S3* sequences in the plastid genome (Fig. 26). The left and right border fragments can not be amplified if the gene is integrated into the nuclear genome, as one of the primers (04 or 06) of the pairs is outside the plastid targeting regions.

FLARE11-S3 expression in the leaves of two of the PCR-positive plants was tested by confocal laser-scanning microscopy. In rice, as in tobacco, the FLARE-S marker confirmed segregation of transplastomic and wild-type plastids (Fig. 27). In rice only a small fraction of chloroplasts expressed FLARE-S. Since individual cells marked with arrows in Fig. 27 contained a mixed population of wild-type and transgenic chloroplasts, FLARE-S in these cells could be expressed only from the plastid genome. Integration of *aadA11gfp-S3* into the nuclear genome downstream of plastid-targeting transit peptide would result in uniform expression of FLARE-S in each of the chloroplasts within the cell.

The sequences of the selectable marker genes of the invention are provided in Figures 28-34. Figure 35 depicts a table describing the selectable marker genes disclosed in the present example.

Direct visual identification of transplastomic sectors requires high level expression of FLARE-S in plastids. High GFP expression levels in Arabidopsis were toxic, interfering with plant regeneration. Toxicity of wild-type (insoluble) GFP was linked to GFP accumulation in the nucleus and cytoplasm, and could be eliminated by targeting it to the endoplasmic reticulum (Haseloff *et al.*, 1997). GFP aggregates were also cytotoxic to *E. coli* cells (Crameri *et al.*, 1996). To enhance fluorescence intensity and to avoid cytotoxicity, soluble versions of the codon-modified GFP were obtained (Davis and Vierstra, 1998). We have utilized the gene for a soluble-modified GFP described by Davis and Vierstra (Davis and Vierstra, 1998) to create variants of FLARE-S, a fusion protein, which does not have an apparent cytotoxic effect. The frequency of plastid transformation, if affected at all, is increased rather then decreased. In tobacco, we normally obtain one transplastomic clone per bombarded leaf sample (Svab and Maliga, 1993), whereas with the FLARE-S genes on average we could recover two clones per sample. Plant regeneration from highly fluorescent tissue was readily obtained, and the regenerated plants have a phenotype indistinguishable from the wild type.

Plastid transformation in rice requires expression of the selective marker in non-green plastids. The rRNA operon has two promoters, one for the eubacterial-type (PEP) and one for the phage-type (NEP) plastid RNA polymerase. The promoter driving FLARE-S expression is recognized only by the eubacterial-type plastid RNA polymerase. Previously, it was assumed that the eubacterial-type promoter is active only in chloroplasts (Maliga, 1998). Accumulation of FLARE-S in roots and petals indicates that PEP is also active in non-green plastids.

Plastid transformation is a process that unavoidably yields chimeric plants, since cells of higher plants contain a large number (300 to 50000) of plastid genome copies (Bendich, 1987), out of which initially only a few are transformed. High level expression of FLARE-S in plastids provides the means for visual identification of transplastomic sectors, even if they are present in a chimeric tissue. GFP and AAD could be expressed from two different genes in a plastid transformation vector. However, transformation with a marker gene encoding a bifunctional protein prevents separation of the two genes and simplifies engineering. The fluorescent selective marker will significantly reduce the work required to obtain genetically stable plastid transformants in tobacco, a species in which plastid transformation is routine. The bottleneck of applying plastid transformation in crop improvement is the lack of technology. In tobacco, chimeric clones with transformed plastids are readily identified by shoot regeneration (Svab *et al.*, 1990). In Arabidopsis, clones with transformed plastids are identified by greening (Sikdar *et al.*, 1998). We have shown here that FLARE-S is a suitable marker to select for transplastomes in embryogenic rice cells, which lack the visually identifiable tissue culture phenotypes exploited in tobacco and Arabidopsis. Data presented here are the first example for stable integration of foreign DNA into the rice plastid genome. These rice plants are heteroplastomic. Uniformly transformed rice plants will be obtained by further selection on streptomycin medium and screening the embryogenic cells for FLARE-S expression. Thus, the FLARE-S marker system will enable extension of plastid transformation to cereal crops.

### The utility of the new chimeric promoters

The σ⁷⁰-type plastid ribosomal RNA operon promoter, Prrn, is the strongest known plastid promoter expressed in all tissue types. The ultimate product of this promoter in the plastid is RNA not protein. Therefore, a series of chimeric promoters were constructed to facilitate protein accumulation from Prrn, using expression of the neomycin phosphotransferase (NPTII) enzyme as the reference protein.
1) The expression cassettes have distinct tissue-specific expression profiles. Some of the expression cassettes described here will facilitate relatively high levels of protein expression in all tissues, including leaves, roots and seeds. Other cassettes have different expression profiles: for example will facilitate moderate levels of protein accumulation in the leaves while lead to relatively high levels of protein accumulation in the roots. Accumulation of a protein at levels of 10% to 50% of total soluble protein is considered high-level protein expression; low-levels of protein expression would be in the range of ≤0.1% total soluble cellular protein.
2) Efficiency of the selectable marker gene depends on the rate at which the gene product accumulates during the early stage of transformation. Since initially present only in a few copies per cell, high levels of expression from a few copies will provide protection from toxic substances early on, facilitating efficient recovery of transformed lines. The expression cassettes will be useful to drive the expression of the genes conferring resistance to the antibiotics streptomycin, spectinomycin and hygromycin, and the herbicides phosphinotrycin and glyphosate. In such applications addition of amino acids at the N-terminus is acceptable, as long as it does not interfere with the expression of the selectable marker genes. NPTII is such an enzyme. In cases like NPTII, an N-terminal fusion and thereby the mRNA "Downstream Box" sequences give an additional at least two to four-fold increase in protein levels. The -DB construct which relied on an *NheI* site, and involved addition of one (N-terminal) amino acid of the source gene coding region is convenient, but is not necessary. When translational fusion is not feasible due to inactivation of proteins, seamless in-frame constructs may be created by PCR methods outlined in the application.
3) A second major area on which application of the chimeric promoters is extremely useful is protein expression for pharmaceutical, industrial or agronomic purposes. The examples include, but are not restricted to, production of vaccines, healthcare products like human hemoglobin, industrial or household enzymes.

### REFERENCES

Allen, G.C., Hall, G.J., Michalowski, S., Newman, W., Spiker, S., Weissinger, A.K. and Thompson, W.F. (1996) High-level transgene expression in plant cells: effects of a strong scaffold attachment region from tobacco. Plant Cell, 8, 899-913.
Allison LA, Simon LD, Maliga P (1996) Deletion of rpoB reveals a second distinct transcription system in plastids of higher plants. EMBO J 15: 2802-2809
Arntzen, C.J. (1997) High-tech herbal medicine: plant-based vaccines [news]. Nature Biotechnology, 15, 221-222.
Baulcombe, D. C. Chapman, S. and Cruz, S. S. 1995. Jellyfish green fluorescent protein as a reporter for virus infections. Plant J. 7: 1045-1053.
Beck, E., Ludwig, G., Auerswald, E.A., Reiss, B. and Schaller, H. (1982) Nucleotide sequence and exact localization of the neomycin phosphotransferase gene from transposon Tn5. Gene, 19, 327-336.
Bendich, A. J. 1987. Why do chloroplasts and mitochondria contain so many copies of their genome? Bio-essays 6: 279-282.
Bonham-Smith, P.C. and Bourque, D.P. (1989) Translation of chloroplast-encoded mRNA: potential initiation and termination signals. Nucleic Acids Res. 17, 2057-2080.
Cao, J., Duan, X., McElroy, D., and Wu, R. (1992) Regeneration of herbicide resistant transgenic rice plants following microprojectile-mediated transformation of suspension culture cells. Plant Cell Report 11:586-591.
Carrer, H. and Maliga, P. (1995) Targeted insertion of foreign genes into the tobacco plastid genome without physical linkage to the selectable marker gene. Biotechnology, 13, 791-794.
Carrer, H., Hockenberry, T.N., Svab, Z. and Maliga, P. (1993) Kanamycin resistance as a selectable marker for plastid transformation in tobacco. Mol. Gen. Genet., 241, 49-56.
Chalfie, M., Tu, Y., Euskirchen, G., Ward, W. W. and Prasher, D. C. 1994. Green fluorescent protein as a marker for gene expression. Science 263: 802-805.
Chaudhuri, S. and Maliga, P. (1996) Sequences directing C to U editing of the plastid psbL mRNA are located within a 22 nucleotide segment spanning the editing site. EMBO J., 15, 5958-5964.
Chinault, A.C., Blakesley, V.A., Roessler, E., Willis, D.G., Smith, C.A., Cook, R.G., and Fenwick, R.G. 1986. Characterization of transferable plasmids for Shigella flexneri 2a that confer resistance trimethoprim, streptomycin and sulfonamides. Plasmid 15: 119-131.
Chiu, W-L., Niwa, Y., Zeng, W., Hirano, T., Kobayashi, H. and Sheen, J. 1996. Engineered gfp as a vital reporter in plants. Curr. Biol. 6: 325-330.
Conrad, U. and Fiedler, U. (1998) Compartment-specific accumulation of recombinant immunoglobulins in plant cells: an essential tool for antibody production and immunomodulation of physiological functions and pathogen activity. Plant Mol. Biol., 38, 101-109.
Corriveau, J.L., and Coleman, A.W. (1988) Rapid screening method to detect potential biparental inheritance of plastid DNA and the results for over 200 angiosperm species. Amer. J. Bot. 75:1443-1458.
Crameri, A., Whitehorn, E. A., Tate, E. and Stemmer, W. P. C. 1996. Improved green fluorescent protein by molecular evolution by DNA shuffling. Nature Biotech. 14: 315-319.
Cubitt, A. B., Heim, R., Adams, S. R., Boyd, A. E., Gross, L. A. and Tsien, R. Y. 1995. Understanding, improving and using green fluorescent proteins. Trends Biochem. Sci. 20: 448-455.
Dams, E., Hendriks, L., Van de Peer, Y., Neefs, J.M., Smits, G., Vandenbempt, I. and De Wachter, R. (1988) Compilation of small ribosomal subunit RNA sequences. Nucleic Acids Res., 16 Suppl, r87-173.
Daniell, H., Datta, R., Varma, S., Gray, S., and Lee, S.B. (1998) Containement of herbicide resistance through genetic engineering of the chloroplast genome. Nat. Biotech. 16:345-348.
Davis, S.J., and Vierstra, R.D. 1998. Soluble, highly fluorescent variants of green fluorescent protein (GFP) for use in higher plants. Plant Mol. Biol. 36: 521-528.
De Block, M., Botterman, J., Vandewiele, M., Dockx, J., Thoen, C., Gossele, V., Rao, Movva, N., Thompson, C., Van Montagu, M., and Leemans, J. (1987). Engineering herbicide resistance in plants by expression of a detoxifying enzyme. EMBO J. 6:2513-2518.
De Block, M., De Brouwer, D., Tenning, P. (1989). Transformation of Brassica napus and Brassica oleracea using Agrobacterium tumefaciens and the expression of the bar and neo genes in the transgenic plants. Plant Physiol . 91:694-701.
Deana, A., Ehrlich, R. and Reiss, C. (1998) Silent mutations in the Escherichia coli ompA leader peptide region strongly affect transcription and translation in vivo. Nucleic Acids Res., 26, 4778-4782.
Ellis, R.J. (1979) The most abundant protein in the world. Trends Biochem. Sci., 4, 241-244.
Epel, B.L., Padgett, H.S., Heinlein, M., and Beachy, R. 1996. Plant virus movement protein dynamics probed with GFP-protein fusion. Gene 173: 75-79.
Etchegaray, J.P. and Inouye, M. (1999) Translational enhancement by an element downstream of the initiation codon in Escherichia coli. J. Biol. Chem., 274, 10079-10085.
Faxin, M., Plumbridge, J. and Isaksson, L.A. (1991) Codon choice and potential complementarity between mRNA downstream of the initiation codon and bases 1471-1480 in 16S ribosomal RNA affects expression of glnS. Nucleic Acids Res., 19, 5247-5251.
Fromm, H., Edelman, M., Aviv, D. and Galun, E. 1987. The molecular basis ofrRNA-dependent spectinomycin resistance in Nicotiana chloroplasts. EMBO J. 11: 3233-3237.
Gallo-Meagher, M., and Irvine, J.E. (1996) Herbicide resistant transgenic sugarcane plants containing the bar gene. Crop Sci. 36:1367-1374.
Golds, T., Maliga, P., and Koop, H.U. 1993. Stable plastid transformation in PEG-treated protoplasts of Nicotiana tabaccum. Biotechnology 11: 95-97.
Gray, A.J., and Raybould, A.F. (1988) Reducing transgene escape routes. Nature 392:653-654.
Hajdukiewicz, P., Allison, L.A., Maliga, P. (1997) The two plastid RNA polymerases encoded by the nuclear and plastid compartments transcribe distinct groups of genes in tobacco plastids. EMBO J. 16, 4041-4048.
Haseloff, J., Siemering, K. R., Prasher, D. C., and Hodge, S. 1997. Removal of a cryptic intron and subcellular localization of green fluorescent protein are required to mark transgenic Arabidopsis plants brightly. Proc. Natl. Acad. Sci. USA 94: 2122-2127.
Hecker, K.H. and Roux, K.H. (1996) High and low annealing temperatures increase both specificity and yield in touchdown and stepdown PCR. Biotechniques, 20, 478-485.
Heim, R., Prasher, D. C. and Tsien, R. Y. 1994. Wavelength mutations and posttranslational autooxidation of green fluorescent protein. Proc. Natl. Acad. Sci. USA 91: 12501-12504.
Hibberd, J.M., Linley, P.J., Khan, M.S., and Gray, J.C. 1998. Transient expression of green fluorescent protein in various plastid types following micro-projectile bombardment. Plant J. 16: 627-632.
Hiratsuka, J., Shimada, H., Whittier, R., Ishibashi, T., Sakamoto, M., Mori, M., Kondo, C., Honji, Y., Sun, C-R., Meng, B-Y., Li, U-Q., Kanno, A., Nishizawa, Y., Hirai, A., Shinozaki, K., and Sugiura, M. 1989. The complete sequence of the rice (Oryza sativa) chloroplast genome: Intermolecular recombination between distict tRNA genes accounts for a major plastid DNA inversion during the evolution of the cereals. Mol. Gen. Genet. 217: 185-194.
Hirose, T. and Sugiura, M. (1996) Cis-acting elements and trans-acting factors for accurate translation of chloroplast psbA mRNAs: development of an in vitro translation system from tobacco chloroplasts. EMBO J., 15, 1687-1695.
Houdt, H.V., Ingelbrecht, I., Montagu, M.V. and Depicker, A. (1997) Post-transcriptional silencing of a neomycin phosphotransferase II transgene correlates with the accumulation of unproductive RNAs and with increased cytosine methylation of 3' flanking positions. Plant J., 12, 379-392.
Ito, K., Kawakami, K. and Nakamura, Y. (1993) Multiple control of Escherichia coli lysyl-tRNA synthetase expression involves a transcriptional repressor and a translational enhancer element. Proc. Natl. Acad. Sci. USA, 90, 302-306.
Kane, J.F. (1995) Effects of rare codon clusters on high-level expression of heterologous proteins in Escherichia coli. Current Opinion In Biotechnology, 6, 494-500.
Khan, M.S. and Maliga, P. (1999) Fluorescent antibiotic resistance marker to track plastid transformation in higher plants. Nature Biotechnology, in press.
Kling, J. (1996) Could transgenic supercrops one day breed superweeds? Science 274:180-181.
Kvhler, R.H., Cao, J., Zipfel, W.R., Webb, W.W., and Hanson, M.R. 1997. Exchange of protein molecules through connections between higher plant plastids. Science 276: 2039-2042.
Kolodziej, P. A., and Young, R. A. 1991. Epitope tagging and protein surveillance. Methods Enzymol. 194: 508-519.
Koop, H.U., Steinm|ller, K., Wagner, H., Rvssler, C., Eibl, C., and Sacher, L. 1996. Integration of foreign sequences into the tobacco plastome via PEG-mediated protoplast transformation. Planta 199: 193-101.
Kusnadi, A., Nikolov, Z. and Howard, J. (1997) Production of recombinant proteins in transgenic plants: practical considerations. Biotechnology and Bioengineering, 56, 473-484.
Laemmli, U. K. 1970. Cleavage of structural proteins during the assembly of the head of the bacteriophage T4. Nature 227: 680-685.
Lefebvre, B., Formstecher, P. and Lefebvre, P. (1995) Improvement of the gene splicing overlap method (SOE) method. BioTechniques 19: 186-187
Maier, R.M., Neckermann, K., Igloi, G.L. and Kvssel, H. (1995) Complete sequence of the maize chloroplast genome: gene content, hotspots of divergence and fine tuning of genetic information by transcript editing. J. Mol. Biol., 251, 614-628.
Makrides, S.C. (1996) Strategies for achieving high-level expression of genes in Escherichia coli. Microbiological Reviews, 60, 512-538.
Maliga P (1995). Biolistic transformation of tobacco cells with nuclear drug resistance genes. In Maliga P, Klessig D, Cashmore A, Gruissem W, Varner J (eds), Methods in Plant Molecular Biology-A Laboratory Manual. Cold Spring Harbor Laboratory Press, Cold Spring Harbor, NY, pp. 37-52.
Maliga, P. (1993) Towards plastid transformation in flowering plants. Trends in Biotechnology 11:101-107.
Maliga, P. 1998. Two plastid RNA polymerases of higher plants: an evolving story. Trends Plant Sci. 3: 4-6.
Mayfield, S.P., Cohen, A., Danon, A. and Yohn, C.B. (1994) Translation of the psbA mRNA of Chlamydomonas reinhardtii requires a structured RNA element contained within the 5' untranslated region. J. Cell Biol., 127, 1537-1545.
McBride, K.E., Scaaf, D.J., Daley, M. and Stalker, D. (1994) Controlled expression of plastid transgenes in plants based on a nuclear-encoded and plastid-targeted T7 RNA polymerase. Proc. Natl. Acad. Sci. USA 91, 7301-7305.
McBride, K.E., Svab, Z., Schaaf, D.J., Hogan, P.S., Stalker, D.M. and Maliga, P. (1995) Amplification of a chimeric Bacillus gene in chloroplasts leads to an extraordinary level of an insecticidal protein in tobacco. Biotechnology (N Y), 13, 362-365.
Medgyesy, P., Pay, A., and Marton, L. (1986) Transmission of paternal chloroplasts in Nicotiana. Mol. Gen. Genet. 204:195-198.
Mettler, I.J. 1987. A simple and rapid method for minipreparation of DNA from tissue-cultured plant cells. Plant Mol. Biol. Rep. 5: 346-349.
Mikkelsen, T.R., Andersen, B., and Jorgensen, R.B. (1996). The risk of crop transgene spread. Nature 380:31.
Misteli, T., and Spector, D.L. 1997. Applications of the green fluorescent protein in cell biology and biotechnology. Nature Biotech. 15: 961-964.
Mitta, M., Fang, L. and Inouye, M. (1997) Deletion analysis of cspA of Escherichia coli: requirement of the AT-rich UP element for cspA transcription and the downstream box in the coding region for its cold shock induction. Mol Microbiol, 26, 321-335.
Moll, B., Posby, L., and Maliga, P. 1990. Streptomycin and lincomycin resistance are selective plastid markers in cultured Nicotiana cells. Mol. Gen. Genet. 221: 245-250.
Muller, A.J., and Grafe, R. 1978. Isolation and characterization of cell lines of Nicotiana tobacum lacking nitrate reductase. Mol. Gen. Genet. 161: 67-76.
Murashige, T. and Skoog, F. (1962) A revised medium for the growth and bioassay with tobacco tissue culture. Physiologia Plantarum, 15, 473-497.
Nickelsen, J., Fleischmann, M., Boudreau, E., Rahire, M. and Rochaix, J.D. (1999) Identification of cis-acting RNA leader elements required for chloroplast psbD gene expression in Chlamydomonas. The Plant Cell, 11, 957-970.
Olins, P.O., Devine, C.S., Rangwala, S.H., Kavka, K.S. (1988) The T7 phage gene 10 leader RNA, a ribosome-binding site that dramatically enhances the expression of foreign genes in Escherichia coli. Gene 73: 227-235.
O'Neill, C., Horvath, G.V., Horvath, E., Dix, P.J., and Medgyesy, P. 1993. Chloroplast transformation in plants: polyethylene glycol (PEG) treatment of protoplasts is an alternative to biolistic delivery system. Plant J. 3: 729-738.
Orozco,E.M., Chen,L.J. and Eilers,R.J. (1990) The divergently transcribed rbcL and atpB genes of tobacco plastid DNA are separated by nineteen base pairs. Curr. Genet., 17, 65-71.
Pang, S-Z., DeBoer, D.L., Wan, Y., Ye, G., Layton, J.G., Neher, M.K., Armstrong, C.L., Fry, J.E., Hinchee, M.A.W., and Fromm, M.E. 1996. An improved green fluorescent protein gene as avital marker in plants. Plant Physiol. 112: 893-900.
Prasher, D. C. 1995. Using GFP to see the light. Trends Genet. 11: 320-323.
Prasher, D. C., Eckenrode, V. K., Ward, W. W., Predergast, F. G. and Cormier, M. J. 1992. Primary structure of the Aequorea victoria green-fluorescent protein. Gene 111: 229-233.
Reichel, C., Mathur, J., Eckes, P., Langenkemper, K., Koncz, C., Schell, J., Reiss, B., and Maas, C. 1996. Enhanced green fluorescence by the expression of an Aequorea victoria green fluorescent protein mutant in mono- and dicotyledonous plant cells. Proc. Natl. Acad. Sci. USA 93: 5888-5893.
Rochaix, J.D. (1996) Post-transcriptional regulation of chloroplast gene expression in Chlamydomonas reinhardtii. Plant Mol. Biol., 32, 327-341.
Rouwendal., G.J.A., Mendes, O., Wolbert, E.J.H., and de Boer, A.D., 1997. Enhanced expression in tobacco of the gene encoding green fluorescent protein by modification of its codon usage. Plant Mol. Biol. 33: 989-999.
Saghai-Maroof, M.A., Soliman, K.M., Jorgensen, R.A., and Allard, R.W. (1984) Ribosomal DNA spacer-length polymorphisms in barley: mendelian inheritance, chromosomal location, and population dynamics. Proc. Natl. Acad. Sci. USA 81: 8014-8018.
Sakamoto, W., Kindle, K.L. and Stern, D.B. (1993) In vivo analysis of Chlamydomonas chloroplast petD gene expression using stable transformation of beta-glucuronidase translational fusions. Proc. Natl. Acad. Sci. USA, 90, 497-501.
Sawasaki, T., Seki, M., Anzai, H., Irifune, K., and Morikawa, H. (1994). Stable transformation of Arabidopsis with the bar gene using particle bombardment. Transgenic Res. 3:279-286.
Serino, G., and Maliga, P. (1997) A negative selection scheme based on the expression of cytosine deaminase in plastids. Plant J . 12:697-701.
Sheen, J., Hwang, S., Niwa, Y., Kobayashi, H., and Galbraith, D.W. 1995. Green fluorescent protein as a new vital marker in plant cells. Plant J. 8: 777-784.
Shimada, H., and Sugiura, M. (1991) Fine structural features of the chloroplast genome: comparison of the sequenced chloroplast genomes. Nucleic Acids Res. 19:983-995.
Shinozaki, K. and Sugiura, M. (1982) The nucleotide sequence of the tobacco chloroplast gene for the large subunit of ribulose-1,5-bisphosphate carboxylase /oxygenase. Gene 20: 91-102.
Shinozaki, K., Deno, H., Wakasugi, T. and Sugiura, M. (1986a) Tobacco chloroplast gene coding for subunit I of proton-translocating ATPase: comparison with the wheat subunit I and E. coli subunit b. Curr. Genet., 10, 421-423.
Shinozaki, K., Ohme, M., Tanaka, M., Wakasugi, T., Hayashida, N., Matsabayashi, T., Zaita, N., Chungwongse, J., Obokata, J., Yamaguchi-Shinozaki, K., Deno, H., Kamogashira, T., Yamada, K., Kasuda, J., Takaiwa, F., Kato, A., Todoh, N., Shimada, H. and Sugiura, M. (1986b) The complete sequence of the tobacco chloroplast genome: its gene organization and expression. EMBO J., 5, 2043-2049.
Sikdar, S.R., Serino, G., Chaudhuri, S., and Maliga, P. 1998. Plastid transformation in Arabidopsis thaliana. Plant Cell Rep. 18: 20-24.
Spencer, T.M., Gordon-Kamm, W.J., Daines, R.J., Start, W.G., and Lemaux, P.G. (1990). Bialaphos selection of stable transformants from maize cell culture. Theor. Appl. Genet. 79:625-631.
Sprengart, M.L., Fuchs, E. and Porter, A.G. (1996) The downstream box: an efficient and independent translation initiation signal in Escherichia coli. EMBO J., 15, 665-674.
Sriraman, P., Silhavy, D. and Maliga, P. (1998a) The phage-type PclpP-53 plastid promoter comprises sequences downstream of the transcription initiation site. Nucleic Acids Res., 26, 4874-4879.
Sriraman, P., Silhavy, D. and Maliga, P. (1998b) Transcription from heterologous rRNA operon promoters in chloroplasts reveals requirement for specific activating factors. Plant Physiol., 117, 1495-1499.
Sriraman, P., Silhavy, D., Maliga, P. (1998b) Transcription from heterologous ribosomal RNA operon promoters in chloroplasts reveals requirement for specific activating factors. Plant Physiol. 117: 1495-1499.
Staub, J., Maliga, P. (1993) Accumulation of D1 polypeptide in tobacco plastids is regulated via the untranslated region of the psbA mRNA. EMBO J. 12:601-606
Staub, J.M. and Maliga, P. (1994) Translation of psbA mRNA is regulated by light via the 5'-untranslated region in tobacco plastids. Plant J., 6, 547-553.
Stemmer, W.P., Crameri, A., Ha, K.D., Brennan, T.M. and Heyneker, H.L. (1995) Single-step assembly of a gene and entire plasmid from large numbers of oligodeoxyribonucleotides. Gene, 164, 49-53.
Stern, D.B., Higgs, D.C. And Yang, J. (1997) Transcription and translation in chloroplasts. Trends Plant Sci. 2, 308-315.
Stiekema WJ, Heidekamp F, Dirkse WG, van Beckum J, deHaan P, ten Bosch C, Louwerse JD (1988) Molecular cloning and analysis of four potato tuber mRNAs. Plant Mol Biol 11: 255-269
Strauch, E., Wohlleben, , W., and P|hler, A. (1988) Cloning of phosphinothricin acetyltransferase gene from Streptomyces viridochromogenes T|4494 and its expression in Streptomyces lividans and Escherichia coli.. Gene 63:65-74.
Studier, F.W., Rosenberg, A.H., Dunn, J.J. and Dubendorff, J.W. (1990) Use of T7 RNA polymerase to direct expression of cloned genes. Methods Enzymol. 185: 60-89.
Sugita, M. and Sugiura, M. (1984) Nucleotide sequence and transcription of the gene for the 32,000 dalton thylakoid membrane protein from Nicotiana tabacum. Mol. Gen. Genet. 195: 308-313.
Svab, Z., Hajdukiewicz, P., and Maliga, P. 1990. Stable transformation of plastids in higher plants. Proc. Natl. Acad. Sci. USA 87: 8526-8530.
Svab, Z., and Maliga, P. (1993). High-frequency plastid transformation in tobacco by selection for a chimeric aadA gene. Proc. Natl. Acad. Sci. USA 90:913-917.
Tachibana. K., Watanabe. T., Sekizawa, T., and Takematsu, T. (1986) Action mechanism of bialaphos II Accumulation of ammonia in plants treated with bialaphos. J. Pest. Sci. 11:33-37.
Tanaka, M., Obokata, J., Chunwongse, J., Shinozaki, K., Sugiura, M. (1987) Rapid splicing and stepwise processing of a transcript from the psbB operon in tobacco chloroplasts: Determination of the intron sites in petB and petD. Mol. Gen. Genet. 209: 427-431.
Thompson, C.J., Movva, N.R, , Tizard, R., Crameri, R., Davies, J.E., Lauwereys, M., Botterman, J. (1987) Characterization of the herbicide-resistance gene bar from Streptomyces hygroscopicus. EMBO J. 6:2519-2523.
Timmons, A.M., Charters, Y.M., Crawford, J.W., Burn, D., Scott, S.E., Subbels, S.J., Wilson, N.J., Robertson, A., O'Brian, E.T., Squire, G.R., and Wilkinson, M.J. (1996) Risks from transgenic crops. Nature 380:487.
Tompson, J.A., Abdullah, R., and Cocking, E.C. 1986. Protoplast culture of rice using media solidified with agarose. Plant Science 47: 123-133.
Uchida, K (1992) Recombination and amplification of multiple portions of genomic DNA by a modified polymerase chain reaction. Anal. Biochem. 202: 159-161.
Varshavsky, A. (1996) The N-end rule: Functions, mysteries, uses. Proc. Natl. Acad. Sci USA 93, 121423-12149.
Vera, A., and Sugiura M (1995). Chloroplast rRNA transcription from structurally different tandem promoters: an additional novel-type promoter. Curr. Genet. 27, 280-284.
Voorma, H.O. (1996) Control of translation in prokaryotes. In: Translational Control, Hershey, J.W.B., Methews, M.B. and Sonenberg, N., eds. pp. 759-777, Cold Spring Harbor Laboratory Press, Plainview, NY
Wehrmann, A., Vliet, V.A., Opsomer, C., Bottermanm J., and Schulz, A. (1996) The bar and pat gene products make them equally applicable for plant engineers. Nat. Biotechnol. 14:1274-1278.
Wohlleben, W., Arnold, W., Broer, I., Hillemann, D., Strauch, E. and P|hler, A. (1988) Nucleotide sequence of the phosphinothricin N-acetyltransferase gene from Streptomyces T|949 and its expression in Nicotiana tabacum. Gene 70:25-37.
Wu, C.J. and Janssen, G.R. (1996) Translation of vph mRNA in Streptomyces lividans and Escherichia coli after removal of the 5' untranslated leader. Mol Microbiol, 22, 339-355.
Zerges, W., Girard-Bascou, J. and Rochaix, J.D. (1997) Translation of the chloroplast psbC mRNA is controlled by interactions between its 5' leader and the nuclear loci TBC1 and TBC3 in Chlamydomonas reinhardtii. Mol. Cell. Biol., 17, 3440-3448.
Zhang, W., and Wu, R. 1988. Efficient regeneration of transgenic plants from rice protoplasts and correctly regulated expression of the foreign gene in the plants. Theor. Appl. Gene. 76: 835-840.
Zhou, J., Liu, W.J., Peng, S.W., Sun, X.Y. and Frazer, I. (1999) Papillomavirus capsid protein expression level depends on the match between codon usage and tRNA availability. Journal Of Virology, 73, 4972-4982.
Zoubenko, O. V., Allison, L. A., Svab, Z. and Maliga, P. 1994. Efficient targeting of foreign genes into the tobacco plastid genome. Nuceic Acids Res. 22: 3819-3824.

## Claims

1. A recombinant DNA construct for expressing at least one heterologous protein in the plastids of higher plants, said construct comprising a 5' regulatory region which includes a promoter element, a leader sequence and a downstream box element operably linked to a coding region of said at least one heterologous protein, said chimeric regulatory region enhancing translational efficiency of an mRNA molecule encoded by said DNA construct.

2. A vector comprising the DNA construct of claim 1 .

3. A recombinant DNA construct as claimed in claim 1, said 5' regulatory region being selected from the group consisting of PrrnLatpB+DBwt, SEQ ID NO: 1,
PrrnLatpB+DBm, SEQ ID NO: 3, PrrnLclpP+DBwt, SEQ ID NO: 4,
PrrnLrbcL+DBwt, SEQ ID NO: 6, PrrnLrbcL+DBm, SEQ ID NO: 8,
PrrnLpsbB+DBwt, SEQ ID N0: 9, and PrrnLpsbA+DBwt, SEQ ID NO: 11.

4. A recombinant DNA, construct as claimed in claim 1, said 5' regulatory region being selected from the group consisting of PrmLT7glO+DB/Ec, SEQ ID NO; 14, and PrmLT7glO+DB/pt, SEQ ID NO:15.

5. A vector comprising a DNA construct as claimed in claim 3.

6. A DNA construct as claimed in claim 1, said downstream box element having a sequence selected from the group consisting of
5'TCCAGTCACTAGCCCTGCCTTCGGCA'3 (SEQ ID NO: 29) and
5'CCCAGTCATGAATCACAAAGTGTAA'3 (SEQ ID NO: 30).

7. A DNA construct as claimed in claim 1, wherein said heterologous protein is expressed from a bar gene encoded by S. hydroscopicus said bar gene inserted into a plasmid selected from the group consisting of pK012, and pJEK33, said pJEK3 having the sequence of SEQ ID NO: 18.

8. A DNA construct as claimed in claim 1, wherein said heterologous protein is expressed from a synthetic bar encoding nucleic acid, said synthetic bar nucleic acid having selected from the group consisting of SEQ ID NO: 19 and SEQ ID NO: 20.

9. A DNA construct as claimed in claim 1, said at least one heterologous protein comprising a fusion protein.

10. A DNA construct as claimed in claim 9, said fusion protein having a first and second coding region operably linked to said 5' regulatory region such that production of said fusion protein is regulated by said 5'regulatory region, said first coding region encoding a selectable marker gene and said second coding region encoding a fluorescent molecule to facilitate visualization of transformed plant cells.

11. A vector comprising the DNA construct of claim 10.

12. A DNA construct as claimed in claim 9, said fusion protein consisting of an aadA coding region operably linked to a green fluorescent protein coding region.

13. A DNA, construct as claimed in claim 10, said aadA coding region being operably linked to said green fluorescent protein coding region via a nucleic acid molecule encoding a peptide linker having a sequence selected from the group consisting of ELVEGKLELVEGLKVA (SEQ ID NO: 104) and ELAVEGKLEVA (SEQ ID NO: 105).

14. A DNA construct as claimed in claim 10, said construct having a sequence selected from the group of SEQ ID NOS: 21-25 and 27.

15. A plasmid for transforming the plastids of higher plants, said plasmid being selected from the group consisting of pMSK49 (SEQ ID NO: 27), and pMSK35 (SEQ ID NO: 26), and optionally comprising at least one heterologous nucleic acid for expression of at least one heterologous protein of interest.

16. A transgenic plant containing a plasmid as claimed in claim 15.

17. A transgenic plant as claimed in claim 15, said plant being selected from the group consisting of monocots and dicots.

## Patentansprüche

1. Rekombinantes DNA-Konstrukt zur Expression von mindestens einem heterologen Protein in den Plastiden von höheren Pflanzen, wobei das Konstrukt eine 5'-regulatorische Region umfasst, die Folgendes einschließt: ein Promotorelement, eine Leadersequenz und ein stromabwärts liegendes Box-Element, operativ mit einer Kodierungsregion von mindestens einem heterologen Protein verknüpft, wobei die chimäre regulatorische Region die translationale Effizienz eines mRNA-Moleküls fördert, das durch das DNA-Konstrukt kodiert ist.

2. Vektor, der das DNA-Konstrukt nach Anspruch 1 umfasst.

3. Rekombinantes DNA-Konstrukt nach Anspruch 1, wobei die 5'-regulatorische Region aus der Gruppe ausgewählt ist, bestehend aus PrmnLatpB+DBwt, SEQ ID NO: 1, PrmLatpB+DBm, SEQ ID NO: 3, PrmnLclpP+DBwt, SEQ ID NO: 4, PrmLrbcL+DBwt, SEQ ID NO: 6, PrmLrbcL+DBm, SEQ ID NO: 8, PrmLpsbB+DBwt, SEQ ID NO: 9 und PmrLpsbA+DBwt, SEQ ID NO: 11.

4. Rekombinantes DNA-Konstrukt nach Anspruch 1, wobei die 5'-regulatorische Region aus der Gruppe ausgewählt ist, bestehend aus PrmnLT7glO+DB/Ec, SEQ ID NO: 14 und PrrnLT7glO+DB/pt, SEQ ID NO: 15.

5. Vektor, der ein DNA-Konstrukt nach Anspruch 3 umfasst.

6. DNA-Konstrukt nach Anspruch 1, wobei das stromabwärts liegende Box-Element eine Sequenz aufweist, die aus der Gruppe ausgewählt ist, bestehend aus
5' TCCAGTCACTAGCCCTGCCTTCGGCA'3 (SEQ ID NO: 29) und
5' CCCAGTCATGAATCACAAAGTGGTAA'3 (SEQ ID NO: 30).

7. DNA-Konstrukt nach Anspruch 1, worin das heterologe Protein aus einem durch *S. hydroscopicus* kodiertes *bar-*Gen exprimiert wird, wobei das *bar-*Gen in ein Plasmid insertiert wird, das aus der Gruppe ausgewählt ist, bestehend aus pK012 und pJEK3, wobei das pJEK3 die Sequenz von SEQ ID NO: 18 aufweist.

8. DNA-Konstrukt nach Anspruch 1, worin das heterologe Protein aus einem synthetischen Nukleinsäure kodierenden *bar* exprimiert wird, wobei die synthetische bar-Nukleinsäure aus der Gruppe ausgewählt ist, bestehend aus SEQ ID NO: 19 und SEQ ID NO: 20.

9. DNA-Konstrukt nach Anspruch 1, wobei mindestens ein heterologes Protein ein Fusionsprotein umfasst.

10. DNA-Konstrukt nach Anspruch 9, wobei das Fusionsprotein eine erste und zweite Kodierungsregion aufweist, operativ mit der 5'-regulatorischen Region dergestalt verknüpft, dass die Produktion des Fusionsproteins durch die 5'-regulatorische Region reguliert wird, wobei die erste Kodierungsregion für ein auswählbares Markergen kodiert und die zweite Kodierungsregion für ein Fluoreszenzmolekül kodiert, um die Sichtbarmachung von transformierten Pflanzenzellen zu fördern.

11. Vektor, der das DNA-Konstrukt nach Anspruch 10 umfasst.

12. DNA-Konstrukt nach Anspruch 9, wobei das Fusionsprotein, das aus einer aadA-Kodierungsregion besteht, die operativ mit einer grün fluoreszierenden Proteinkodierungsregion verknüpft ist.

13. DNA-Konstrukt nach Anspruch 10, wobei die aadA-Kodietungsregion operativ mit der grün fluoreszierenden Proteinkodierungsregion über ein Nukleinsäuremolekül verknüpft ist, das für einen Peptidlinker kodiert, der eine Sequenz aufweist, die aus der Gruppe ausgewählt ist, bestehend aus ELVEGKLELVEGLKVA (SEQ ID NO: 104) und ELAVEGKLEVA (SEQ ID NO: 105).

14. DNA-Konstrukt nach Anspruch 10, wobei das Konstrukt eine Sequenz aufweist, die aus der Gruppe von SEQ ID NO: 21-25 und 27 ausgewählt ist.

15. Plasmid zum Transformieren der Plastide von höheren Pflanze, wobei das Plasmid aus der Gruppe ausgewählt ist, bestehend aus pMSK49 (SEQ ID NO: 27) und pMSK35 (SEQ ID NO: 26), und optional mindestens eine heterologe Nukleinsäure zur Expression von mindestens einem heterologen Protein von Interesse umfasst.

16. Transgene Pflanze, die ein Plasmid nach Anspruch 15 enthält.

17. Transgene Pflanze nach Anspruch 15, wobei die Pflanze aus der Gruppe ausgewählt ist, bestehend aus Monokotylen und Dikotylen.

## Revendications

1. Produit de recombinaison d'ADN recombiné destiné à exprimer au moins une protéine hétérologue dans les plastes de plantes supérieures, ledit produit de recombinaison comprenant une région régulatrice en 5' qui comporte un élément de promoteur, une séquence de tête et un élément de boîte en aval lié de manière opérante à une région codante de ladite au moins une protéine hétérologue, ladite région régulatrice chimère améliorant l'efficacité de traduction d'une molécule d'ARNm codée par ledit produit de recombinaison d'ADN.

2. Vecteur comprenant le produit de recombinaison d'ADN selon la revendication 1.

3. Produit de recombinaison d'ADN recombiné selon la revendication 1, ladite région régulatrice en 5' étant choisie parmi le groupe constitué par PrnnLatpB+DBwt, SEQ. ID n°1, PrrnLatpB+DBm, SEQ. ID n°3, PrrnLclpP+DBwt, SEQ. ID n°4, PrrnLrbcL+DBwt, SEQ. ID n°6, PrrnLrbcL+DBm, SEQ. ID n°8, PrrnLpsbB+DBwt, SEQ. ID n°9, et PrrnLpsbA+DBwt, SEQ. ID n°11.

4. Produit de recombinaison d'ADN recombiné selon la revendication 1, ladite région régulatrice en 5' étant choisie parmi le groupe constitué par PrrnLT7glO+DB/Ec, SEQ. ID n°14, et PrrnLT7glO+DB/pt, SEQ. ID n°15.

5. Vecteur comprenant un produit de recombinaison d'ADN selon la revendication 3.

6. Produit de recombinaison d'ADN selon la revendication 1, ledit élément de boîte en aval ayant une séquence choisie parmi le groupe constitué par
5'TCCAGTCACTAGCCCTGCCTTCGGCA'3 (SEQ. ID n°29) et
5'CCCAGTCATGAATCACAAAGTGGTAA'3 (SEQ. ID n°30).

7. Produit de recombinaison d'ADN selon la revendication 1, dans lequel ladite protéine hétérologue est exprimée à partir d'un gène bar codé par S. hydroscopicus, ledit gène bar étant inséré dans un plasmide choisi parmi le groupe constitué par pK012 et pJEK3, ledit pJEK3 ayant la séquence de SEQ. ID n°18.

8. Produit de recombinaison d'ADN selon la revendication 1, dans lequel ladite protéine hétérologue est exprimée à partir d'un acide nucléique codant bar synthétique, ledit acide nucléique bar synthétique étant choisi parmi le groupe constitué par SEQ. ID n°19 et SEQ. ID n°20.

9. Produit de recombinaison d'ADN selon la revendication 1, ladite au moins une protéine hétérologue comprenant une protéine fusion.

10. Produit de recombinaison d'ADN selon la revendication 9, ladite protéine fusion ayant une première et une deuxième région codante liées de manière opérante à ladite région régulatrice en 5', de sorte que la production de ladite protéine fusion est régulée par ladite région régulatrice en 5', ladite première région codante codant pour un gène marqueur sélectionnable et ladite deuxième région codante codant pour une molécule fluorescente afin de faciliter la visualisation des cellules végétales transformées.

11. Vecteur comprenant le produit de recombinaison d'ADN selon la revendication 10.

12. Produit de recombinaison d'ADN selon la revendication 9, ladite protéine fusion étant constituée d'une région codante aadA liée de manière opérante à une région codant pour une protéine fluorescente verte.

13. Produit de recombinaison d'ADN selon la revendication 10, ladite région codante aadA étant liée de manière opérante à ladite région codant pour une protéine fluorescente verte par l'intermédiaire d'une molécule d'acide nucléique codant pour un peptide lieur ayant une séquence choisie parmi le groupe constitué par ELVEGKLELVEGLKVA (SEQ. ID n°104) et ELAVEGKLEVA (SEQ. ID n°105).

14. Produit de recombinaison d'ADN selon la revendication 10, ledit produit de recombinaison ayant une séquence choisie parmi le groupe constitué par SEQ. ID n°21-25 et 27.

15. Plasmide destiné à transformer les plastes de plantes supérieures, ledit plasmide étant choisi parmi le groupe constitué par pMSK49 (SEQ. ID n°27) et pMSK35 (SEQ. ID n°26), et comprenant éventuellement au moins un acide nucléique hétérologue destiné à l'expression d'au moins une protéine hétérologue d'intérêt.

16. Plante transgénique contenant un plasmide selon la revendication 15.

17. Plante transgénique selon la revendication 15, ladite plante étant choisie parmi le groupe constitué par les monocotylédones et les dicotylédones.
